(19) Europäisches Patentamt / European Patent Office / Office européen des brevets

(11) **EP 2 275 448 A2**

(12) **EUROPEAN PATENT APPLICATION**

(43) Date of publication:
**19.01.2011 Bulletin 2011/03**

(51) Int Cl.:
***C07K 16/28*** *(2006.01)* ***C12N 15/13*** *(2006.01)*
***C12N 5/10*** *(2006.01)*

(21) Application number: **10182225.2**

(22) Date of filing: **17.12.2004**

(84) Designated Contracting States:
**AT BE BG CH CY CZ DE DK EE ES FI FR GB GR HU IE IS IT LI LT LU MC NL PL PT RO SE SI SK TR**

(30) Priority: **19.12.2003 US 531409 P**

(62) Document number(s) of the earlier application(s) in accordance with Art. 76 EPC:
**04814762.3 / 1 718 677**

(71) Applicant: **Genentech, Inc.**
**South San Francisco CA 94080-4990 (US)**

(72) Inventors:
- **Huang, Arthur Jyh-Yen**
  **Oakland, CA 94610 (US)**
- **Yansura, Daniel G.**
  **Pacifica, CA 94044 (US)**
- **Schwall, Ralph, H.**
  **. (US)**

(74) Representative: **King, Hilary Mary et al**
**Mewburn Ellis LLP**
**33 Gutter Lane**
**London**
**EC2V 8AS (GB)**

Remarks:
This application was filed on 29-09-2010 as a divisional application to the application mentioned under INID code 62.

(54) **Monovalent antibody fragments useful as therapeutics**

(57) The invention provides methods and compositions comprising a novel stabilized monovalent antibody fragment.

EP 2 275 448 A2

Sequence Listing

<110> GENENTECH, INC.

<120> MONOVALENT ANTIBODY FRAGMENTS USEFUL AS THERAPEUTICS

<130> HMK/FP6716633

<140> EP
<141> 2004-12-17

<150> EP 04814762.3
<151> 2004-12-17

<150> PCT/US2004/042619
<151> 2004-12-17

<150> US 60/531409
<151> 2003-12-19

<160> 14

<210> 1
<211> 5
<212> PRT
<213> Mus musculus

<400> 1
```
Ser Tyr Trp Leu His
                  5
```

<210> 2
<211> 17
<212> PRT
<213> Mus musculus

<400> 2
```
Met Ile Asp Pro Ser Asn Ser Asp Thr Arg Phe Asn Pro Asn Phe
  1               5                  10                  15

Lys Asp
```

<210> 3
<211> 10
<212> PRT
<213> Mus musculus

<400> 3
```
Tyr Gly Ser Tyr Val Ser Pro Leu Asp Tyr
                  5                  10
```

<210> 4
<211> 17
<212> PRT
<213> Mus musculus

<400> 4
```
Lys Ser Ser Gln Ser Leu Leu Tyr Thr Ser Ser Gln Lys Asn Tyr
  1               5                  10                  15

Leu Ala
```

<210> 5
<211> 7
<212> PRT
<213> Mus musculus

<400> 5
Trp Ala Ser Thr Arg Glu Ser
5

<210> 6
<211> 9
<212> PRT
<213> Mus musculus

<400> 6
Gln Gln Tyr Tyr Ala Tyr Pro Trp Thr
5

<210> 7
<211> 119
<212> PRT
<213> Mus musculus

<400> 7
Gln Val Gln Leu Gln Gln Ser Gly Pro Glu Leu Val Arg Pro Gly
1 5 10 15

Ala Ser Val Lys Met Ser Cys Arg Ala Ser Gly Tyr Thr Phe Thr
20 25 30

Ser Tyr Trp Leu His Trp Val Lys Gln Arg Pro Gly Gln Gly Leu
35 40 45

Glu Trp Ile Gly Met Ile Asp Pro Ser Asn Ser Asp Thr Arg Phe
50 55 60

Asn Pro Asn Phe Lys Asp Lys Ala Thr Leu Asn Val Asp Arg Ser
65 70 75

Ser Asn Thr Ala Tyr Met Leu Leu Ser Ser Leu Thr Ser Ala Asp
80 85 90

Ser Ala Val Tyr Tyr Cys Ala Thr Tyr Gly Ser Tyr Val Ser Pro
95 100 105

Leu Asp Tyr Trp Gly Gln Gly Thr Ser Val Thr Val Ser Ser
110 115

<210> 8
<211> 113
<212> PRT
<213> Mus musculus

<400> 8
Asp Ile Met Met Ser Gln Ser Pro Ser Ser Leu Thr Val Ser Val
1 5 10 15

Gly Glu Lys Val Thr Val Ser Cys Lys Ser Ser Gln Ser Leu Leu
20 25 30

Tyr Thr Ser Ser Gln Lys Asn Tyr Leu Ala Trp Tyr Gln Gln Lys
35 40 45

Pro Gly Gln Ser Pro Lys Leu Leu Ile Tyr Trp Ala Ser Thr Arg
50 55 60

Glu Ser Gly Val Pro Asp Arg Phe Thr Gly Ser Gly Ser Gly Thr
65 70 75

Asp Phe Thr Leu Thr Ile Thr Ser Val Lys Ala Asp Asp Leu Ala
80 85 90

Val Tyr Tyr Cys Gln Gln Tyr Tyr Ala Tyr Pro Trp Thr Phe Gly
95 100 105

Gly Gly Thr Lys Leu Glu Ile Lys
110

<210> 9
<211> 46
<212> DNA
<213> Artificial sequence

<220>
<223> sequence was synthesized

<400> 9
taaatttaac gcgtacgctg acattatgat gtcccagtct ccatcc 46

<210> 10
<211> 36
<212> DNA
<213> Artificial sequence

<220>
<223> sequence was synthesized

<400> 10
gggcgagctc aggccctgat gggtgacttc gcaggc 36

<210> 11
<211> 42
<212> DNA
<213> Artificial sequence

<220>
<223> sequence was synthesized

<400> 11
gctacaaacg cgtacgctca ggttcagctg cagcagtctg gg 42

<210> 12
<211> 28
<212> DNA
<213> Artificial sequence

<220>
<223> sequence was synthesized

<400> 12
aagagacggt gaccgaggtt ccttgacc 28

<210> 13
<211> 43
<212> DNA
<213> Artificial sequence

<220>
<223> sequence was synthesized

<400> 13
tttccctttg aattcttggt tgttaacgtt gccgacgcgc atc 43

<210> 14
<211> 54
<212> DNA
<213> Artificial sequence

<220>
<223> sequence was synthesized

<400> 14
tttcccttta tcgatgataa gctgtcaaac atgagtaaac aataaaaaac 50

gccc 54

**Description**

RELATED APPLICATIONS

This application is a non-provisional application filed under 37 CFR 1.53(b)(1), claiming priority benefit of provisional application number 60/531,409 filed December 19, 2003, the contents of which are incorporated herein by reference in its entirety.

TECHNICAL FIELD

The present invention relates generally to the fields of molecular biology and antibody therapeutics. More specifically, the invention concerns novel forms of monovalent antibody fragments with unique characteristics for use as therapeutic agents, and uses of said antibody fragments.

BACKGROUND

Recent years have seen increasing promises of using antibodies as diagnostic and therapeutic agents for various disorders and diseases. The importance of antibodies in general for diagnostic, research and therapeutic purposes is reflected in the significant amount of effort that has been expended to study and to modify antibody sequences and structures from those found in natural antibodies, to achieve desired characteristics.

The prevailing view is that an ideal therapeutic antibody would possess certain minimal characteristics, including target specificity, biostability and bioavailability following administration to a subject patient, and sufficient target binding affinity to maximize therapeutic effects. Unfortunately, there has been limited success in efforts to generate antibody therapeutics that possess all, or even most of these minimal characteristics. For example, full length antibodies such as IgG exhibit desirable pharmacokinetics (e.g., substantial half lives in vivo) and good target binding affinities due to avidity effects derived from the presence of two antigen binding arms in a single antibody molecule. However, such full length antibodies suffer from bioavailability problems as a consequence of its greater molecular size. Furthermore, a full length antibody may in some cases exhibit agonistic effects (which is undesirable) upon binding to a target antigen even though it is an antagonistic antibody as a Fab fragment. See, e.g., US Pat. No. 6,468,529. This phenomenon is unfortunate where the antagonistic effect is the desired therapeutic function. In some instances, this phenomenon may be due to the "cross-linking" effect of a bivalent antibody that when bound to a cell surface receptor promotes receptor dimerization that leads to receptor activation.

While a monovalent antibody would not be expected to have the "cross-finking" effect, to date monovalent antibodies have not been desirable as therapeutics because of certain limitations inherent in its structure/architecture. For example, monovalent antibody in Fab form possesses inferior pharmacodynamics (e.g., unstable in vivo and rapid clearance following administration) with respect to use as therapeutic agents. Furthermore, compared with their multivalent counterparts, monovalent antibodies generally have lower apparent binding affinity due to absence of avidity binding effects.

In general, the choice of antibody form for use as therapeutic agents has been governed by an acceptance of the reality that each has undesirable limitations. Nonetheless, it is apparent that the full length antibody form has been the form of choice in recent years, likely due at least in part to its biostability *in vivo.* Monovalent antibodies may be acceptable where, on the balance, biostability is not as critical a factor for therapeutic efficacy than bioavailability. For example, due in part to better tissue penetrance compared to full length antibodies, monovalent Fab antibodies may be better vehicles for delivery of heterologous molecules such as toxins to the target cells or tissues where the heterologous molecule exerts a therapeutic function. See e.g., US Pat. No. 5,169,939. Other examples of attempts to develop monovalent antibodies as therapeutics include settings wherein monovalency is critical for obtaining a therapeutic effect, e.g., where there are concerns that bivalency of an antibody might induce a target cell to undergo antigenic modulation which might consequently provide a means for the target cell to avoid cytotoxic agents, effectors and complement. Examples of such antibodies are described in Cobbold & Waldmann, Nature (1984), 308:460-462; EP 0 131 424; Glennie & Stevenson, Nature (1982), 295:712-714; Nielsen & Routledge, Blood (2002), 100:4067-4073; Stevenson et al., Anticancer Drug Des. (1989), 3(4):219-230; Routledge et al., Transplantation (1995), 60:847-853; Clark et al., Eur. J. Immunol. (1989), 19:381-388; Bolt et al., Eur. J. Immunol. (1993), 23:403-411; Routledge et al., Eur. J. Immunol. (1991), 21: 2717-2725; Staerz et al., Nature (1985), 314:628-631; and U.S. Pat. No. 5,968,509. Notably, these monovalent antibody fragments contain functional Fc sequences, which are included because their effector functions (such as complement-mediated lysis of T cells) are needed for therapeutic function. Other than the scenario described, the art does not appear to have recognized a need or utility for including an Fc region in monovalent antibodies that are used and/or developed as therapeutics. The reluctance to include an Fc region in monovalent antibodies where the Fc region is not necessary for therapeutic function is underscored by the practical difficulties of obtaining such antibodies. Existing antibody production technology does not provide an efficient method to obtain in high quantities and in sufficiently purified form heterodimers comprising a single antigen binding component (i.e., monovalency) and an Fc region.

Notably, some efforts have been made to increase in vivo stability of antibody fragments with varying degrees of success. For example, a Fab fragment may be attached to stability moieties such as polyethylene glycol or other stabilizing molecules such as heterologous peptides. See, e.g., Dennis et al., J. Biol. Chem. (2002), 277:35035-35043; PCT Pub. No. WO01/45746.

In view of the above, there remains a significant need for improved antibody forms, and methods of producing and using such antibodies, for example as therapeutic or prophylactic agents. The invention described herein addresses this need and provides other benefits.

All references cited herein, including patent applications and publications, are incorporated by reference in their entirety.

## DISCLOSURE OF THE INVENTION

The invention provides a form of antibody that provides various advantages with respect to therapeutic utility, functionality and methods of production thereof. In one aspect, an antibody of the invention provides a monovalent characteristic which is essential for certain non-immune response based therapeutic schemes. For example, in pathological conditions requiring an antagonistic function, and where bivalency of an antibody results in an undesirable agonistic effect, the monovalent trait of an antibody of the invention results in and/or ensures an antagonistic function upon binding of the antibody to a target molecule. Furthermore, an antibody of the invention is characterized by superior pharmacokinetic attributes (such as an enhanced half life and/or reduced clearance rate *in vivo*) compared to Fab forms having similar/substantially identical antigen binding characteristics, thus overcoming a major drawback in the use of conventional monovalent Fab antibodies. In one aspect, an antibody of the invention comprises little to no immune effector functions, a trait which is particularly useful in treating pathological conditions wherein an immune effector response is deleterious.

In another aspect, an antibody of the invention is characterized by alterations that greatly improve production yield. Furthermore, as opposed to certain conventional methods for producing monovalent antibody fragments (e.g., enzymic digestion, followed in some instances by chemical couplings), the recombinant nature of the production methods of the invention makes it possible to obtain antibody populations that are of a sufficiently high degree of homogeneity and/or purity useful for development and/or commercialization as therapeutic agents.

Accordingly, in one aspect, the invention provides a monovalent antibody fragment comprising a single target molecule binding arm and an Fc region (i.e., a complex of Fc polypeptides), wherein the monovalent antibody fragment is more stable *in vivo* than a counterpart antibody fragment lacking said Fc region. In one aspect, the invention provides an antibody fragment comprising a single antigen binding arm and an Fc region that increases stability of the antibody fragment (i.e., it is more stable, e.g. it exhibits a longer *in vivo* half life) compared to a Fab molecule comprising said antigen binding arm, wherein said Fc region comprises a complex of a first and a second Fc polypeptide, wherein one but not both of the Fc polypeptides is an N-terminally truncated heavy chain. In one embodiment, an N-terminally truncated heavy chain consists or consists essentially of a hinge sequence contiguously linked to at least a portion of a heavy chain CH2 and/or CH3 domain sufficient to form a complex with the first Fc polypeptide and confer said increased stability. In one embodiment, an N-terminally truncated heavy chain consists or consists essentially of a hinge sequence contiguously linked to a heavy chain CH2 and/or CH3 domain capable of forming a complex with the first Fc polypeptide and conferring said increased stability. In one embodiment, the N terminal sequence of the N-terminally truncated heavy chain is part or all of a hinge sequence (i.e., the truncated heavy chain comprises an N terminus which comprises or is part or all of a hinge sequence). In one embodiment, the N-terminally truncated heavy chain is of an IgG heavy chain. In one embodiment, the Fc region is capable of binding to FcRn. In one embodiment, the Fc region does not possess an immune effector function other than binding to FcRn. Generally and preferably, the N-terminally truncated heavy chain does not specifically bind an antigen.

As described herein, an antibody fragment of the invention is characterized by significantly enhanced stability compared to its Fab fragment counterpart. In some embodiments, an antibody fragment of the invention exhibits at least about 2X, at least about 5X, at least about 10X, at least about 25X, at least about 50X, at least about 100X, at least about 200X, at least about 300X, at least about 350X, at least about 400X, at least about 450X, at least about 500X the in vivo half life of its Fab fragment counterpart. In vivo half life can be measured by any of a variety of methods known in the art, some of which are described herein. In one embodiment, in vivo half life is measured by administering to a suitable mammal (such as mouse) an amount of an antibody, and measuring the rate of decrease in amount of the administered antibody in the mammal.

Immune effector functions are unnecessary or even deleterious in certain clinical settings. In some embodiments, an antibody of the invention is aglycosylated. Such antibodies do not exhibit substantial immune effector functions that are dependent on glycosylation of the Fc region. Generally and preferably, an aglycosylated antibody of the invention

does not exhibit substantial immune effector functions except for binding to FcRn. In some embodiments, an antibody fragment of the invention does not possess substantial or completely lacks effector functions other than FcRn binding. In one embodiment, said effector function is complement lysis. In one embodiment, said effector function is antibody dependent cell cytotoxicity (ADCC). In one embodiment, the antibody fragment binds FcRn. Aglycosylated antibodies can be produced by a variety of methods known in the art. A convenient method comprises expressing the antibody in a prokaryotic host cell such as *E. coli.*

In one embodiment, an antibody fragment of the invention is glycosylated. Glycosylation can be achieved by methods known in the art, e.g., by producing the antibody in a mammalian host cell such as Chinese Hamster Ovary (CHO) cell.

In some embodiments, an antibody fragment of the invention does not target a component of the immune response, and therefore its mechanism of therapeutic action does not comprise regulation and/or engagement of an immune response. E.g., in one embodiment, an antibody fragment of the invention has little to no immunosuppressive properties. For instance, said immunosuppressive properties may comprise ability to directly or indirectly effect T cell depletion. In one embodiment, said antibody fragment does not specifically bind a T cell surface antigen, which in some embodiments is CD3 or CD4. In one embodiment, said T cell surface antigen is CD3. In yet another embodiment, the antibody fragment does not specifically bind an immunoglobulin polypeptide, for example it does not specifically bind constant determinants on the lambda chain of surface immunoglobulins or idiotypic determinants on surface immunoglobulins.

An antibody fragment of the invention is capable of specifically binding to a target molecule of interest. For example, in some embodiments, an antibody fragment specifically binds a tumor antigen. In some embodiments, the antibody fragment specifically binds a cell surface receptor that is activated upon receptor multimerization (e.g., dimerization). In some embodiments, binding of an antibody of the invention to a target molecule inhibits binding of another molecule (such as a ligand, where the target molecule is a receptor) to said target molecule. Thus, in one example, an antibody fragment of the invention when bound to a target molecule inhibits binding of a cognate binding partner to the target molecule. A cognate binding partner can be a ligand, or a hetero or homodimerizing molecule. In one embodiment, an antibody fragment of the invention when bound to a target molecule inhibits target molecule multimerization. For example, in some embodiments wherein an antibody fragment of the invention is an antagonist, binding of the antibody fragment to a cell surface receptor may inhibit dimerization of the receptor with another unit of the receptor, whereby activation of the receptor is inhibited (due at least in part to a lack of receptor dimerization). Numerous receptor molecules are known in the art to be capable of and/or to require dimerization (either homo- or heterodimerization) for effecting their normal functions. Such receptors include receptor tyrosine kinases such as fibroblast growth factor receptors and the HGF receptor, c-met. Other protein-protein interactions include receptor-ligand interactions, such as VEGF (vascular endothelial growth factor) binding to flt, flk, etc., and hepatocyte growth factor (HGF) binding to c-met. In one embodiment, an antibody fragment of the invention is capable of competing with HGF for binding to c-met. In another embodiment, an antibody fragment of the invention is capable of competing with VEGF for binding to a VEGF receptor.

In one aspect, the invention provides an antibody fragment that is an antagonist in single-armed form (as described herein), but is an agonist or has agonist activity in a two-armed form (i.e., wherein the two arms have the same antigen binding capability).

In one aspect, the invention provides an antibody fragment comprising: (i) a first polypeptide comprising a light chain variable domain (and in some embodiments further comprising a light chain constant domain), (ii) a second polypeptide comprising a heavy chain variable domain, a first Fc polypeptide sequence (and in some embodiments further comprising a non-Fc heavy chain constant domain sequence), and (iii) a third polypeptide comprising a second Fc polypeptide sequence. Generally, the second polypeptide is a single polypeptide comprising a heavy chain variable domain, heavy chain constant domain (e.g., all or part of CH1) and the first Fc polypeptide. For example, the first Fc polypeptide sequence is generally linked to the heavy chain constant domain by a peptide bond [i.e., not a non-peptidyl bond]. In one embodiment, the first polypeptide comprises a non-human light chain variable domain fused to a human light chain constant domain. In one embodiment, the second polypeptide comprises a non-human heavy chain variable domain fused to a human heavy chain constant domain. In one embodiment, the third polypeptide comprises an N-terminally truncated heavy chain which comprises at least a portion of a hinge sequence at its N terminus. In one embodiment, the third polypeptide comprises an N-terminally truncated heavy chain which does not comprise a functional or wild type hinge sequence at its N terminus. In some embodiments, the two Fc polypeptides of an antibody fragment of the invention are covalently linked. For example, the two Fc polypeptides may be linked through intermolecular disulfide bonds, for instance through intermolecular disulfide bonds between cysteine residues of the hinge region.

In one aspect, the invention provides a composition comprising a population of immunoglobulins wherein at least (or at least about) 50%, 75%, 85%, 90%, 95% of the immunoglobulins are antibody fragments of the invention. A composition comprising said population of immunoglobulins can be in any of a variety of forms, including but not limited to host cell lysate, cell culture medium, host cell paste, or semi-purified or purified forms thereof. Purification methods are well known in the art, some of which are described herein.

In one aspect, the invention provides an antibody fragment comprising at least one characteristic that promotes heterodimerization, while minimizing homodimerization, of the Fc sequences within the antibody fragment. Such characteristic(s) improves yield and/or purity and/or homogeneity of the immunoglobulin populations obtainable by methods of the invention as described herein. In one embodiment, a first Fc polypeptide and a second Fc polypeptide meet/interact at an interface. In some embodiments wherein the first and second Fc polypeptides meet at an interface, the interface of the second Fc polypeptide (sequence) comprises a protuberance which is positionable in a cavity in the interface of the first Fc polypeptide (sequence). In one embodiment, the first Fc polypeptide has been altered from a template/original polypeptide to encode the cavity or the second Fc polypeptide has been altered from a template/original polypeptide to encode the protuberance, or both. In one embodiment, the first Fc polypeptide has been altered from a template/original polypeptide to encode the cavity and the second Fc polypeptide has been altered from a template/original polypeptide to encode the protuberance. In one embodiment, the interface of the second Fc polypeptide comprises a protuberance which is positionable in a cavity in the interface of the first Fc polypeptide, wherein the cavity or protuberance, or both, have been introduced into the interface of the first and second Fc polypeptides, respectively. In some embodiments wherein the first and second Fc polypeptides meet at an interface, the interface of the first Fc polypeptide (sequence) comprises a protuberance which is positionable in a cavity in the interface of the second Fc polypeptide (sequence). In one embodiment, the second Fc polypeptide has been altered from a template/original polypeptide to encode the cavity or the first Fc polypeptide has been altered from a template/original polypeptide to encode the protuberance, or both. In one embodiment, the second Fc polypeptide has been altered from a template/original polypeptide to encode the cavity and the first Fc polypeptide has been altered from a template/original polypeptide to encode the protuberance. In one embodiment, the interface of the first Fc polypeptide comprises a protuberance which is positionable in a cavity in the interface of the second Fc polypeptide, wherein the protuberance or cavity, or both, have been introduced into the interface of the first and second Fc polypeptides, respectively.

In one embodiment, the protuberance and cavity each comprise a naturally occurring amino acid residue. In one embodiment, the Fc polypeptide comprising the protuberance is generated by replacing an original residue from the interface of a template/original polypeptide with an import residue having a larger side chain volume than the original residue. In one embodiment, the Fc polypeptide comprising the protuberance is generated by a method comprising a step wherein nucleic acid encoding an original residue from the interface of said polypeptide is replaced with nucleic acid encoding an import residue having a larger side chain volume than the original. In one embodiment, the original residue is threonine. In one embodiment, the original residue is T366. In one embodiment, the import residue is arginine (R). In one embodiment, the import residue is phenylalanine (F). In one embodiment, the import residue is tyrosine (Y). In one embodiment, the import residue is tryptophan (W). In one embodiment, the import residue is R, F, Y or W. In one embodiment, a protuberance is generated by replacing two or more residues in a template/original polypeptide. In one embodiment, the Fc polypeptide comprising a protuberance comprises replacement of threonine at position 366 with tryptophan, amino acid numbering according to the EU numbering scheme of Kabat et al. (pp. 688-696 in Sequences of proteins of immunological interest, 5th ed., Vol. 1 (1991; NIH, Bethesda, MD)).

In some embodiments, the Fc polypeptide comprising a cavity is generated by replacing an original residue in the interface of a template/original polypeptide with an import residue having a smaller side chain volume than the original residue. For example, the Fc polypeptide comprising the cavity may be generated by a method comprising a step wherein nucleic acid encoding an original residue from the interface of said polypeptide is replaced with nucleic acid encoding an import residue having a smaller side chain volume than the original. In one embodiment, the original residue is threonine. In one embodiment, the original residue is leucine. In one embodiment, the original residue is tyrosine. In one embodiment, the import residue is not cysteine (C). In one embodiment, the import residue is alanine (A). In one embodiment, the import residue is serine (S). In one embodiment, the import residue is threonine (T). In one embodiment, the import residue is valine (V). A cavity can be generated by replacing one or more original residues of a template/original polypeptide. For example, in one embodiment, the Fc polypeptide comprising a cavity comprises replacement of two or more original amino acids selected from the group consisting of threonine, leucine and tyrosine. In one embodiment, the Fc polypeptide comprising a cavity comprises two or more import residues selected from the group consisting of alanine, serine, threonine and valine. In some embodiments, the Fc polypeptide comprising a cavity comprises replacement of two or more original amino acids selected from the group consisting of threonine, leucine and tyrosine, and wherein said original amino acids are replaced with import residues selected from the group consisting of alanine, serine, threonine and valine. In some embodiments, an original amino acid that is replaced is T366, L368 and/or Y407. In one embodiment, the Fc polypeptide comprising a cavity comprises replacement of threonine at position 366 with serine, amino acid numbering according to the EU numbering scheme of Kabat et al. *supra.* In one embodiment, the Fc polypeptide comprising a cavity comprises replacement of leucine at position 368 with alanine, amino acid numbering according to the EU numbering scheme of Kabat et al. *supra.* In one embodiment, the Fc polypeptide comprising a cavity comprises replacement of tyrosine at position 407 with valine, amino acid numbering according to the EU numbering scheme of Kabat et al. *supra.* In one embodiment, the Fc polypeptide comprising a cavity comprises two or more amino acid replacements selected from the group consisting of T366S, L368A and Y407V, amino acid numbering

according to the EU numbering scheme of Kabat et al. *supra.* In some embodiments of these antibody fragments, the Fc polypeptide comprising the protuberance comprises replacement of threonine at position 366 with tryptophan, amino acid numbering according to the EU numbering scheme of Kabat et al. *supra.*

In one aspect, an antibody fragment of the invention comprises an Fc region the presence of which is required for increasing stability of the antibody fragment relative to a Fab fragment comprising the same antigen binding arm (sequences). Said Fc region is formed through the complexing (multimerizing) of separate Fc polypeptide sequences. Said separate Fc polypeptide sequences may or may not contain the same sequences and/or domains, provided they are capable of dimerizing to form an Fc region (as defined herein). A first Fc polypeptide is generally contiguously linked to one or more domains of an immunoglobulin heavy chain in a single polypeptide, for example with hinge, constant and/or variable domain sequences. In one embodiment, the first Fc polypeptide comprises at least a portion of a hinge sequence, at least a portion of a CH2 domain and/or at least a portion of a CH3 domain. In one embodiment, the first Fc polypeptide comprises the hinge sequence and the CH2 and CH3 domains of an immunoglobulin. In one embodiment, the second Fc polypeptide (i.e., the Fc polypeptide which is part of an N-terminally truncated heavy chain) comprises at least a portion of a hinge sequence, at least a portion of a CH2 domain and/or at least a portion of a CH3 domain. In one embodiment, the second Fc polypeptide comprises the hinge sequence and the CH2 and CH3 domains of an immunoglobulin. In one embodiment, an antibody fragment of the invention comprises first and second Fc polypeptides each of which comprising at least a portion of at least one antibody constant domain. In one embodiment, the antibody constant domain is a CH2 and/or CH3 domain. In any of the embodiments of an antibody fragment of the invention that comprises a constant domain, the antibody constant domain can be from any immunoglobulin class, for example an IgG. The immunoglobulin source can be of any suitable species of origin (e.g., an IgG may be human $IgG_1$) or of synthetic form.

An antibody of the invention comprises a single antigen binding arm. Binding to a single antigen can involve binding to one or more binding targets (e.g., determinants/epitopes). In one embodiment, an antibody of the invention is monospecific. In another embodiment, an antibody of the invention is an immunoadhesin, which in one embodiment is monospecific.

An antibody fragment of the invention may be conjugated with a heterologous moiety. Any heterologous moiety would be suitable so long as its conjugation to the antibody does not substantially reduce a desired function and/or characteristic of the antibody. For example, in some embodiments, an immunoconjugate comprises a heterologous moiety which is a cytotoxic agent. In some embodiments, said cytotoxic agent is selected from the group consisting of a radioactive isotope, a chemotherapeutic agent and a toxin. In some embodiments, said toxin is selected from the group consisting of calichemicin, maytansine and trichothene. In some embodiments, an immunoconjugate comprises a heterologous moiety which is a detectable marker. In some embodiments, said detectable marker is selected from the group consisting of a radioactive isotope, a member of a ligand-receptor pair, a member of an enzyme-substrate pair and a member of a fluorescence resonance energy transfer pair.

In a variety of settings, it is highly desirable to obtain a composition comprising a highly homogeneous population of antibody fragments of the invention. This can be achieved by a variety of methods known in the art. For example, polypeptides making up an antibody fragment of the invention are generally recombinantly expressed (as opposed to, e.g., enzymic digestion of full length immunoglobulins). In some embodiments, a composition of the invention comprises antibody fragments that are substantially homogeneous with respect to the N-terminus of the binding arm and/or C terminus of the Fc region. A composition is "substantially homogeneous" if at least 75%, at least 80%, at least 90%, at least 95%, at least 98% of the antibody fragments of the invention contained therein have the same amino acid residue at the N-terminus of the binding arm and/or C terminus of the Fc region. Said composition can be unpurified, semi-purified or purified forms of a source composition in which the antibody fragments are initially generated.

In one aspect, the invention provides compositions comprising an antibody fragment of the invention and a carrier, which in one embodiment is a pharmaceutically acceptable carrier. In one embodiment, the antibody fragment is conjugated to a heterologous moiety.

In another aspect, the invention provides articles of manufacture comprising a container and a composition contained therein, wherein the composition comprises an antibody fragment of the invention. In some embodiments, these articles of manufacture further comprise instruction for using said composition. In one embodiment, the antibody fragment is provided in a therapeutically effective amount.

In yet another aspect, the invention provides polynucleotides encoding an antibody fragment of the invention. Components of an antibody fragment of the invention can be encoded by a single polynucleotide or separate (multiple) polynucleotides. In one embodiment, a single polynucleotide encodes (a) the light and heavy chain components of the antigen binding arm, and (b) the N-terminally truncated heavy chain polypeptide. In one embodiment, a single polynucleotide encodes the light and heavy chain components of the antigen binding arm, and a separate polynucleotide encodes the N-terminally truncated heavy chain polypeptide. In one embodiment, separate polynucleotides encode the light chain component of the antigen binding arm, the heavy chain component of the antigen binding arm and the N-terminally truncated heavy chain polypeptide, respectively.

In one aspect, the invention provides recombinant vectors for expressing an antibody of the invention.

In one aspect, the invention provides host cells comprising a polynucleotide or recombinant vector of the invention. In one embodiment, the host cell is a prokaryotic cell, for example, E. coli. In one embodiment, a host cell is a eukaryotic cell, for example a mammalian cell such as Chinese Hamster Ovary (CHO) cell.

In one aspect, the invention provides a method of generating an antibody fragment of the invention, said method comprising expressing in a suitable host cell (e.g., E. coli or CHO) nucleic acid encoding the antibody fragment under conditions that permit heteromultimerization that results in formation of the antibody fragment. In one embodiment, at least 50%, at least 70%, at least 80%, at least 90%, at least 95% of the immunoglobulin polypeptides generated in the host cell culture are an antibody fragment of the invention. In one embodiment, the antibody fragment generated by a method of the invention comprises a protuberance in one Fc polypeptide and a cavity in another Fc polypeptide as described herein. In one embodiment, the invention provides a method comprising expressing three polynucleotides in a host cell, wherein a first polynucleotide encodes a first component of an antigen binding arm (e.g., heavy chain CDR sequence(s) or variable domain (and in some examples, further comprising a non-Fc heavy chain constant domain sequence)) and a first Fc polypeptide, a second polynucleotide encodes a second component of the antigen binding arm (e.g., light chain CDR sequence(s) or variable domain (and in some examples, further comprising a light chain constant domain)), and a third polynucleotide encodes an N-terminally truncated heavy chain comprising a second Fc polypeptide, wherein an antibody fragment of the invention is formed by heteromultimerization of these polypeptides. In one embodiment, the method comprises introducing said polynucleotides into a suitable host cell. In one embodiment, the method comprises recovering the antibody fragment of the invention from the cell culture, e.g. from cell lysates or culture medium.

In one aspect, the invention provides a method comprising expressing in a suitable host cell nucleic acid encoding components of an antibody fragment of the invention, wherein each cistron encoding a component comprises a translational initiation region (TIR) operably linked to a nucleic acid sequence encoding said component, and wherein the strength of each TIR is adjusted to obtain a suitable ratio of expression levels of the components whereby a desired amount of said antibody fragment is generated. In one embodiment, the TIRs are of approximately equal strength. In one embodiment, the relative TIR is 1, for example in accordance with Simmons & Yansura, Nature Biotechnol. (1996), 14:629-634 and Simmons et al., J. Immunol. Methods (2002), 263:133-147. In some embodiments, the TIR comprises a prokaryotic secretion signal sequence or variant thereof. In some embodiments, the prokaryotic secretion signal sequence is selected from the group consisting of STII, OmpA, PhoE, LamB, MBP and PhoA secretion signal sequences.

Antibodies of the invention find a variety of uses in a variety of settings. For example, an antibody of the invention is generally a therapeutic antibody. An antibody of the invention can exert its therapeutic effect by any of a variety of mechanisms. For example, an antibody of the invention may be an agonist antibody. In another example, an antibody of the invention may be an antagonistic antibody. In yet another example, an antibody of the invention may be a blocking antibody. In another example, an antibody of the invention is a neutralizing antibody.

In one aspect, the invention provides methods of treating or delaying progression of a disease comprising administering to a subject having the disease an effective amount of an antibody fragment of the invention effective in treating or delaying progression of the disease. In one embodiment, the disease is a tumor or cancer. In one embodiment, the disease is an immunological disorder, e.g. an autoimmune disease, e.g., rheumatoid arthritis, immune thrombocytopenic purpura, systemic lupus erythematosus, psoriasis, Sjogren's syndrome, insulin dependent diabetes mellitus, etc. In another embodiment, the disease is associated with abnormal vascularization (such as angiogenesis). In yet another embodiment, the disease is associated with dysregulation of growth factor-receptor signaling. In one example, said growth factor-receptor signaling is associated with a tyrosine kinase. In one example, said growth factor-receptor signaling is associated with the HGF-c-met axis.

An antibody of the invention is suitable for treating or preventing any of a number of pathological conditions resulting from any of a number of cellular, genetic and/or biochemical abnormalities. For example, an antibody of the invention is particularly suitable for treating and/or preventing pathological conditions associated with abnormalities within the HGF/c-met signaling pathway. In one embodiment, an antibody of the invention is a c-met antagonist. In one embodiment, the antibody is a chimeric antibody, for example, an antibody comprising antigen binding sequences from a non-human donor grafted to a heterologous non-human, human or humanized sequence (e.g., framework and/or constant domain sequences). In one embodiment, the non-human donor is a mouse. In one embodiment, an antigen binding sequence is synthetic, e.g. obtained by mutagenesis (e.g., phage display screening, etc.). In one embodiment, a chimeric antibody of the invention has murine V regions and human C region. In one embodiment, the murine light chain V region is fused to a human kappa light chain. In one embodiment, the murine heavy chain V region is fused to a human IgG1 C region. In one embodiment, the antigen binding sequences comprise at least one, at least two or all three CDRs of a light and/or heavy chain. In one embodiment, the antigen binding sequences comprise a heavy chain CDR3. In one embodiment, the antigen binding sequences comprise part or all of the CDR and/or variable domain sequences of the monoclonal antibody produced by the hybridoma cell line deposited under American Type Culture Collection Accession Number ATCC HB-11894 (hybridoma 1A3.3.13) or HB-11895 (hybridoma 5D5.11.6). In one embodiment, the antigen binding sequences comprise at least CDR3 of the heavy chain of the monoclonal antibody produced by the hybridoma cell line 1A3.3.13 or 5D5.11.6. Humanized antibodies of the invention include those that have amino acid substitutions in the FR and affinity maturation variants with changes in the grafted CDRs. The substituted amino acids in the CDR or FR are not limited to those present in the donor or recipient antibody. In other embodiments, the antibodies of the invention further comprise changes in amino acid residues in the Fc region that lead to improved effector function including enhanced CDC and/or ADCC function and B-cell killing. Other antibodies of the invention include those having specific changes that improve stability. Antibodies of the invention also include fucose deficient variants having improved ADCC function in vivo.

In one embodiment, an antibody fragment of the invention comprises an antigen binding arm comprising a heavy chain comprising at least one, at least two or all three of CDR sequences selected from the group consisting of SYWLH (SEQ ID NO:1), MIDPSNSDTRFNPNFKD (SEQ ID NO:2) and YGSYVSPLDY (SEQ ID NO:3). In one embodiment, the antigen binding arm comprises heavy chain CDR-H1 having amino acid sequence SYWLH. In one embodiment, the antigen binding arm comprises heavy chain CDR-H2 having amino acid sequence MIDPSNSDTRFNPNFKD. In one embodiment, the antigen binding arm comprises heavy chain CDR-H3 having amino acid sequence YGSYVSPLDY. In one embodiment, an antibody fragment of the invention comprises an antigen binding arm comprising a light chain comprising at least one, at least two or all three of CDR sequences selected from the group consisting of KSSQSLLYTSSQKNYLA (SEQ ID NO:4), WASTRES (SEQ ID NO:5) and QQYYAYPWT (SEQ ID NO:6). In one embodiment, the antigen binding arm comprises heavy chain CDR-L1 having amino acid sequence KSSQSLLYTSSQKNYLA. In one embodiment, the antigen binding arm comprises heavy chain CDR-L2 having amino acid sequence WASTRES. In one embodiment, the antigen binding arm comprises heavy chain CDR-L3 having amino acid sequence QQYYAYPWT. In one embodiment, an antibody fragment of the invention comprises an antigen binding arm comprising a heavy chain comprising at least one, at least two or all three of CDR sequences selected from the group consisting of SYWLH (SEQ ID NO:1), MIDPSNSDTRFNPNFKD (SEQ ID NO:2) and YGSYVSPLDY (SEQ ID NO:3) and a light chain comprising at least one, at least two or all three of CDR sequences selected from the group consisting of KSSQSLLYTSSQKNYLA (SEQ ID NO:4), WASTRES (SEQ ID NO:5) and QQYYAYPWT (SEQ ID NO:6).

The invention provides a humanized antibody that binds human c-met, or an antigen-binding fragment thereof, wherein the antibody is effective to inhibit HGF/c-met activity in vivo, the antibody comprising in the H chain Variable region ($V_H$) at least a CDR3 sequence of the monoclonal antibody produced by the hybridoma cell line deposited under American Type Culture Collection Accession Number ATCC HB-11894 (hybridoma 1A3.3.13) or HB-11895 (hybridoma 5D5.11.6) and substantially a human consensus sequence (e.g., substantially the human consensus framework (FR) residues of human heavy chain subgroup III ($V_H$III)). In one embodiment, the antibody further comprises the H chain CDR1 sequence and/or CDR2 sequence of the monoclonal antibody produced by the hybridoma cell line deposited under American Type Culture Collection Accession Number ATCC HB-11894 (hybridoma 1A3.3.13) or HB-11895 (hybridoma 5D5.11.6). In another embodiment, the preceding antibody comprises the L chain CDR1 sequence, CDR2 sequence and/or CDR3 sequence of the monoclonal antibody produced by the hybridoma cell line deposited under American Type Culture Collection Accession Number ATCC HB-11894 (hybridoma 1A3.3.13) or HB-11895 (hybridoma 5D5.11.6) with substantially the human consensus framework (FR) residues of human light chain κ subgroup I (VκI).

In one embodiment, an antibody fragment of the invention comprises an antigen binding arm comprising a heavy chain variable domain having the sequence:

QVQLQQSGPELVRPGASVKMSCRASGYTFTSYWLHWVKQRPGQGLE
WIGMIDPSNSDTRFNPNFKDKATLNVDRSSNTAYMLLSSLTSADSAVY
YCATYGSYVSPLDYWGQGTSVTVSS (SEQ ID NO:7)

In one embodiment, an antibody fragment of the invention comprises an antigen binding arm comprising a light chain variable domain having the sequence:

DIMMSQSPSSLTVSVGEKVTVSCKSSQSLLYTSSQKNYLAWYQQKPGQ
SPKLLIYWASTRESGVPDRFTGSGSGTDFTLTITSVKADDLAVYYCQQY
YAYPWTFGGGTKLEIK (SEQ ID NO:8)

In one aspect, the invention provides use of an antibody fragment of the invention (e.g., a c-met antagonist antibody fragment of the invention) in the preparation of a medicament for the therapeutic and/or prophylactic treatment of a disease, such as a cancer, a tumor, a cell proliferative disorder, an immune (such as autoimmune) disorder and/or

an angiogenesis-related disorder.

In one aspect, the invention provides use of a nucleic acid of the invention (e.g., a nucleic acid encoding a c-met antagonist antibody fragment of the invention) in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of an expression vector of the invention (e.g., a vector encoding a c-met antagonist antibody fragment of the invention) in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of a host cell of the invention (e.g., a host cell comprising a vector encoding a c-met antagonist antibody fragment of the invention) in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of an article of manufacture of the invention (e.g., an article of manufacture comprising a c-met antagonist antibody fragment of the invention and/or a nucleic acid encoding a c-met antagonist antibody fragment of the invention) in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of a kit of the invention (e.g., a kit comprising a c-met antagonist antibody fragment of the invention and/or a nucleic acid encoding a c-met antagonist antibody fragment of the invention) in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides a method of inhibiting c-met activated cell proliferation, said method comprising contacting a cell or tissue with an effective amount of a c-met antagonist antibody fragment of the invention, whereby cell proliferation associated with c-met activation is inhibited.

In one aspect, the invention provides a method of treating a pathological condition associated with dysregulation of c-met activation in a subject, said method comprising administering to the subject an effective amount of a c-met antagonist antibody fragment of the invention, whereby said condition is treated.

In one aspect, the invention provides a method of inhibiting the growth of a cell that expresses c-met or hepatocyte growth factor, or both, said method comprising contacting said cell with a c-met antagonist antibody fragment of the invention thereby causing an inhibition of growth of said cell. In one embodiment, the cell is contacted by HGF expressed by a different cell (e.g., through a paracrine effect).

In one aspect, the invention provides a method of therapeutically treating a mammal having a cancerous tumor comprising a cell that expresses c-met or hepatocyte growth factor, or both, said method comprising administering to said mammal an effective amount of a c-met antagonist antibody fragment of the invention, thereby effectively treating said mammal. In one embodiment, the cell is contacted by HGF expressed by a different cell (e.g., through a paracrine effect).

In one aspect, the invention provides a method for treating or preventing a cell proliferative disorder associated with increased expression or activity of c-met or hepatocyte growth, or both, said method comprising administering to a subject in need of such treatment an effective amount of a c-met antagonist antibody fragment of the invention, thereby effectively treating or preventing said cell proliferative disorder. In one embodiment, said proliferative disorder is cancer.

In one aspect, the invention provides a method for inhibiting the growth of a cell, wherein growth of said cell is at least in part dependent upon a growth potentiating effect of c-met or hepatocyte growth factor, or both, said method comprising contacting said cell with an effective amount of a c-met antagonist antibody fragment of the invention, thereby inhibiting the growth of said cell. In one embodiment, the cell is contacted by HGF expressed by a different cell (e.g., through a paracrine effect).

In one aspect, the invention provides a method of therapeutically treating a tumor in a mammal, wherein the growth of said tumor is at least in part dependent upon a growth potentiating effect of c-met or hepatocyte growth factor, or both, said method comprising contacting said cell with an effective amount of a c-met antagonist antibody fragment of the invention, thereby effectively treating said tumor. In one embodiment, the cell is contacted by HGF expressed by a different cell (e.g., through a paracrine effect).

Methods of the invention can be used to affect any suitable pathological state, for example, cells and/or tissues associated with dysregulation of the HGF/c-met signaling pathway. In one embodiment, a cell that is targeted in a method of the invention is a cancer cell. For example, a cancer cell can be one selected from the group consisting of a breast cancer cell, a colorectal cancer cell, a lung cancer cell, a papillary carcinoma cell (e.g., of the thyroid gland), a colon cancer cell, a pancreatic cancer cell, a prostate cancer cell, an ovarian cancer cell, a cervical cancer cell, a central nervous system cancer cell, an osteogenic sarcoma cell, a renal carcinoma cell, a hepatocellular carcinoma cell, a bladder cancer cell, a gastric carcinoma cell, a head and neck squamous carcinoma cell, a melanoma cell, a lymphoma

cell, a myeloma cell (e.g., multiple myeloma), a glioma/glioblastoma cell (e.g., anaplastic astrocytoma, glioblastoma multiforme, anaplastic oligodendroglioma, anaplastic oligodendroastrocytoma), and a leukemia cell. In one embodiment, a cell that is targeted in a method of the invention is a hyperproliferative and/or hyperplastic cell. In one embodiment, a cell that is targeted in a method of the invention is a dysplastic cell. In yet another embodiment, a cell that is targeted in a method of the invention is a metastatic cell.

Methods of the invention can further comprise additional treatment steps. For example, in one embodiment, a method further comprises a step wherein a targeted cell and/or tissue (e.g., a cancer cell) is exposed to radiation treatment or a chemotherapeutic agent.

Activation of c-met is an important biological process the dysregulation of which leads to numerous pathological conditions. Accordingly, in one embodiment of methods of the invention, a cell that is targeted (e.g., a cancer cell) is one in which activation of c-met is enhanced as compared to a normal cell of the same tissue origin. In one embodiment, a method of the invention causes the death of a targeted cell. For example, contact with an antagonist antibody fragment of the invention may result in a cell's inability to signal through the c-met pathway, which results in cell death.

Dysregulation of c-met activation (and thus signaling) can result from a number of cellular changes, including, for example, overexpression of HGF (c-met's cognate ligand) and/or c-met itself. Accordingly, in some embodiments, a method of the invention comprises targeting a cell wherein c-met or hepatoctye growth factor, or both, is more abundantly expressed by said cell (e.g., a cancer cell) as compared to a normal cell of the same tissue origin. A c-met-expressing cell can be regulated by HGF from a variety of sources, i.e. in an autocrine or paracrine manner. For example, in one embodiment of methods of the invention, a targeted cell is contacted/bound by hepatocyte growth factor expressed in a different cell (e.g., via a paracrine effect). Said different cell can be of the same or of a different tissue origin. In one embodiment, a targeted cell is contacted/bound by HGF expressed by the targeted cell itself (e.g., via an autocrine effect/loop). In one embodiment, c-met activity (or activation) in a targeted cell is ligand dependent. In one embodiment, c-met activity (or activation) is ligand independent.

## BRIEF DESCRIPTION OF THE DRAWINGS

FIGURE 1 shows anti-Fab Western blot results for anti-c-met Fab/c antibody (one-armed antibody) expression.

FIGURE 2 shows anti-Fc Western blot results for anti-c-met Fab/c antibody (one-armed antibody) expression.

FIGURE 3 shows anti-Fab Western blot results for expression of anti-c-met Fab/c antibody (one-armed antibody) comprising a protuberance and cavity in the Fc region.

FIGURE 4 shows anti-Fc Western blot results for expression of anti-c-met Fab/c antibody (one-armed antibody) comprising a protuberance and cavity in the Fc region.

FIGURE 5 shows results of a competitive binding assay wherein one-armed anti-c-met antibody blocked HGF binding to c-met.

FIGURE 6 shows results of a KIRA assay in U87 cells treated with or without HGF and/or anti-c-met 5D5 one-armed antibody.

FIGURE 7 shows cell proliferation of BaF3-hMet cells in the presence of varying amounts of anti-c-met 5D5 antibody.

FIGURE 8 shows a cell migration assay wherein one-armed anti-c-met antibody blocked HGF function.

FIGURES 9A-B show results of pharmacokinetics analysis of one-armed anti-c-met antibody.

FIGURES 10A-B show results of treatment of tumors with one-armed anti-c-met antibody. In Figure 10B, "OA" indicates one-armed.

## MODES FOR CARRYING OUT THE INVENTION

The invention provides methods, compositions, kits and articles of manufacture for using monovalent antibody fragments having unique characteristics that render them particularly advantageous for use in treating certain pathological conditions. Moreover, the antibody fragments can be readily prepared with pragmatic yields and desirable purity. Antibody fragments of the invention are characterized by superior physicochemical and/or therapeutic capabilities as compared to existing monovalent antibodies. In general, monovalent antibody fragments of the invention comprise a single antigen binding arm and an Fc region, wherein the antibody fragment exhibits enhanced stability *in vivo* compared to a Fab antibody fragment comprising said antigen binding arm but lacking said Fc region. In some embodiments, an antibody fragment of the invention comprises an alteration in one or more residues of each of the Fc sequences that form the multimerization interface between the Fc polypeptides that make up the Fc region. The invention provides methods of making and using antibody fragments of the invention. The invention makes possible the efficient and commercially-viable production of novel antibody fragments of the invention. The antibody fragments can be used for treating pathological conditions in which use of a therapeutic antibody that is monovalent in nature and highly stable is highly desirable

and/or required. Details of methods, compositions, kits and articles of manufacture of the invention are provided herein.

*General Techniques*

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., ed., 1994); "A Practical Guide to Molecular Cloning" (Perbal Bernard V., 1988).

*Definitions*

The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "recombinant vectors"). In general, expression vectors of utility in recombinant DNA techniques are often in the form of plasmids. In the present specification, "plasmid" and "vector" may be used interchangeably as the plasmid is the most commonly used form of vector.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, alpha.-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR.sub.2 ("amidate"), P(O)R, P(O)OR', CO or CH.sub.2 ("formacetal"), in which each R or R' is independently H or substituted or unsubstituted alkyl (1-20 C.) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single stranded, generally synthetic polynucleotides that are generally, but not necessarily, less than about 200 nucleotides in length. The terms "oligonucleotide" and "polynucleotide" are not mutually exclusive The description above for polynucleotides is equally and fully applicable to oligonucleotides.

The term "hepatocyte growth factor" or "HGF", as used herein, refers, unless specifically or contextually indicated

otherwise, to any native or variant (whether native/naturally occurring or synthetic) HGF polypeptide that is capable of activating the HGF/c-met signaling pathway under conditions that permit such process to occur. The term "wild type HGF" generally refers to a polypeptide comprising the amino acid sequence of a naturally occurring HGF protein. The term "wild type HGF sequence" generally refers to an amino acid sequence found in a naturally occurring HGF.

The terms "antibody" and "immunoglobulin" are used interchangeably in the broadest sense and include monoclonal antibodies (e.g., full length or intact monoclonal antibodies), polyclonal antibodies, monovalent antibodies, multivalent antibodies, multispecific antibodies (*e.g.*, bispecific antibodies so long as they exhibit the desired biological activity) and antibody fragments as described herein. An antibody can be human, humanized and/or affinity matured.

"Antibody fragments" comprise only a portion of an intact antibody, wherein the portion preferably retains at least one, preferably most or all, of the functions normally associated with that portion when present in an intact antibody.

The phrase "antigen binding arm", as used herein, refers to a component part of an antibody fragment of the invention that has an ability to specifically bind a target molecule of interest. Generally and preferably, the antigen binding arm is a complex of immunoglobulin polypeptide sequences, e.g., CDR and/or variable domain sequences of an immunoglobulin light and heavy chain.

The phrase "N-terminally truncated heavy chain", as used herein, refers to a polypeptide comprising parts but not all of a full length immunoglobulin heavy chain, wherein the missing parts are those normally located on the N terminal region of the heavy chain. Missing parts may include, but are not limited to, the variable domain, CH1, and part or all of a hinge sequence. Generally, if the wild type hinge sequence is not present, the remaining constant domain(s) in the N-terminally truncated heavy chain would comprise a component that is capable of linkage to another Fc sequence (i.e., the "first" Fc polypeptide as described herein). For example, said component can be a modified residue or an added cysteine residue capable of forming a disulfide linkage.

The term "monoclonal antibody" as used herein refers to an antibody obtained from a population of substantially homogeneous antibodies, *i.e.*, the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (*e.g.*, murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

An "affinity matured" antibody is one with one or more alterations in one or more CDRs thereof which result in an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995);

Yelton et al. J. Immunol. 155:1994-2004 (1995); Jackson et al., J. Immunol. 154(7):3310-9 (1995); and Hawkins et al, J. Mol. Biol. 226:889-896 (1992).

As used herein, the term "immunoadhesin" designates antibody-like molecules which combine the "binding domain" of a heterologous protein (an "adhesin", *e.g.* a receptor, ligand or enzyme) with the effector component of immunoglobulin constant domains. Structurally, the immunoadhesins comprise a fusion of the adhesin amino acid sequence with the desired binding specificity which is other than the antigen recognition and binding site (antigen combining site) of an antibody (*i.e.* is "heterologous") and an immunoglobulin constant domain sequence. The immunoglobulin constant domain sequence in the immunoadhesin may be obtained from any immunoglobulin, such as $IgG_1$, $IgG_2$, $IgG_3$, or $IgG_4$ subtypes, IgA, IgE, IgD or IgM.

A "heteromultimer", "heteromultimeric complex", or "heteromultimeric polypeptide" is a molecule comprising at least a first polypeptide and a second polypeptide, wherein the second polypeptide differs in amino acid sequence from the first polypeptide by at least one amino acid residue. The heteromultimer can comprise a "heterodimer" formed by the first and second polypeptide or can form higher order tertiary structures where polypeptides in addition to the first and second polypeptide are present.

As used herein, "polypeptide" refers generally to peptides and proteins having more than about ten amino acids.

The phrase "immunosuppressive properties", or variants thereof, as used herein refers to properties of an antibody that directly or indirectly result in inhibition of one or more normal activities and/or functions involving the immune system, including but not limited to humoral and cell-mediated immunity.

The term "Fc region", as used herein, generally refers to a dimer complex comprising the C-terminal polypeptide sequences of an immunoglobulin heavy chain, wherein a C-terminal polypeptide sequence is that which is obtainable by papain digestion of an intact antibody. The Fc region may comprise native or variant Fc sequences. Although the boundaries of the Fc sequence of an immunoglobulin heavy chain might vary, the human IgG heavy chain Fc sequence is usually defined to stretch from an amino acid residue at about position Cys226, or from about position Pro230, to the carboxyl terminus of the Fc sequence. The Fc sequence of an immunoglobulin generally comprises two constant domains, a CH2 domain and a CH3 domain, and optionally comprises a CH4 domain. By "Fc polypeptide" herein is meant one of the polypeptides that make up an Fc region. An Fc polypeptide may be obtained from any suitable immunoglobulin, such as $IgG_1$, $IgG_2$, $IgG_3$, or $IgG_4$ subtypes, IgA, IgE, IgD or IgM. In some embodiments, an Fc polypeptide comprises part or all of a wild type hinge sequence (generally at its N terminus). In some embodiments, an Fc polypeptide does not comprise a functional or wild type hinge sequence.

"Antibody-dependent cell-mediated cytotoxicity" and "ADCC" refer to a cell-mediated reaction in which nonspecific cytotoxic cells that express Fc receptors (FcRs) (*e.g.* Natural Killer (NK) cells, neutrophils, and macrophages) recognize bound antibody on a target cell and subsequently cause lysis of the target cell.

The terms "Fc receptor" and "FcR" are used to describe a receptor that binds to the Fc region of an antibody. For example, an FcR can be a native sequence human FcR. Generally, an FcR is one which binds an IgG antibody (a gamma receptor) and includes receptors of the Fc(RI, Fc(RII, and Fc(RIII subclasses, including allelic variants and alternatively spliced forms of these receptors. Fc(RII receptors include Fc(RIIA (an "activating receptor") and Fc(RIIB (an "inhibiting receptor"), which have similar amino acid sequences that differ primarily in the cytoplasmic domains thereof. Immunoglobulins of other isotypes can also be bound by certain FcRs (see, e.g., Janeway et al., Immuno Biology: the immune system in health and disease, (Elsevier Science Ltd., NY) (4th ed., 1999)). Activating receptor Fc(RIIA contains an immunoreceptor tyrosine-based activation motif (ITAM) in its cytoplasmic domain. Inhibiting receptor Fc (RIIB contains an immunoreceptor tyrosine-based inhibition motif (ITIM) in its cytoplasmic domain (reviewed in Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976); and Kim et al., J. Immunol. 24:249 (1994)).

The "hinge region," "hinge sequence", and variations thereof, as used herein, includes the meaning known in the art, which is illustrated in, for example, Janeway et al., Immuno Biology: the immune system in health and disease, (Elsevier Science Ltd., NY) (4th ed., 1999); Bloom et al., Protein Science (1997), 6:407-415; Humphreys et al., J. Immunol. Methods (1997), 209:193-202.

The term "cistron," as used herein, is intended to refer to a genetic element broadly equivalent to a translational unit comprising the nucleotide sequence coding for a polypeptide chain and adjacent control regions. "Adjacent control regions" include, for example, a translational initiation region (TIR; as defined herein below) and a termination region.

The "translation initiation region" or TIR, as used herein refers to a nucleic acid region providing the efficiency of translational initiation of a gene of interest. In general, a TIR within a particular cistron encompasses the ribosome binding site (RBS) and sequences 5' and 3' to RBS. The RBS is defined to contain, minimally, the Shine-Dalgarno region and the start codon (AUG). Accordingly, a TIR also includes at least a portion of the nucleic acid sequence to be translated. In some embodiments, a TIR of the invention includes a secretion signal sequence encoding a signal peptide that

precedes the sequence coding for the light or heavy chain within a cistron. A TIR variant contains sequence variants (particularly substitutions) within the TIR region that alter the property of the TIR, such as its translational strength as defined herein below. Preferably, a TIR variant of the invention contains sequence substitutions within the first 2 to about 14, preferably about 4 to 12, more preferably about 6 codons of the secretion signal sequence that precedes the sequence coding for the light or heavy chain within a cistron.

The term "translational strength" as used herein refers to a measurement of a secreted polypeptide in a control system wherein one or more variants of a TIR is used to direct secretion of a polypeptide and the results compared to the wild-type TIR or some other control under the same culture and assay conditions. Without being limited to any one theory, "translational strength" as used herein can include, for example, a measure of mRNA stability, efficiency of ribosome binding to the ribosome binding site, and mode of translocation across a membrane.

"Secretion signal sequence" or "signal sequence" refers to a nucleic acid sequence coding for a short signal peptide that can be used to direct a newly synthesized protein of interest through a cellular membrane, for example the inner membrane or both inner and outer membranes of prokaryotes. As such, the protein of interest such as the immunoglobulin light or heavy chain polypeptide may be secreted into the periplasm of prokaryotic host cells or into the culture medium. The signal peptide encoded by the secretion signal sequence may be endogenous to the host cells, or they may be exogenous, including signal peptides native to the polypeptide to be expressed. Secretion signal sequences are typically present at the amino terminus of a polypeptide to be expressed, and are typically removed enzymatically between biosynthesis and secretion of the polypeptide from the cytoplasm. Thus, the signal peptide is usually not present in a mature protein product.

A "blocking" antibody or an "antagonist" antibody is one which inhibits or reduces biological activity of the antigen it binds (e.g., c-met and VEGF receptor).

An "agonist antibody", as used herein, is an antibody which mimics at least one of the functional activities of a polypeptide of interest (e.g., HGF and VEGF).

A "tumor antigen," as used herein, includes the meaning known in the art, which includes any molecule that is differentially expressed on a tumor cell compared to a normal cell. In some embodiments, the molecule is expressed at a detectably or significantly higher or lower level in a tumor cell compared to a normal cell. In some embodiments, the molecule exhibits a detectably or significantly higher or lower level of biological activity in a tumor cell compared to a normal cell. In some embodiments, the molecule is known or thought to contribute to a tumorigenic characteristic of the tumor cell. Numerous tumor antigens are known in the art. Whether a molecule is a tumor antigen can also be determined according to techniques and assays well known to those skilled in the art, such as for example clonogenic assays, transformation assays, in vitro or in vivo tumor formation assays, gel migration assays, gene knockout analysis, etc.

A "disorder" is any condition that would benefit from treatment with an antibody or method of the invention. This includes chronic and acute disorders or diseases including those pathological conditions which predispose the mammal to the disorder in question. Non-limiting examples of disorders to be treated herein include malignant and benign tumors; non-leukemias and lymphoid malignancies; neuronal, glial, astrocytal, hypothalamic and other glandular, macrophagal, epithelial, stromal and blastocoelic disorders; and inflammatory, immunologic and other angiogenesis-related disorders.

The terms "cancer" and "cancerous" refer to or describe the physiological condition in mammals that is typically characterized by unregulated cell growth/proliferation. Examples of cancer include but are not limited to, carcinoma, lymphoma, blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, myeloma (e.g., multiple myeloma), hepatocellular cancer, gastrointestinal cancer, pancreatic cancer, glioblastoma/glioma (e.g., anaplastic astrocytoma, glioblastoma multiforme, anaplastic oligodendroglioma, anaplastic oligodendroastrocytoma), cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma and various types of head and neck cancer.

An "autoimmune disease" herein is a non-malignant disease or disorder arising from and directed against an individual's own tissues. The autoimmune diseases herein specifically exclude malignant or cancerous diseases or conditions, especially excluding B cell lymphoma, acute lymphoblastic leukemia (ALL), chronic lymphocytic leukemia (CLL), Hairy cell leukemia and chronic myeloblastic leukemia. Examples of autoimmune diseases or disorders include, but are not limited to, inflammatory responses such as inflammatory skin diseases including psoriasis and dermatitis (*e.g.* atopic dermatitis); systemic scleroderma and sclerosis; responses associated with inflammatory bowel disease (such as Crohn's disease and ulcerative colitis); respiratory distress syndrome (including adult respiratory distress syndrome; ARDS); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus (*e.g.* Type I diabetes mellitus or insulin dependent diabetes mellitis); multiple sclerosis; Reynaud's syndrome; autoimmune thyroiditis; allergic encephalomyelitis; Sjorgen's syndrome; juvenile onset diabetes; and immune responses associated

with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia (including, but not limited to cryoglobinemia or Coombs positive anemia) ; myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia etc.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or disorder. In one embodiment, antibodies and methods of the invention effect tumor regression. In one embodiment, antibodies and methods of the invention effect inhibition of tumor/cancer growth.

An "effective amount" refers to an amount effective, at dosages and for periods of time necessary, to achieve the desired therapeutic or prophylactic result. A "therapeutically effective amount" of an antibody of the invention may vary according to factors such as the disease state, age sex, and weight of the individual, and the ability of the antibody to elicit a desired response in the individual. A therapeutically effective amount is also one in which any toxic or detrimental effects of the antibody are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The phrase "does not possess substantial effector function" with respect to an antibody fragment of the invention, as used herein, means the difference between the amount of detectable effector function activity of an antibody fragment of the invention and the amount of the activity exhibited by a wild type glycosylated counterpart of the antibody is statistically significant as evident to one skilled in the art, wherein the amount of activity of the antibody fragment of the invention is lower than the amount of activity exhibited by the wild type counterpart. In one embodiment, an antibody fragment of the invention does not exhibit an effector function activity level (other than FcRn binding) that is above background level that is of statistical significance. The phrase "little to no immunosuppressive properties" with respect to an antibody fragment of the invention, as used herein, means the antibody does not elicit a biologically meaningful amount of immunosuppression upon administration to a subject. As would be understood in the art, amount of an activity may be determined quantitatively or qualitatively, so long as a comparison between an antibody of the invention and a reference counterpart can be done. The activity can be measured or detected according to any assay or technique known in the art, including, e.g., those described herein. The amount of activity for an antibody of the invention and its reference counterpart can be determined in parallel or in separate runs.

The phrase "substantially similar", "substantially identical", "substantially the same", and variations thereof, as used herein, denotes a sufficiently high degree of similarity between two numeric values (generally one associated with an antibody of the invention and the other associated with its reference counterpart) such that one of skill in the art would consider the difference between the two values to be of little or no biological significance within the context of the biological, physical or quantitation characteristic measured by said values. The difference between said two values is preferably less than about 50%, preferably less than about 40%, preferably less than about 30%, preferably less than about 20%, preferably less than about 10% as a function of the value for the reference counterpart.

An antibody fragment of the invention is "more stable" or has "increased stability" compared to another antibody form (such as a Fab fragment counterpart), and variations thereof, as used herein, means the antibody fragment of the invention exhibits a detectable/measurable increase in stability *in vivo* compared to a reference antibody (such as a Fab fragment counterpart). Stability can be based on half life, clearance rate and/or any other parameter viewed in the art as indicative of how much of the antibody fragment of the invention remains in a subject at particular timepoints following administration of the antibody fragment to the subject. Methods of determining stability parameters, such as half life and/or clearance rate, are well known in the art, some of which are described herein.

"Complement dependent cytotoxicity" and "CDC" refer to the lysing of a target in the presence of complement. The complement activation pathway is initiated by the binding of the first component of the complement system (C1q) to a molecule (*e.g.* an antibody) complexed with a cognate antigen.

"Binding affinity" generally refers to the strength of the sum total of noncovalent interactions between a single binding site of a molecule (e.g., an antibody) and its binding partner (e.g., an antigen or FcRn receptor). The affinity of a molecule X for its partner Y can generally be represented by the dissociation constant (Kd). Affinity can be measured by common methods known in the art, including those described herein. Low-affinity antibodies bind antigen (or FcRn

receptor) weakly and tend to dissociate readily, whereas high-affinity antibodies bind antigen (or FcRn receptor) more tightly and remain bound longer.

The term "cytotoxic agent" as used herein refers to a substance that inhibits or prevents the function of cells and/or causes destruction of cells. The term is intended to include radioactive isotopes (*e.g* $At^{211}$, $I^{131}$, $I^{125}$, $Y^{90}$, $Re^{186}$, $Re^{188}$, $Sm^{153}$, $Bi^{212}$ > $P^{32}$ and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma 1I and calicheamicin omegaI1 (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HC1 liposome injection (DOXIL®) and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., paclitaxel (TAXOL®), albumin-engineered nanoparticle formulation of paclitaxel (ABRAXANE™), and doxetaxel (TAXOTERE®); chloranbucil; 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluorometlhylornithine (DMFO); retinoids such as retinoic acid; pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX® tamoxifen), raloxifene (EVISTA®), droloxifene, 4-hydroxytamoxifen, trioxifene, keoxifene, LY117018, onapristone, and toremifene (FARESTON®); anti-progesterones; estrogen receptor down-regulators (ERDs); estrogen receptor antagonists such as fulvestrant (FASLODEX®); agents that function to suppress or shut down the ovaries, for example, leutinizing hormone-releasing hormone (LHRH) agonists such as leuprolide acetate (LUPRON® and ELIGARD®), goserelin acetate, buserelin acetate and tripterelin; other anti-androgens such as flutamide, nilutamide and bicalutamide; and aromatase inhibitors that inhibit the enzyme aromatase,

which regulates estrogen production in the adrenal glands, such as, for example, 4(5)-imidazoles, aminoglutethimide, megestrol acetate (MEGASE®), exemestane (AROMASIN®), formestanie, fadrozole, vorozole (RIVISOR®), letrozole (FEMARA®), and anastrozole (ARIMIDEX®). In addition, such definition of chemotherapeutic agents includes bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor (e.g., LURTOTECAN®); rmRH (e.g., ABARELIX®); lapatinib ditosylate (an ErbB-2 and EGFR dual tyrosine kinase small-molecule inhibitor also known as GW572016); COX-2 inhibitors such as celecoxib (CELEBREX®; 4-(5-(4-methylphenyl)-3-(trifluoromethyl)-1H-pyrazol-1-yl) benzenesulfonamide; and pharmaceutically acceptable salts, acids or derivatives of any of the above.

Except where indicated otherwise by context, the terms "first" polypeptide and "second" polypeptide, and variations thereof, are merely generic identifiers, and are not to be taken as identifying specific polypeptides or components of antibodies of the invention.

A "protuberance" refers to at least one amino acid side chain which projects from the interface of a first polypeptide and is therefore positionable in a compensatory cavity in the adjacent interface (*i.e.* the interface of a second polypeptide) so as to stabilize the heteromultimer, and thereby favor heteromultimer formation over homomultimer formation, for example. The protuberance may exist in the original interface or may be introduced synthetically (*e.g.* by altering nucleic acid encoding the interface). Normally, nucleic acid encoding the interface of the first polypeptide is altered to encode the protuberance. To achieve this, the nucleic acid encoding at least one "original" amino acid residue in the interface of the first polypeptide is replaced with nucleic acid encoding at least one "import" amino acid residue which has a larger side chain volume than the original amino acid residue. It will be appreciated that there can be more than one original and corresponding import residue. The upper limit for the number of original residues which are replaced is the total number of residues in the interface of the first polypeptide. The side chain volumes of the various amino residues are shown in the following table.

**TABLE 1**

| Properties of Amino Acid Residues | | | | |
|---|---|---|---|---|
| **Amino Acid** | **One-Letter Abbreviation** | **MASS[a] (daltons)** | **VOLUME[b] (Angstrom$^3$)** | **Accessible Surface Area[c] (Angstrom$^2$)** |
| Alanine (Ala) | A | 71.08 | 88.6 | 115 |
| Arginine (Arg) | R | 156.20 | 173.4 | 225 |
| Asparagine (Asn) | N | 114.11 | 117.7 | 160 |
| Aspartic acid (Asp) | D | 115.09 | 111.1 | 150 |
| Cysteine (Cys) | C | 103.14 | 108.5 | 135 |
| Glutamine (Gln) | Q | 128.14 | 143.9 | 180 |
| Glutamic acid (Glu) | E | 129.12 | 138.4 | 190 |
| Glycine (Gly) | G | 57.06 | 60.1 | 75 |
| Histidine (His) | H | 137.15 | 153.2 | 195 |
| Isoleucine (Ile) | I | 113.17 | 166.7 | 175 |
| Leucine (Leu) | L | 113.17 | 166.7 | 170 |
| Lysine (Lys) | K | 128.18 | 168.6 | 200 |
| Methionine (Met) | M | 131.21 | 162.9 | 185 |
| Phenylalinine (Phe) | F | 147.18 | 189.9 | 210 |
| Proline (Pro) | P | 97.12 | 122.7 | 145 |
| Serine (Ser) | S | 87.08 | 89.0 | 115 |
| Threonine (Thr) | T | 101.11 | 116.1 | 140 |

(continued)

| Properties of Amino Acid Residues | | | | |
|---|---|---|---|---|
| **Amino Acid** | **One-Letter Abbreviation** | **MASS[a] (daltons)** | **VOLUME[b] (Angstrom$^3$)** | **Accessible Surface Area[c] (Angstrom$^2$)** |
| Tryptophan (Trp) | W | 186.21 | 227.8 | 255 |
| Tyrosine (Tyr) | Y | 163.18 | 193.6 | 230 |
| Valine (Val) | V | 99.14 | 140.0 | 155 |

[a] a Molecular weight amino acid minus that of water. Values from Handbook of Chemistry and Physics, 43rd ed. Cleveland, Chemical Rubber Publishing Co., 1961.
[b] Values from A.A. Zamyatnin, Prog. Biophys. Mol. Biol. 24:107-123, 1972.
[c] Values from C. Chothia, J. Mol. Biol. 105:1-14, 1975. The accessible surface area is defined in Figures 6-20 of this reference.

The preferred import residues for the formation of a protuberance are generally naturally occurring amino acid residues and are preferably selected from arginine (R), phenylalanine (F), tyrosine (Y) and tryptophan (W). Most preferred are tryptophan and tyrosine. In one embodiment, the original residue for the formation of the protuberance has a small side chain volume, such as alanine, asparagine, aspartic acid, glycine, serine, threonine or valine.

A "cavity" refers to at least one amino acid side chain which is recessed from the interface of a second polypeptide and therefore accommodates a corresponding protuberance on the adjacent interface of a first polypeptide. The cavity may exist in the original interface or may be introduced synthetically (*e.g.* by altering nucleic acid encoding the interface). Normally, nucleic acid encoding the interface of the second polypeptide is altered to encode the cavity. To achieve this, the nucleic acid encoding at least one "original" amino acid residue in the interface of the second polypeptide is replaced with DNA encoding at least one "import" amino acid residue which has a smaller side chain volume than the original amino acid residue. It will be appreciated that there can be more than one original and corresponding import residue. The upper limit for the number of original residues which are replaced is the total number of residues in the interface of the second polypeptide. The side chain volumes of the various amino residues are shown in Table 1 above. The preferred import residues for the formation of a cavity are usually naturally occurring amino acid residues and are preferably selected from alanine (A), serine (S), threonine (T) and valine (V). Most preferred are serine, alanine or threonine. In one embodiment, the original residue for the formation of the cavity has a large side chain volume, such as tyrosine, arginine, phenylalanine or tryptophan.

An "original" amino acid residue is one which is replaced by an "import" residue which can have a smaller or larger side chain volume than the original residue. The import amino acid residue can be a naturally occurring or non-naturally occurring amino acid residue, but preferably is the former. "Naturally occurring" amino acid residues are those residues encoded by the genetic code and listed in Table 1 above. By "non-naturally occurring" amino acid residue is meant a residue which is not encoded by the genetic code, but which is able to covalently bind adjacent amino acid residue(s) in the polypeptide chain. Examples of non-naturally occurring amino acid residues are norleucine, ornithine, norvaline, homoserine and other amino acid residue analogues such as those described in Ellman et al., Meth. Enzym. 202:301-336 (1991), for example. To generate such non-naturally occurring amino acid residues, the procedures of Noren et al. Science 244: 182 (1989) and Ellman et al., *supra* can be used. Briefly, this involves chemically activating a suppressor tRNA with a non-naturally occurring amino acid residue followed by *in vitro* transcription and translation of the RNA. The method of the instant invention involves replacing at least one original amino acid residue, but more than one original residue can be replaced. Normally, no more than the total residues in the interface of the first or second polypeptide will comprise original amino acid residues which are replaced. Typically, original residues for replacement are "buried". By "buried" is meant that the residue is essentially inaccessible to solvent. Generally, the import residue is not cysteine to prevent possible oxidation or mispairing of disulfide bonds.

The protuberance is "positionable" in the cavity which means that the spatial location of the protuberance and cavity on the interface of a first polypeptide and second polypeptide respectively and the sizes of the protuberance and cavity are such that the protuberance can be located in the cavity without significantly perturbing the normal association of the first and second polypeptides at the interface. Since protuberances such as Tyr, Phe and Trp do not typically extend perpendicularly from the axis of the interface and have preferred conformations, the alignment of a protuberance with a corresponding cavity relies on modeling the protuberance/cavity pair based upon a three-dimensional structure such as that obtained by X-ray crystallography or nuclear magnetic resonance (NMR). This can be achieved using widely accepted techniques in the art.

By "original or template nucleic acid" is meant the nucleic acid encoding a polypeptide of interest which can

be "altered" (*i.e.* genetically engineered or mutated) to encode a protuberance or cavity. The original or starting nucleic acid may be a naturally occurring nucleic acid or may comprise a nucleic acid which has been subjected to prior alteration (*e.g.* a humanized antibody fragment). By "altering" the nucleic acid is meant that the original nucleic acid is mutated by inserting, deleting or replacing at least one codon encoding an amino acid residue of interest. Normally, a codon encoding an original residue is replaced by a codon encoding an import residue. Techniques for genetically modifying a DNA in this manner have been reviewed in Mutagenesis: a Practical Approach, M.J. McPherson, Ed., (IRL Press, Oxford, UK. (1991), and include site-directed mutagenesis, cassette mutagenesis and polymerase chain reaction (PCR) mutagenesis, for example. By mutating an original/template nucleic acid, an original/template polypeptide encoded by the original/template nucleic acid is thus correspondingly altered.

The protuberance or cavity can be "introduced" into the interface of a first or second polypeptide by synthetic means, *e.g.* by recombinant techniques, *in vitro* peptide synthesis, those techniques for introducing non-naturally occurring amino acid residues previously described, by enzymatic or chemical coupling of peptides or some combination of these techniques. Accordingly, the protuberance or cavity which is "introduced" is "non-naturally occurring" or "non-native", which means that it does not exist in nature or in the original polypeptide (*e.g.* a humanized monoclonal antibody).

Generally, the import amino acid residue for forming the protuberance has a relatively small number of "rotamers" (*e.g.* about 3-6). A "rotomer" is an energetically favorable conformation of an amino acid side chain. The number of rotomers of the various amino acid residues are reviewed in Ponders and Richards, J. Mol. Biol. 193: 775-791 (1987).

"Isolated" heteromultimer means heteromultimer which has been identified and separated and/or recovered from a component of its natural cell culture environment. Contaminant components of its natural environment are materials which would interfere with diagnostic or therapeutic uses for the heteromultimer, and may include enzymes, hormones, and other proteinaceous or nonproteinaceous solutes. In some embodiments, the heteromultimer will be purified (1) to greater than 95% by weight of protein as determined by the Lowry method, or more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or silver stain.

The heteromultimers of the present invention are generally purified to substantial homogeneity. The phrases "substantially homogeneous", "substantially homogeneous form" and "substantial homogeneity" are used to indicate that the product is substantially devoid of by-products originated from undesired polypeptide combinations (*e.g.* homomultimers). Expressed in terms of purity, substantial homogeneity means that the amount of by-products does not exceed 10%, or is below 5%, or is below 1%, or is below 0.5%, wherein the percentages are by weight.

The expression "control sequences" refers to DNA sequences necessary for the expression of an operably linked coding sequence in a particular host organism. The control sequences that are suitable for prokaryotes, for example, include a promoter, optionally an operator sequence, a ribosome binding site, and possibly, other as yet poorly understood sequences. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However, enhancers do not have to be contiguous. Linking can be accomplished by ligation at convenient restriction sites. If such sites do not exist, the synthetic oligonucleotide adaptors or linkers are used in accord with conventional practice.

Vectors, Host Cells and Recombinant Methods

For recombinant production of an antibody of the invention, the nucleic acid encoding it is isolated and inserted into a replicable vector for further cloning (amplification of the DNA) or for expression. DNA encoding the antibody is readily isolated and sequenced using conventional procedures (*e.g.*, by using oligonucleotide probes that are capable of binding specifically to genes encoding the heavy and light chains of the antibody). Many vectors are available. The choice of vector depends in part on the host cell to be used. Generally, preferred host cells are of either prokaryotic or eukaryotic (generally mammalian) origin.

*Generating antibodies using prokaryotic host cells:*

*Vector Construction*

Polynucleotide sequences encoding polypeptide components of the antibody of the invention can be obtained using standard recombinant techniques. Desired polynucleotide sequences may be isolated and sequenced from antibody producing cells such as hybridoma cells. Alternatively, polynucleotides can be synthesized using nucleotide synthesizer or PCR techniques. Once obtained, sequences encoding the polypeptides are inserted into a recombinant vector capable of replicating and expressing heterologous polynucleotides in prokaryotic hosts. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication, a selection marker gene, a promoter, a ribosome binding site (RBS), a signal sequence, the heterologous nucleic acid insert and a transcription termination sequence.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, *E. coli* is typically transformed using pBR322, a plasmid derived from an *E. coli* species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins. Examples of pBR322 derivatives used for expression of particular antibodies are described in detail in Carter et al., U.S. Patent No. 5,648,237.

In addition, phage vectors containing replicon and control sequences that are compatible with the host microorganism can be used as transforming vectors in connection with these hosts. For example, bacteriophage such as λGEM.TM.-11 may be utilized in making a recombinant vector which can be used to transform susceptible host cells such as *E. coli* LE392.

The expression vector of the invention may comprise two or more promoter-cistron pairs, encoding each of the polypeptide components. A promoter is an untranslated regulatory sequence located upstream (5') to a cistron that modulates its expression. Prokaryotic promoters typically fall into two classes, inducible and constitutive. Inducible promoter is a promoter that initiates increased levels of transcription of the cistron under its control in response to changes in the culture condition, e.g. the presence or absence of a nutrient or a change in temperature.

A large number of promoters recognized by a variety of potential host cells are well known. The selected promoter can be operably linked to cistron DNA encoding the light or heavy chain by removing the promoter from the source DNA via restriction enzyme digestion and inserting the isolated promoter sequence into the vector of the invention. Both the native promoter sequence and many heterologous promoters may be used to direct amplification and/or expression of the target genes. In some embodiments, heterologous promoters are utilized, as they generally permit greater transcription and higher yields of expressed target gene as compared to the native target polypeptide promoter.

Promoters suitable for use with prokaryotic hosts include the PhoA promoter, the β-galactamase and lactose promoter systems, a tryptophan (trp) promoter system and hybrid promoters such as the *tac* or the *trc* promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al. (1980) Cell 20: 269) using linkers or adaptors to supply any required restriction sites.

In one aspect of the invention, each cistron within the recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected for the purpose of this invention should be one that is recognized and processed (i.e. cleaved by a signal peptidase) by the host cell. For prokaryotic host cells that do not recognize and process the signal sequences native to the heterologous polypeptides, the signal sequence is substituted by a prokaryotic signal sequence selected, for example, from the group consisting of the alkaline phosphatase, penicillinase, Ipp, or heat-stable enterotoxin II (STII) leaders, LamB, PhoE, PelB, OmpA and MBP. In one embodiment of the invention, the signal sequences used in both cistrons of the expression system are STII signal sequences or variants thereof.

In another aspect, the production of the immunoglobulins according to the invention can occur in the cytoplasm of the host cell, and therefore does not require the presence of secretion signal sequences within each cistron. In that regard, immunoglobulin light and heavy chains are expressed, folded and assembled to form functional immunoglobulins within the cytoplasm. Certain host strains (e.g., the *E. coli trxB*$^-$ strains) provide cytoplasm conditions that are favorable for disulfide bond formation, thereby permitting proper folding and assembly of expressed protein subunits. Proba and Pluckthun Gene, 159:203 (1995).

The present invention provides an expression system in which the quantitative ratio of expressed polypeptide components can be modulated in order to maximize the yield of secreted and properly assembled antibodies of the invention. Such modulation is accomplished at least in part by simultaneously modulating translational strengths for the polypeptide components.

One technique for modulating translational strength is disclosed in Simmons et al., U.S. Pat. No. 5,840,523. It

utilizes variants of the translational initiation region (TIR) within a cistron. For a given TIR, a series of amino acid or nucleic acid sequence variants can be created with a range of translational strengths, thereby providing a convenient means by which to adjust this factor for the desired expression level of the specific chain. TIR variants can be generated by conventional mutagenesis techniques that result in codon changes which can alter the amino acid sequence, although silent changes in the nucleotide sequence are preferred. Alterations in the TIR can include, for example, alterations in the number or spacing of Shine-Dalgarno sequences, along with alterations in the signal sequence. One method for generating mutant signal sequences is the generation of a "codon bank" at the beginning of a coding sequence that does not change the amino acid sequence of the signal sequence (i.e., the changes are silent). This can be accomplished by changing the third nucleotide position of each codon; additionally, some amino acids, such as leucine, serine, and arginine, have multiple first and second positions that can add complexity in making the bank. This method of mutagenesis is described in detail in Yansura et al. (1992) METHODS: A Companion to Methods in Enzymol. 4:151-158.

Preferably, a set of vectors is generated with a range of TIR strengths for each cistron therein. This limited set provides a comparison of expression levels of each chain as well as the yield of the desired antibody products under various TIR strength combinations. TIR strengths can be determined by quantifying the expression level of a reporter gene as described in detail in Simmons et al. U.S. Pat. No. 5, 840,523. Based on the translational strength comparison, the desired individual TIRs are selected to be combined in the expression vector constructs of the invention.

Prokaryotic host cells suitable for expressing antibodies of the invention include Archaebacteria and Eubacteria, such as Gram-negative or Gram-positive organisms. Examples of useful bacteria include Escherichia (e.g., *E. coli*), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. In one embodiment, gram-negative cells are used. In one embodiment, *E. coli* cells are used as hosts for the invention. Examples *of E. coli* strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 *ΔfhuA (ΔtonA) ptr3 lac Iq lacL8 ΔompTΔ(nmpc-fepE) degP41 kan*$^R$ (U.S. Pat. No. 5,639,635). Other strains and derivatives thereof, such as *E. coli* 294 (ATCC 31,446), *E. coli B, E. coli*λ 1776 (ATCC 31,537) and *E. coli* RV308(ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990). It is generally necessary to select the appropriate bacteria taking into consideration replicability of the replicon in the cells of a bacterium. For example, *E. coli,* Serratia, or Salmonella species can be suitably used as the host when well known plasmids such as pBR322, pBR325, pACYC 177, or pKN410 are used to supply the replicon. Typically the host cell should secrete minimal amounts of proteolytic enzymes, and additional protease inhibitors may desirably be incorporated in the cell culture.

*Antibody Production*

Host cells are transformed with the above-described expression vectors and cultured in conventional nutrient media modified as appropriate for inducing promoters, selecting transformants, or amplifying the genes encoding the desired sequences.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/ DMSO. Yet another technique used is electroporation.

Prokaryotic cells used to produce the polypeptides of the invention are grown in media known in the art and suitable for culture of the selected host cells. Examples of suitable media include luria broth (LB) plus necessary nutrient supplements. In some embodiments, the media also contains a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. For *E. coli* growth, for example, the preferred temperature ranges from about 20°C to about 39°C, more preferably from about 25°C to about 37°C, even more preferably at about 30°C. The pH of the medium may be any pH ranging from about 5 to about 9, depending mainly on the host organism. For *E. coli,* the pH is preferably from about 6.8 to about 7.4, and more preferably about 7.0.

If an inducible promoter is used in the expression vector of the invention, protein expression is induced under conditions suitable for the activation of the promoter. In one aspect of the invention, PhoA promoters are used for

controlling transcription of the polypeptides. Accordingly, the transformed host cells are cultured in a phosphate-limiting medium for induction. Preferably, the phosphate-limiting medium is the C.R.A.P medium (see, e.g., Simmons et al., J. Immunol. Methods (2002), 263:133-147). A variety of other inducers may be used, according to the vector construct employed, as is known in the art.

In one embodiment, the expressed polypeptides of the present invention are secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therein. Cells may be removed from the culture and the culture supernatant being filtered and concentrated for further purification of the proteins produced. The expressed polypeptides can be further isolated and identified using commonly known methods such as polyacrylamide gel electrophoresis (PAGE) and Western blot assay.

In one aspect of the invention, antibody production is conducted in large quantity by a fermentation process. Various large-scale fed-batch fermentation procedures are available for production of recombinant proteins. Large-scale fermentations have at least 1000 liters of capacity, preferably about 1,000 to 100,000 liters of capacity. These fermentors use agitator impellers to distribute oxygen and nutrients, especially glucose (the preferred carbon/energy source). Small scale fermentation refers generally to fermentation in a fermentor that is no more than approximately 100 liters in volumetric capacity, and can range from about 1 liter to about 100 liters.

In a fermentation process, induction of protein expression is typically initiated after the cells have been grown under suitable conditions to a desired density, e.g., an $OD_{550}$ of about 180-220, at which stage the cells are in the early stationary phase. A variety of inducers may be used, according to the vector construct employed, as is known in the art and described above. Cells may be grown for shorter periods prior to induction. Cells are usually induced for about 12-50 hours, although longer or shorter induction time may be used.

To improve the production yield and quality of the polypeptides of the invention, various fermentation conditions can be modified. For example, to improve the proper assembly and folding of the secreted antibody polypeptides, additional vectors overexpressing chaperone proteins, such as Dsb proteins (DsbA, DsbB, DsbC, DsbD and or DsbG) or FkpA (a peptidylprolyl cis,trans-isomerase with chaperone activity) can be used to co-transform the host prokaryotic cells. The chaperone proteins have been demonstrated to facilitate the proper folding and solubility of heterologous proteins produced in bacterial host cells. Chen et al. (1999) JBio Chem 274:19601-19605; Georgiou et al., U.S. Patent No. 6,083,715; Georgiou et al., U.S. Patent No. 6,027,888; Bothmann and Pluckthun (2000) J. Biol. Chem. 275: 17100-17105; Ramm and Pluckthun (2000) J. Biol. Chem. 275:17106-17113; Arie et al. (2001) Mol. Microbial. 39:199-210.

To minimize proteolysis of expressed heterologous proteins (especially those that are proteolytically sensitive), certain host strains deficient for proteolytic enzymes can be used for the present invention. For example, host cell strains may be modified to effect genetic mutation(s) in the genes encoding known bacterial proteases such as Protease III, OmpT, DegP, Tsp, Protease I, Protease Mi, Protease V, Protease VI and combinations thereof. Some *E. coli* protease-deficient strains are available and described in, for example, Joly et al. (1998), *supra;* Georgiou et al., U.S. Patent No. 5,264,365; Georgiou et al., U.S. Patent No. 5,508,192; Hara et al., Microbial Drug Resistance, 2:63-72 (1996).

In one embodiment, *E. coli* strains deficient for proteolytic enzymes and transformed with plasmids overexpressing one or more chaperone proteins are used as host cells in the expression system of the invention.

*Antibody Purification*

In one embodiment, the antibody protein produced herein is further purified to obtain preparations that are substantially homogeneous for further assays and uses. Standard protein purification methods known in the art can be employed. The following procedures are exemplary of suitable purification procedures: fractionation on immunoaffinity or ion-exchange columns, ethanol precipitation, reverse phase HPLC, chromatography on silica or on a cation-exchange resin such as DEAE, chromatofocusing, SDS-PAGE, ammonium sulfate precipitation, and gel filtration using, for example, Sephadex G-75.

In one aspect, Protein A immobilized on a solid phase is used for immunoaffinity purification of the full length antibody products of the invention. Protein A is a 41kD cell wall protein from *Staphylococcus aureas* which binds with a high affinity to the Fc region of antibodies. Lindmark et al (1983) J. Immunol. Meth. 62:1-13. The solid phase to which Protein A is immobilized is preferably a column comprising a glass or silica surface, more preferably a controlled pore glass column or a silicic acid column. In some applications, the column has been coated with a reagent, such as glycerol, in an attempt to prevent nonspecific adherence of contaminants.

As the first step of purification, the preparation derived from the cell culture as described above is applied onto the Protein A immobilized solid phase to allow specific binding of the antibody of interest to Protein A. The solid phase is then washed to remove contaminants non-specifically bound to the solid phase. Finally the antibody of interest is recovered from the solid phase by elution.

*Generating antibodies using eukaryotic host cells:*

The vector components generally include, but are not limited to, one or more of the following: a signal sequence, an origin of replication, one or more marker genes, an enhancer element, a promoter, and a transcription termination sequence.

*(i) Signal sequence component*

A vector for use in a eukaryotic host cell may also contain a signal sequence or other polypeptide having a specific cleavage site at the N-terminus of the mature protein or polypeptide of interest. The heterologous signal sequence selected preferably is one that is recognized and processed (*i.e.*, cleaved by a signal peptidase) by the host cell. In mammalian cell expression, mammalian signal sequences as well as viral secretory leaders, for example, the herpes simplex gD signal, are available.

The DNA for such precursor region is ligated in reading frame to DNA encoding the antibody.

*(ii) Origin of replication*

Generally, an origin of replication component is not needed for mammalian expression vectors. For example, the SV40 origin may typically be used only because it contains the early promoter.

*(iii) Selection gene component*

Expression and cloning vectors may contain a selection gene, also termed a selectable marker. Typical selection genes encode proteins that (a) confer resistance to antibiotics or other toxins, *e.g.*, ampicillin, neomycin, methotrexate, or tetracycline, (b) complement auxotrophic deficiencies, where relevant, or (c) supply critical nutrients not available from complex media.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II, preferably primate metallothionein genes, adenosine deaminase, ornithine decarboxylase, *etc.*

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity (e.g., ATCC CRL-9096).

Alternatively, host cells (particularly wild-type hosts that contain endogenous DHFR) transformed or co-transformed with DNA sequences encoding an antibody, wild-type DHFR protein, and another selectable marker such as aminoglycoside 3'-phosphotransferase (APH) can be selected by cell growth in medium containing a selection agent for the selectable marker such as an aminoglycosidic antibiotic, *e.g.*, kanamycin, neomycin, or G418. See U.S. Patent No. 4,965,199.

*(iv) Promoter component*

Expression and cloning vectors usually contain a promoter that is recognized by the host organism and is operably linked to the antibody polypeptide nucleic acid. Promoter sequences are known for eukaryotes. Virtually alleukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Antibody polypeptide transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, *e.g.*, the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in U.S. Patent No. 4,601,978. See also Reyes et al., Nature 297:598-601 (1982) on expression of human β-interferon cDNA in mouse cells under the control of a thymidine kinase promoter from herpes simplex virus. Alternatively, the Rous Sarcoma Virus long terminal repeat can be used as the promoter.

*(v) Enhancer element component*

Transcription of DNA encoding the antibody polypeptide of this invention by higher eukaryotes is often increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the antibody polypeptide-encoding sequence, but is preferably located at a site 5' from the promoter.

*(vi) Transcription termination component*

Expression vectors used in eukaryotic host cells will typically also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding an antibody. One useful transcription termination component is the bovine growth hormone polyadenylation region. See W094/11026 and the expression vector disclosed therein.

*(vii) Selection and transformation of host cells*

Suitable host cells for cloning or expressing the DNA in the vectors herein include higher eukaryote cells described herein, including vertebrate host cells. Propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)) ; baby hamster kidney cells (BHK, ATCC CCL 10); Chinese hamster ovary cells/-DHFR (CHO, Urlaub et al., Proc. Natl. Acad. Sci. USA 77:4216 (1980)) ; mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383: 44-68 (1982)); MRC 5 cells; FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

*(viii) Culturing the host cells*

The host cells used to produce an antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem.102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

*(ix) Purification of antibody*

When using recombinant techniques, the antibody can be produced intracellularly, or directly secreted into the medium. If the antibody is produced intracellularly, as a first step, the particulate debris, either host cells or lysed fragments, are removed, for example, by centrifugation or ultrafiltration. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human γ1, γ2, or γ4 heavy chains (Lindmark et al., J. Immunol. Meth. 62:1-13 (1983)). Protein G is recommended for all mouse isotypes and for human γ3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a $C_H3$ domain, the Bakerbond ABX™resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (*e.g.*, from about 0-0.25M salt).

<u>Activity Assays</u>

The antibodies of the present invention can be characterized for their physical/chemical properties and biological functions by various assays known in the art.

The purified immunoglobulins can be further characterized by a series of assays including, but not limited to, N-terminal sequencing, amino acid analysis, non-denaturing size exclusion high pressure liquid chromatography (HPLC), mass spectrometry, ion exchange chromatography and papain digestion.

In certain embodiments of the invention, the immunoglobulins produced herein are analyzed for their biological activity. In some embodiments, the immunoglobulins of the present invention are tested for their antigen binding activity. The antigen binding assays that are known in the art and can be used herein include without limitation any direct or competitive binding assays using techniques such as western blots, radioimmunoassays, ELISA (enzyme linked immnosorbent assay), "sandwich" immunoassays, immunoprecipitation assays, fluorescent immunoassays, and protein A immunoassays. An illustrative antigen binding assay is provided below in the Examples section.

In one embodiment, the present invention contemplates an altered antibody that possesses some but not all effector functions, which make it a desired candidate for many applications in which the half life of the antibody *in vivo* is important yet certain effector functions (such as complement and ADCC) are unnecessary or deleterious. In certain embodiments, the Fc activities of the produced immunoglobulin are measured to ensure that only the desired properties are maintained. In vitro and/or in vivo cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol 9:457-92 (1991). An example of an in vitro assay to assess ADCC activity of a molecule of interest is described in US Patent No. 5,500,362 or 5,821,337. Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. PNAS (USA) 95:652-656 (1998). C1q binding assays may also be carried out to confirm that the antibody is unable to bind C1q and hence lacks CDC activity. To assess complement activation, a CDC assay, e.g. as described in Gazzano-Santoro et al., J. Immunol. Methods 202:163 (1996), may be performed. FcRn binding and in vivo clearance/half life determinations can also be performed using methods known in the art, e.g. those desribed in the Examples section.

Humanized Antibodies

The present invention encompasses humanized antibodies. Various methods for humanizing non-human antibodies are known in the art. For example, a humanized antibody can have one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al. (1986) Nature 321:522-525; Riechmann et al. (1988) Nature 332: 323-327; Verhoeyen et al. (1988) Science 239:1534-1536), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework for the humanized antibody (Sims et al. (1993) J. Immunol. 151:2296; Chothia et al. (1987) J. Mol. Biol. 196:901. Another method uses a particular framework derived from the consensus sequence of all human antibodies of a particular subgroup of light or heavy chains. The same framework may be used for several different humanized antibodies (Carter et al. (1992) Proc. Natl. Acad. Sci. USA, 89:4285; Presta et al. (1993) J. Immunol., 151:2623.

It is further important that antibodies be humanized with retention of high affinity for the antigen and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.*, the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Antibody Variants

In one aspect, the invention provides antibody fragment comprising modifications in the interface of Fc polypeptides comprising the Fc region, wherein the modifications facilitate and/or promote heterodimerization. These modifications comprise introduction of a protuberance into a first Fc polypeptide and a cavity into a second Fc polypeptide, wherein the protuberance is positionable in the cavity so as to promote complexing of the first and second Fc polypeptides. Methods of generating antibodies with these modifications are known in the art, e.g., as described in U.S. Pat. No. 5,731,168.

In some embodiments, amino acid sequence modification(s) of the antibodies described herein are contemplated. For example, it may be desirable to improve the binding affinity and/or other biological properties of the antibody. Amino acid sequence variants of the antibody are prepared by introducing appropriate nucleotide changes into the antibody nucleic acid, or by peptide synthesis. Such modifications include, for example, deletions from, and/or insertions into and/or substitutions of, residues within the amino acid sequences of the antibody. Any combination of deletion, insertion, and substitution is made to arrive at the final construct, provided that the final construct possesses the desired characteristics. The amino acid alterations may be introduced in the subject antibody amino acid sequence at the time that sequence is made.

A useful method for identification of certain residues or regions of the antibody that are preferred locations for mutagenesis is called "alanine scanning mutagenesis" as described by Cunningham and Wells (1989) Science, 244: 1081-1085. Here, a residue or group of target residues are identified (*e.g.*, charged residues such as arg, asp, his, lys, and glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the performance of a mutation at a given site, ala scanning or random mutagenesis is conducted at the target codon or region and the expressed immunoglobulins are screened for the desired

activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody to an enzyme (*e.g*. for ADEPT) or a polypeptide which increases the serum half-life of the antibody.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. Conservative substitutions are shown in Table 2 under the heading of "preferred substitutions". If such substitutions result in a change in biological activity, then more substantial changes, denominated "exemplary substitutions" in Table 2, or as further described below in reference to amino acid classes, may be introduced and the products screened.

**TABLE 2**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; asp, lys; arg | gln |
| Asp (D) | glu; asn | glu |
| Cys (C) | ser; ala | ser |
| Gln (Q) | asn; glu | asn |
| Glu (E) | asp; gln | asp |
| Gly (G) | ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | tyr |
| Pro (P) | ala | ala |
| Ser (S) | thr; cys | cys |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;

(2) neutral hydrophilic: cys, ser, thr;

(3) acidic: asp, glu;

(4) basic: asn, gln, his, lys, arg;

(5) residues that influence chain orientation: gly, pro; and

(6) aromatic: trp, tyr, phe.

Non-conservative substitutions will entail exchanging a member of one of these classes for another class.

One type of substitutional variant involves substituting one or more hypervariable region residues of a parent antibody (*e.g.* a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (*e.g.* 6-7 sites) are mutated to generate all possible amino acid substitutions at each site. The antibodies thus generated are displayed from filamentous phage particles as fusions to the gene III product of M13 packaged within each particle. The phage-displayed variants are then screened for their biological activity (*e.g.* binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

It may be desirable to introduce one or more amino acid modifications in an Fc region of the immunoglobulin polypeptides of the invention, thereby generating a Fc region variant. The Fc region variant may comprise a human Fc region sequence (*e.g.*, a human IgG1, IgG2, IgG3 or IgG4 Fc region) comprising an amino acid modification (*e.g.* a substitution) at one or more amino acid positions including that of a hinge cysteine.

In accordance with this description and the teachings of the art, it is contemplated that in some embodiments, an antibody used in methods of the invention may comprise one or more alterations as compared to the wild type counterpart antibody, e.g. in the Fc region. These antibodies would nonetheless retain substantially the same characteristics required for therapeutic utility as compared to their wild type counterpart. For example, it is thought that certain alterations can be made in the Fc region that would result in altered (*i.e.,* either improved or diminished) C1q binding and/or Complement Dependent Cytotoxicity (CDC), e.g., as described in WO99/51642. See also Duncan & Winter Nature 322:738-40 (1988); US Patent No. 5,648,260; US Patent No. 5,624,821; and WO94/29351 concerning other examples of Fc region variants.

## Immunoconjugates

The invention also pertains to immunoconjugates, or antibody-drug conjugates (ADC), comprising an antibody conjugated to a cytotoxic agent such as a chemotherapeutic agent, a drug, a growth inhibitory agent, a toxin (e.g., an enzymatically active toxin of bacterial, fungal, plant, or animal origin, or fragments thereof), or a radioactive isotope (i.e., a radioconjugate).

The use of antibody-drug conjugates for the local delivery of cytotoxic or cytostatic agents, i.e. drugs to kill or inhibit tumor cells in the treatment of cancer (Syrigos and Epenetos (1999) Anticancer Research 19:605-614; Niculescu-Duvaz and Springer (1997) Adv. Drg Del. Rev. 26:151-172; U.S. patent 4,975,278) theoretically allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al., (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al. (ed.s), pp. 475-506). Maximal efficacy with minimal toxicity is sought thereby. Both polyclonal antibodies and monoclonal antibodies have been reported as useful in these strategies (Rowland et al., (1986) Cancer Immunol. Immunother., 21: 183-87). Drugs used in these methods include daunomycin, doxorubicin, methotrexate, and vindesine (Rowland et al., (1986) supra). Toxins used in antibody-toxin conjugates include bacterial toxins such as diphtheria toxin, plant toxins such as ricin, small molecule toxins such as geldanamycin (Mandler et al (2000) Jour. of the Nat. Cancer Inst. 92(19): 1573-1581; Mandler et al (2000) Bioorganic & Med. Chem. Letters 10:1025-1028; Mandler et al (2002) Bioconjugate

Chem. 13:786-791), maytansinoids (EP 1391213; Liu et al., (1996) Proc. Natl. Acad. Sci. USA 93:8618-8623), and calicheamicin (Lode et al (1998) Cancer Res. 58:2928; Hinman et al (1993) Cancer Res. 53:3336-3342). The toxins may effect their cytotoxic and cytostatic effects by mechanisms including tubulin binding, DNA binding, or topoisomerase inhibition. Some cytotoxic drugs tend to be inactive or less active when conjugated to large antibodies or protein receptor ligands.

ZEVALIN® (ibritumomab tiuxetan, Biogen/Idec) is an antibody-radioisotope conjugate composed of a murine IgG1 kappa monoclonal antibody directed against the CD20 antigen found on the surface of normal and malignant B lymphocytes and $^{111}$In or $^{90}$Y radioisotope bound by a thiourea linker-chelator (Wiseman et al (2000) Eur. Jour. Nucl. Med. 27(7):766-77; Wiseman et al (2002) Blood 99(12):4336-42; Witzig et al (2002) J. Clin. Oncol. 20(10):2453-63; Witzig et al (2002) J. Clin. Oncol. 20(15):3262-69). Although ZEVALIN has activity against B-cell non-Hodgkin's Lymphoma (NHL), administration results in severe and prolonged cytopenias in most patients. MYLOTARG™ (gemtuzumab ozogamicin, Wyeth Pharmaceuticals), an antibody drug conjugate composed of a hu CD33 antibody linked to calicheamicin, was approved in 2000 for the treatment of acute myeloid leukemia by injection (Drugs of the Future (2000) 25(7): 686; US Patent Nos. 4970198; 5079233; 5585089; 5606040; 5693762; 5739116; 5767285; 5773001). Cantuzumab mertansine (Immunogen, Inc.), an antibody drug conjugate composed of the huC242 antibody linked via the disulfide linker SPP to the maytansinoid drug moiety, DM1, is advancing into Phase II trials for the treatment of cancers that express CanAg, such as colon, pancreatic, gastric, and others. MLN-2704 (Millennium Pharm., BZL Biologics, Immunogen Inc.), an antibody drug conjugate composed of the anti-prostate specific membrane antigen (PSMA) monoclonal antibody linked to the maytansinoid drug moiety, DM1, is under development for the potential treatment of prostate tumors. The auristatin peptides, auristatin E (AE) and monomethylauristatin (MMAE), synthetic analogs of dolastatin, were conjugated to chimeric monoclonal antibodies cBR96 (specific to Lewis Y on carcinomas) and cAC10 (specific to CD30 on hematological malignancies) (Doronina et al (2003) Nature Biotechnology 21(7):778-784) and are under therapeutic development.

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. See, e.g., WO 93/21232 published October 28, 1993. A variety of radionuclides are available for the production of radioconjugated antibodies. Examples include $^{212}$Bi, $^{131}$I, $^{131}$In, $^{90}$Y, and $^{186}$Re. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HC1), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

Conjugates of an antibody and one or more small molecule toxins, such as a calicheamicin, maytansinoids, a trichothecene, and CC1065, and the derivatives of these toxins that have toxin activity, are also contemplated herein.

Maytansine and maytansinoids

In one embodiment, an antibody (full length or fragments) of the invention is conjugated to one or more maytansinoid molecules.

Maytansinoids are mitototic inhibitors which act by inhibiting tubulin polymerization. Maytansine was first isolated from the east African shrub Maytenus serrata (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533, the disclosures of which are hereby expressly incorporated by reference.

Maytansinoid-antibody conjugates

In an attempt to improve their therapeutic index, maytansine and maytansinoids have been conjugated to antibodies specifically binding to tumor cell antigens. Immunoconjugates containing maytansinoids and their therapeutic use are disclosed, for example, in U.S. Patent Nos. 5,208,020, 5,416,064 and European Patent EP 0 425 235 B1, the

disclosures of which are hereby expressly incorporated by reference. Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an in vivo tumor growth assay. Chari et al., Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA.1 that binds the HER-2/neu oncogene. The cytotoxicity of the TA.1-maytansonoid conjugate was tested in vitro on the human breast cancer cell line SK-BR-3, which expresses 3 x $10^5$ HER-2 surface antigens per cell. The drug conjugate achieved a degree of cytotoxicity similar to the free maytansinoid drug, which could be increased by increasing the number of maytansinoid molecules per antibody molecule. The A7-maytansinoid conjugate showed low systemic cytotoxicity in mice.

Antibody-maytansinoid conjugates (immunoconjugates)

Antibody-maytansinoid conjugates are prepared by chemically linking an antibody to a maytansinoid molecule without significantly diminishing the biological activity of either the antibody or the maytansinoid molecule. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent No. 5,208,020 and in the other patents and nonpatent publications referred to hereinabove. Preferred maytansinoids are maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Patent No. 5,208,020 or EP Patent 0 425 235 B1, and Chari et al., Cancer Research 52:127-131 (1992). The linking groups include disulfide groups, thioether groups, acid labile groups, photolabile groups, peptidase labile groups, or esterase labile groups, as disclosed in the above-identified patents, disulfide and thioether groups being preferred.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particularly preferred coupling agents include N-succinimidyl-3-(2-pyridyldithio) propionate (SPDP) (Carlsson et al., Biochem. J. 173:723-737 [1978]) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hydroxymethyl, the C-15 position modified with a hydroxyl group, and the C-20 position having a hydroxyl group. In a preferred embodiment, the linkage is formed at the C-3 position of maytansinol or a maytansinol analogue.

Calicheamicin

Another immunoconjugate of interest comprises an antibody conjugated to one or more calicheamicin molecules. The calicheamicin family of antibiotics are capable of producing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, $\gamma_1^I$,$\alpha_2^I$, $\alpha_3^I$, N-acetyl-$\gamma_1^I$, PSAG and $\theta^I_1$ (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

Other cytotoxic agents

Other antitumor agents that can be conjugated to the antibodies of the invention include BCNU, streptozoicin,

vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an immunoconjugate formed between an antibody and a compound with nucleolytic activity (e.g., a ribonuclease or a DNA endonuclease such as a deoxyribonuclease; DNase).

For selective destruction of the tumor, the antibody may comprise a highly radioactive atom. A variety of radioactive isotopes are available for the production of radioconjugated antibodies. Examples include $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu. When the conjugate is used for detection, it may comprise a radioactive atom for scintigraphic studies, for example $tc^{99m}$ or $I^{123}$ or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as $tc^{99m}$ or $I^{123}$, .$Re^{186}$, $Re^{188}$ and $In^{111}$ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal,CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See W094/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

The compounds of the invention expressly contemplate, but are not limited to, ADC prepared with cross-linker reagents: BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A). See pages 467-498, 2003-2004 Applications Handbook and Catalog.

Preparation of antibody drug conjugates

In the antibody drug conjugates (ADC) of the invention, an antibody (Ab) is conjugated to one or more drug moieties (D), e.g. about 1 to about 20 drug moieties per antibody, through a linker (L). The ADC of Formula I may be prepared by several routes, employing organic chemistry reactions, conditions, and reagents known to those skilled in the art, including: (1) reaction of a nucleophilic group of an antibody with a bivalent linker reagent, to form Ab-L, via a covalent bond, followed by reaction with a drug moiety D; and (2) reaction of a nucleophilic group of a drug moiety with a bivalent linker reagent, to form D-L, via a covalent bond, followed by reaction with the nucleophilic group of an antibody.

$$Ab-(L-D)_p \qquad I$$

Nucleophilic groups on antibodies include, but are not limited to: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes,

ketones, carboxyl, and maleimide groups. Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol.

Antibody drug conjugates of the invention may also be produced by modification of the antibody to introduce electrophilic moieties, which can react with nucleophilic subsituents on the linker reagent or drug. The sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g. by borohydride reagents to form stable amine linkages. In one embodiment, reaction of the carbohydrate portion of a glycosylated antibody with either glactose oxidase or sodium meta-periodate may yield carbonyl (aldehyde and ketone) groups in the protein that can react with appropriate groups on the drug (Hermanson, Bioconjugate Techniques). In another embodiment, proteins containing N-terminal serine or threonine residues can react with sodium meta-periodate, resulting in production of an aldehyde in place of the first amino acid (Geoghegan & Stroh, (1992) Bioconjugate Chem. 3:138-146; US 5362852). Such aldehyde can be reacted with a drug moiety or linker nucleophile.

Likewise, nucleophilic groups on a drug moiety include, but are not limited to: amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups.

Alternatively, a fusion protein comprising the antibody and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

In yet another embodiment, the antibody may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

Antibody Derivatives

The antibodies of the present invention can be further modified to contain additional nonproteinaceous moieties that are known in the art and readily available. Preferably, the moieties suitable for derivatization of the antibody are water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymers are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

Antigen Specificity

The present invention is applicable to antibodies of any appropriate antigen binding specificity. Preferably, the antibodies used in methods of the invention are specific to antigens that are biologically important polypeptides. More preferably, the antibodies of the invention are useful for therapy or diagnosis of diseases or disorders in a mammal. Antibodies of the invention include, but are not limited to blocking antibodies, agonist antibodies, neutralizing antibodies or antibody conjugates. Non-limiting examples of therapeutic antibodies include anti-c-met, anti-VEGF, anti-IgE, anti-CD11, anti-CD18, anti-CD40, anti-tissue factor (TF), anti-HER2, and anti-TrkC antibodies. Antibodies directed against non-polypeptide antigens (such as tumor-associated glycolipid antigens) are also contemplated.

Where the antigen is a polypeptide, it may be a transmembrane molecule (*e.g.* receptor) or a ligand such as a growth factor. Exemplary antigens include molecules such as renin; a growth hormone, including human growth hormone and bovine growth hormone; growth hormone releasing factor; parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; clotting factors such as factor VIIIC, factor IX, tissue factor (TF), and von Willebrands factor; anti-clotting factors such as Protein C; atrial natriuretic factor; lung surfactant; a plasminogen activator, such as urokinase or human urine or tissue-type plasminogen activator (t-PA); bombesin; thrombin; hemopoietic growth factor; tumor necrosis factor-alpha and -beta; enkephalinase; RANTES (regulated on activation normally T-cell expressed and secreted); human macrophage inflammatory protein (MIP-1-alpha); a serum albumin such as human serum albumin; Muellerian-inhibiting substance; relaxin A-chain; relaxin B-chain; prorelaxin; mouse gonadotropin-associated peptide; a microbial protein, such as beta-lactamase; DNase; IgE; a cytotoxic T-lymphocyte associated antigen (CTLA), such as CTLA-4; inhibin; activin; vascular endothelial growth factor (VEGF); receptors for hormones or growth factors; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-β; platelet-derived growth factor (PDGF); fibroblast growth factor such as aFGF and bFGF; epidermal growth factor (EGF); transforming growth factor (TGF) such as TGF-alpha and TGF-beta, including TGF-β1, TGF-2, TGF-β3, TGF-β4, or TGF-β5; insulin-like growth factor-I and -II (IGF-I and IGF-II); des(1-3)-IGF-I (brain IGF-I), insulin-like growth factor binding proteins; CD proteins such as CD3, CD4, CD8, CD19, CD20 and CD40; erythropoietin; osteoinductive factors; immunotoxins; a bone morphogenetic protein (BMP); an interferon such as interferon-alpha, - beta, and -gamma; colony stimulating factors (CSFs), *e.g.*, M-CSF, GM-CSF, and G-CSF; interleukins (ILs), *e.g.*, IL-1 to IL-10; superoxide dismutase; T-cell receptors; surface membrane proteins; decay accelerating factor; viral antigen such as, for example, a portion of the HIV envelope; transport proteins; homing receptors; addressins; regulatory proteins; integrins such as CD11a, CD11b, CD11c, CD 18, an ICAM, VLA-4 and VCAM; a tumor associated antigen such as HER2, HER3 or HER4 receptor; and fragments of any of the above-listed polypeptides.

Antigens for antibodies encompassed by one embodiment of the present invention include CD proteins such as CD3, CD4, CD8, CD19, CD20, CD34, and CD46; members of the ErbB receptor family such as the EGF receptor, HER2, HER3 or HER4 receptor; cell adhesion molecules such as LFA-1, Mac1, p150.95, VLA-4, ICAM-1, VCAM, α4/β7 integrin, and αv/β3 integrin including either α or β subunits thereof (*e.g.* anti-CD11a, anti-CD 18 or anti-CD11b antibodies); growth factors such as VEGF; tissue factor (TF); TGF-∃; alpha interferon (∀-IFN); an interleukin, such as IL-8; IgE; blood group antigens Apo2, death receptor; flk2/flt3 receptor; obesity (OB) receptor; *mpl* receptor; CTLA-4; protein C etc. In some embodiments, targets herein are VEGF, TF, CD 19, CD20, CD40, TGF-∃, CD11a, CD18, Apo2 and C24.

In some embodiments, an antibody of the invention is capable of binding specifically to a tumor antigen. In some embodiments, an antibody of the invention is capable of binding specifically to a tumor antigen wherein the tumor antigen is not a cluster differentiation factor (i.e., a CD protein). In some embodiments, an antibody of the invention is capable of binding specifically to a CD protein. In some embodiments, an antibody of the invention is capable of binding specifically to a CD protein other than CD3 or CD4. In some embodiments, an antibody of the invention is capable of binding specifically to a CD protein other than CD19 or CD20. In some embodiments, an antibody of the invention is capable of binding specifically to a CD protein other than CD40. In some embodiments, an antibody of the invention is capable of binding specifically to CD 19 or CD20. In some embodiments, an antibody of the invention is capable of binding specifically to CD40. In some embodiments, an antibody of the invention is capable of binding specifically to CD 11. In one embodiment, an antibody of the invention binds an antigen that is not expressed in an immune cell. In one embodiment, an antibody of the invention binds an antigen that is not expressed in T cells. In one embodiment, an antibody of the invention binds an antigen that is not expressed in B cells.

In one embodiment, an antibody of the invention is capable of binding specifically to a cell survival regulatory factor. In some embodiments, an antibody of the invention is capable of binding specifically to a cell proliferation regulatory factor. In some embodiments, an antibody of the invention is capable of binding specifically to a molecule involved in cell cycle regulation. In other embodiments, an antibody of the invention is capable of binding specifically to a molecule involved in tissue development or cell differentiation. In some embodiments, an antibody of the invention is capable of binding specifically to a cell surface molecule. In some embodiments, an antibody of the invention is capable of binding to a tumor antigen that is not a cell surface receptor polypeptide.

In one embodiment, an antibody of the invention is capable of binding specifically to a lymphokine. In another embodiment, an antibody of the invention is capable of binding specifically to a cytokine.

In one embodiment, antibodies of the invention are capable of binding specifically to a molecule involved in vasculogenesis. In another embodiment, antibodies of the invention are capable of binding specifically to a molecule involved in angiogenesis.

Soluble antigens or fragments thereof, optionally conjugated to other molecules, can be used as immunogens for generating antibodies. For transmembrane molecules, such as receptors, fragments of these molecules (*e.g.* the extracellular domain of a receptor) can be used as the immunogen. Alternatively, cells expressing the transmembrane molecule can be used as the immunogen. Such cells can be derived from a natural source (*e.g.* cancer cell lines) or may be cells which have been transformed by recombinant techniques to express the transmembrane molecule. Other antigens and forms thereof useful for preparing antibodies will be apparent to those in the art.

Pharmaceutical Formulations

Therapeutic formulations comprising an antibody of the invention are prepared for storage by mixing the antibody having the desired degree of purity with optional physiologically acceptable carriers, excipients or stabilizers (Remington's Pharmaceutical Sciences 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.*, Zn-protein complexes); and/or non-ionic surfactants such as TWEEN™, PLURONICS™ or polyethylene glycol (PEG).
The formulation herein may also contain more than one active compound as necessary for the particular indication being treated, preferably those with complementary activities that do not adversely affect each other. Such molecules are suitably present in combination in amounts that are effective for the purpose intended.
The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).
The formulations to be used for *in vivo* administration must be sterile. This is readily accomplished by filtration through sterile filtration membranes.
Sustained-release preparations may be prepared. Suitable examples of sustained-release preparations include semipermeable matrices of solid hydrophobic polymers containing the immunoglobulin of the invention, which matrices are in the form of shaped articles, *e.g.*, films, or microcapsule. Examples of sustained-release matrices include polyesters, hydrogels (for example, poly(2-hydroxyethyl-methacrylate), or poly(vinylalcohol)), polylactides (U. S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Uses

An immunoglobulin of the present invention may be used in, for example, *in vitro, ex vivo* and *in vivo* therapeutic methods. The invention provides various methods based on using monovalent antibody fragments having superior properties compared to conventional monovalent antibodies. In certain pathological conditions, it is necessary and/or desirable to utilize monovalent antibodies. Also, in some instances, a therapeutic antibody may effect its therapeutic action without involving immune system-mediated acitivities, such as the effector functions ADCC, phagocytosis and CDC. In such situations, it is desirable to generate forms of antibodies in which such activities are substantially reduced or eliminated. It is also advantageous if the antibody is of a form that can be made efficiently and with high yield. The present invention provides these antibodies, which can be used for a variety of purposes, for example as therapeutics, prophylactics and diagnostics. For example, the invention provides methods of treating a disease, said methods comprising administering to a subject in need of treatment a highly stable antibody fragment comprising a single antigen binding arm, whereby the disease is treated. Any of the antibody fragments of the invention described herein can be used in therapeutic (or prophylactic or diagnostic) methods described herein.
Antibodies of the invention can be used as an antagonist to partially or fully block the specific antigen activity *in vitro, ex vivo* and/or *in vivo.* Moreover, at least some of the antibodies of the invention can neutralize antigen activity from other species. Accordingly, the antibodies of the invention can be used to inhibit a specific antigen activity, e.g., in a cell culture containing the antigen, in human subjects or in other mammalian subjects having the antigen with which

an antibody of the invention cross-reacts (e.g. chimpanzee, baboon, marmoset, cynomolgus and rhesus, pig or mouse). In one embodiment, the antibody of the invention can be used for inhibiting antigen activities by contacting the antibody with the antigen such that antigen activity is inhibited. Preferably, the antigen is a human protein molecule.

In one embodiment, an antibody of the invention can be used in a method for inhibiting an antigen in a subject suffering from a disorder in which the antigen activity is detrimental, comprising administering to the subject an antibody of the invention such that the antigen activity in the subject is inhibited. Preferably, the antigen is a human protein molecule and the subject is a human subject. Alternatively, the subject can be a mammal expressing the antigen with which an antibody of the invention binds. Still further the subject can be a mammal into which the antigen has been introduced (e.g., by administration of the antigen or by expression of an antigen transgene). An antibody of the invention can be administered to a human subject for therapeutic purposes. Moreover, an antibody of the invention can be administered to a non-human mammal expressing an antigen with which the immunoglobulin cross-reacts (e.g., a primate, pig or mouse) for veterinary purposes or as an animal model of human disease. Regarding the latter, such animal models may be useful for evaluating the therapeutic efficacy of antibodies of the invention (e.g., testing of dosages and time courses of administration). Blocking antibodies of the invention that are therapeutically useful include, for example but not limited to, anti-c-met, anti-VEGF, anti-IgE, anti-CD 11, anti-interferon and anti-tissue factor antibodies. The antibodies of the invention can be used to treat, inhibit, delay progression of, prevent/delay recurrence of, ameliorate, or prevent diseases, disorders or conditions associated with abnormal expression and/or activity of one or more antigen molecules, including but not limited to malignant and benign tumors; non-leukemias and lymphoid malignancies; neuronal, glial, astrocytal, hypothalamic and other glandular, macrophagal, epithelial, stromal and blastocoelic disorders; and inflammatory, angiogenic and immunologic disorders.

In one aspect, a blocking antibody of the invention is specific to a ligand antigen, and inhibits the antigen activity by blocking or interfering with the ligand-receptor interaction involving the ligand antigen, thereby inhibiting the corresponding signal pathway and other molecular or cellular events. The invention also features receptor-specific antibodies which do not necessarily prevent ligand binding but interfere with receptor activation, thereby inhibiting any responses that would normally be initiated by the ligand binding. The invention also encompasses antibodies that either preferably or exclusively bind to ligand-receptor complexes. An antibody of the invention can also act as an agonist of a particular antigen receptor, thereby potentiating, enhancing or activating either all or partial activities of the ligand-mediated receptor activation.

In certain embodiments, an immunoconjugate comprising an antibody conjugated with a cytotoxic agent is administered to the patient. In some embodiments, the immunoconjugate and/or antigen to which it is bound is/are internalized by the cell, resulting in increased therapeutic efficacy of the immunoconjugate in killing the target cell to which it binds. In one embodiment, the cytotoxic agent targets or interferes with nucleic acid in the target cell. Examples of such cytotoxic agents include any of the chemotherapeutic agents noted herein (such as a maytansinoid or a calicheamicin), a radioactive isotope, or a ribonuclease or a DNA endonuclease.

Antibodies of the invention can be used either alone or in combination with other compositions in a therapy. For instance, an antibody of the invention may be co-administered with another antibody, chemotherapeutic agent(s) (including cocktails of chemotherapeutic agents), other cytotoxic agent(s), anti-angiogenic agent(s), cytokines, and/or growth inhibitory agent(s). Where an antibody of the invention inhibits tumor growth, it may be particularly desirable to combine it with one or more other therapeutic agent(s) which also inhibits tumor growth. For instance, anti-VEGF antibodies blocking VEGF activities may be combined with anti-ErbB antibodies (*e.g.* HERCEPTIN® anti-HER2 antibody) in a treatment of metastatic breast cancer. Alternatively, or additionally, the patient may receive combined radiation therapy (*e.g.* external beam irradiation or therapy with a radioactive labeled agent, such as an antibody). Such combined therapies noted above include combined administration (where the two or more agents are included in the same or separate formulations), and separate administration, in which case, administration of the antibody of the invention can occur prior to, and/or following, administration of the adjunct therapy or therapies.

The antibody of the invention (and adjunct therapeutic agent) is/are administered by any suitable means, including parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous administration. In addition, the antibody is suitably administered by pulse infusion, particularly with declining doses of the antibody. Dosing can be by any suitable route, e.g. by injections, such as intravenous or subcutaneous injections, depending in part on whether the administration is brief or chronic.

The antibody composition of the invention will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibodies of the invention present in the formulation, the type of disorder or treatment, and other factors discussed above. These are

generally used in the same dosages and with administration routes as used hereinbefore or about from 1 to 99% of the heretofore employed dosages.

For the prevention or treatment of disease, the appropriate dosage of an antibody of the invention (when used alone or in combination with other agents such as chemotherapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 μg/kg to 15 mg/kg (*e.g.* 0.1mg/kg-10mg/kg of antibody is an initial candidate dosage for administration to the patient, whether, for example, by one or more separate administrations, or by continuous infusion. One typical daily dosage might range from about 1 μg/kg to 100 mg/kg or more, depending on the factors mentioned above. For repeated administrations over several days or longer, depending on the condition, the treatment is sustained until a desired suppression of disease symptoms occurs. One exemplary dosage of the antibody would be in the range from about 0.05mg/kg to about 10mg/kg. Thus, one or more doses of about 0.5mg/kg, 2.0mg/kg, 4.0mg/kg or 10mg/kg (or any combination thereof) may be administered to the patient. Such doses may be administered intermittently, *e.g.* every week or every three weeks (*e.g.* such that the patient receives from about two to about twenty, *e.g.* about six doses of the antibody). An initial higher loading dose, followed by one or more lower doses may be administered. An exemplary dosing regimen comprises administering an initial loading dose of about 4 mg/kg, followed by a weekly maintenance dose of about 2 mg/kg of the antibody. However, other dosage regimens may be useful. The progress of this therapy is easily monitored by conventional techniques and assays.

## Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, etc. The containers may be formed from a variety of materials such as glass or plastic. The container holds a composition which is by itself or when combined with another compositions effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice, such as cancer. Moreover, the article of manufacture may comprise (a) a first container with a composition contained therein, wherein the composition comprises an antibody of the invention; and (b) a second container with a composition contained therein, wherein the composition comprises a further cytotoxic agent. The article of manufacture in this embodiment of the invention may further comprise a package insert indicating that the first and second antibody compositions can be used to treat a particular condition, e.g. cancer. Alternatively, or additionally, the article of manufacture may further comprise a second (or third) container comprising a pharmaceutically-acceptable buffer such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The following are examples of the methods and compositions of the invention. It is understood that various other embodiments may be practiced, given the general description provided above.

## EXAMPLES

### EXAMPLE 1: Generation and characterization of an antibody fragment of the invention (also referred to below as "one-armed antibody" or "Fab/c antibody")

*Construction of Expression Vector*

All plasmids for the expression of full-length or Fab/c anti-c-met antibodies were based on a separate cistron system (Simmons et al., J. Immunol. Methods, 263: 133-147 (2002)) which relied on separate *phoA* promoters (AP) (Kikuchi et al., Nucleic Acids Res., 9: 5671-5678 (1981)) for the transcription of heavy and light chains and the Fc fragment, followed by the *trp* Shine-Dalgarno sequence for translation initiation (Yanofsky et al., Nucleic Acids Res., 9: 6647-6668 (1981) and Chang et al., Gene, 55: 189-196 (1987)). Additionally, the heat-stable enterotoxin II signal sequence (STII) (Picken et al., Infect. Immun., 42: 269-275 (1983) and Lee et al., Infect. Immun., 42: 264-268 (1983)) was used for periplasmic secretion of heavy and light chains and the Fc fragment. Fine control of translation for both chains and the Fc fragment was achieved with previously described STII signal sequence variants of measured relative translational strengths, which contain silent codon changes in the translation initiation region (TIR) (Simmons and Yansura,

Nature Biotechnol., 14: 629-634 (1996) and Simmons et al., J. Immunol. Methods, 263: 133-147 (2002)). Finally, the $\lambda_{t0}$ transcriptional terminator (Schlosstissek and Grosse, Nucleic Acids Res., 15: 3185 (1987)) was placed downstream of the coding sequences for both chains and the Fc fragment. All plasmids use the framework of a pBR322-based vector system (Sutcliffe, Cold Spring Harbor Symp. Quant. Biol., 43: 77-90 (1978)). The source anti-c-met antibody was the 5D5 antibody described in U.S. Pat Nos. 5,686,292; 5,646,036; 6,207,152; 6,214,344 & 6,468,529. The hybridoma cell line for the 5D5 source antibody was previously deposited with the American Type Culture Collection, 12301 Parklawn Drive, Rockville, Md., USA, as ATCC No. HB-11895 (Hybridoma SD5.11.6) (Deposit Date: May 23, 1995).

*Plasmid pxcM11C*

Two intermediate plasmids were required to generate the desired pxcM11C plasmid that encodes a chimeric 5D5 anti-c-met antibody. The variable domains of the 5D5 heavy and light chains were first transferred separately onto pBR322-based plasmids for the expression of each chain. The following describes the preparation of these intermediate plasmids pxcMLC and pxcMHC followed by the construction of pxcM11C.

*pxcMLC*

This plasmid was constructed in order to transfer the murine light variable domain of the 5D5 antibody to a human light chain framework compatible for generating the full-length antibody. The construction of this plasmid involved the ligation of three DNA fragments. The first was the pPho51 vector (Simmons and Yansura, Nature Biotechnol, 14: 629-634 (1996), variant 1) in which the small MluI-BamHI fragment had been removed. The second part of the ligation was an approximately 516 base pair AlwNI-BamHI fragment from pST7LC encoding the last 15 amino acids of light chain, the $\lambda_{t0}$ terminator, and the beginning of the *tet* gene. The plasmid pST7LC is a derivative of variant 6 (see above reference) with a human kappa light chain downstream of the STII signal sequence. The third part of the ligation was an approximately 623 base pair MluI-AlwNI PCR fragment generated from a plasmid containing 5D5 Fab sequences (described in Example 2 below under "Cloning and Recombinant Expression of 5D5 Fab"), using the following primers:

5' - TAAATTTAACGCGTACGCTGACATTATGATGTCCCAGTCTCCATCC
(SEQ ID NO: 9)

5'-GGGCGAGCTCAGGCCCTGATGGGTGACTTCGCAGGC
(SEQ ID NO: 10)

*pxcMHC*

This plasmid was constructed to introduce the murine heavy variable domain of the 5D5 antibody into a human heavy chain framework compatible for generating the full-length antibody. The construction of pxcMHC involved the ligation of two DNA fragments. The first was the pST2HC vector in which the small MluI-BstEII fragment had been removed. The plasmid pST2HC is a derivative of variant 3 (see above reference) in which a human IgG1 heavy chain was fused downstream of the STII signal sequence. The second part of the ligation was an approximately 346 base pair MluI-BstEII PCR fragment generated from a plasmid containing 5D5 Fab sequences (described in Example 2 below under "Cloning and Recombinant Expression of 5D5 Fab"), using the following primers:

5' - GCTACAAACGCGTACGCTCAGGTTCAGCTGCAGCAGTCTGGG
(SEQ ID NO: 11)

5' - AAGAGACGGTGACCGAGGTTCCTTGACC
(SEQ ID NO: 12)

*pxcM11C*

The pxcM11C plasmid was constructed to express a full-length 5D5 chimeric antibody. The construction of the plasmid involved the ligation of four DNA fragments. The first was the paTF20 vector (Simmons et al., J. Immunol. Methods, 263: 133-147 (2002), paTF20 is the polycistronic vector with TIR's of 1 - light and 1 - heavy) in which the small MluI-BstEII fragment had been removed. The second part of the ligation was an approximately 623 base pair MluI-AlwNI fragment from pxcMLC. The third part was an approximately 547 base pair AlwNI-BsiWI fragment from paTF50 (see above reference; paTF50 is a separate cistronic vector with TIR's of 1 - light and 1 - heavy). The final part of the ligation

was the approximately 349 base pair BsiWI-BstEII fragment from pxcMHC.

*Plasmid pxcM11C-Fc*

For the construction of pxcM11C-Fc, the cassette coding for the expression of the Fc fragment with all the control elements described above including the AP promoter, STII signal sequence and the $\lambda_{t0}$ transcriptional terminator was added to the pxcM11C plasmid. Two plasmids, pBR322.VNERK.HC and pBR322.Fc, leading to the construction of pxcM11c-Fc need to be described first.

*pBR322. VNERK.HC*

The pBR322.VNERK.HC plasmid is a derivative of variant 1 (Simmons and Yansura, Nature Biotechnol, 14: 629-634 (1996)) with a human heavy chain downstream of the STII signal sequence. This plasmid was constructed by ligating together two DNA fragments. The first was the vector pBR322 in which the small EcoRI-ClaI fragment had been removed. The second part in the ligation was an approximately 1885 base pair EcoRI-ClaI PCR fragment generated from pVG11.VNERK encoding the AP promoter, STII signal sequence, heavy chain, the $\lambda_{t0}$ terminator, and the beginning of the *tet* gene using the following primers:

5' - TTTCCCTTTGAATTCTTGGTTGTTAACGTTGCCGACGCGCATC
(SEQ ID NO: 13)

5' -
TTTCCCTTTATCGATGATAAGCTGTCAAACATGAGTAAACAATAAAAAACG
CCC (SEQ ID NO: 14)

The plasmid pVG11.VNERK is a derivative of the separate cistron vector with TIRs of 1 - light and 1 - heavy (Simmons et al., J. Immunol. Methods, 263: 133-147 (2002)) in which the light and heavy variable domains have been changed to an anti-VEGF antibody (VNERK).

*pBR322.Fc*

The pBR322.Fc plasmid is a derivative of the pBR322.VNERK.HC plasmid which encodes for the expression of the Fc fragment with all the control elements described above.

The pBR322.Fc plasmid was constructed by ligating together two DNA fragments. The first was the vector pBR322.VNERK.HC in which the small MluI-NsiI fragment had been removed. The second part in the ligation was an approximately 1319 base pair MauI-NsiI fragment from pJAL226 encoding amino acids SGTT followed by amino acids 221 to 429 of a human IgG1. pJAL226 is a derivative of variant 3 (Simmons and Yansura, Nature Biotechnol, 14: 629-634 (1996)) with a human Fc fragment downstream of the STII signal sequence.

*pxcM11C-Fc*

pxcM11C-Fc was constructed by ligating together two DNA fragments. The first was the vector pxcM11C in which the small HpaI-ClaI fragment had been removed. The second part in the ligation was an approximately 1198 base pair HpaI-ClaI fragment from pBR322.Fc. This ligation resulted in the desired plasmid designated pxcM11C- Fc.

*Plasmid pxcM11C.H-Fc.K*

The plasmid pxcM11C.H-Fc.K is a derivative of pxcM11C-Fc in which the CH3 domain of the pxcM11C heavy chain was replaced with a CH3 domain with the "hole" (also referred to herein as "cavity") mutations (T366S, L368A, Y407V) (Merchant et al., Nature Biotechnology, 16:677-681 (1998)). In addition, the CH3 domain of the Fc fragment was replaced with a CH3 domain with the "knob" (also referred to herein as "protuberance") mutation (T366W) (see above reference).

*pxcM11C.H*

The plasmid was constructed in two steps. In the first step, the "hole" mutations were introduced by ligating together two DNA fragments. The first was the vector pxcM11C in which the small SacII-NsiI fragment had been removed. The second part in the ligation was an approximately 411 base pair SacII-NsiI fragment from pBR322.VNERK.HC.H.

pBR322.VNERK.HC.H is a derivative of pBR322.VNERK.HC plasmid (see above) in which the "hole" mutations (T366S, L368A, Y407V) were introduced (Merchant et al., Nature Biotechnology, 16:677-681 (1998)). This intermediate plasmid is designated as pxcM11C.H.

*pxcM*11*C.H-Fc.K*

The second step introduces the "knob" mutation into the Fc fragment, and involved the ligation of two DNA fragments. The first was the vector pxcM11C.H in which the small ClaI-HpaI fragment had been removed. The second part of the ligation was an approximately 1198 base pair ClaI-HpaI fragment from pBR322.Fc.K encoding the expression cassette for the Fc fragment with the "knob" mutation. pBR322.Fc.K is a derivative of the pBR322.Fc plasmid (see above) which codes for the expression of the Fc fragment with the "knob" mutation (T366W) (Merchant et al., Nature Biotechnology, 16:677-681 (1998)) and all the control elements described above. This ligation resulted in the desired plasmid designated pxcM11C.H-Fc.K.

*Expression and characterization of Anti-c-Met One Armed Fab/c antibody*

Small-scale inductions of the antibodies were carried out using the parent construct (pxcM11C for 5D5 anti-c-Met) and the one armed Fab/c antibody construct (pxcM11C-Fc). For small scale expression of each construct, the *E. coli* strain 33D3 (W3110 ΔfhuA (ΔtonA) ptr3 lac Iq lacL8 ΔompT Δ(nmpc-fepE) degP41 kan$^R$) was used as host cells. Following transformation, selected transformant picks were inoculated into 5 mL Luria-Bertani medium supplemented with carbenicillin (50 μg/mL) and grown at 30°C on a culture wheel overnight. Each culture was then diluted (1:100) into C.R.A.P. phosphate-limiting media (Simmons et al., J. Immunol. Methods 263:133-147 (2002)). Carbenicillin was then added to the induction culture at a concentration of 50 μg/mL and the culture was grown for approximately 24 hours at 30°C on a culture wheel. Unless otherwise noted, all shake flask inductions were performed in a 5 mL volume.

Non-reduced whole cell lysates from induced cultures were prepared as follows: (1) 1 $OD_{600}$-mL induction samples were centrifuged in a microfuge tube; (2) each pellet was resuspended in 90 μL TE (10 mM Tris pH 7.6, 1 mM EDTA); (3) 10 μL of 100 mM iodoacetic acid (Sigma I-2512) was added to each sample to block any free cysteines and prevent disulfide shuffling; (4) 20 μL of 10% SDS was added to each sample. The samples were vortexed, heated to about 90°C for 3 minutes and then vortexed again. After the samples had cooled to room temperature, 750 μL acetone was added to precipitate the protein. The samples were vortexed and left at room temperature for about 15 minutes. Following centrifugation for 5 minutes in a microcentrifuge, the supernatant of each sample was aspirated off, and each protein pellet was resuspended in 50 μL $dH_20$ + 50 μL 2X NOVEX SDS sample buffer. The samples were then heated for 4 minutes at about 90°C, vortexed well and allowed to cool to room temperature. A final 5 minute centrifugation was then done and the supernatants were transferred to clean tubes.

Reduced whole cell lysates from induced cultures were prepared as follows: (1) 1 $OD_{600}$-mL induction samples were centrifuged in a microfuge tube; (2) each pellet was resuspended in 90 μL TE (10 mM Tris pH 7.6, 1 mM EDTA); (3) 10 μL of 1 M dithiothreitol (Sigma D-5545) was added to each sample to reduce disulfide bonds; (4) 20 μL of 10% SDS was added to each sample. The samples were vortexed, heated to about 90°C for 3 minutes and then vortexed again. After the samples had cooled to room temperature, 750 μL acetone was added to precipitate the protein. The samples were vortexed and left at room temperature for about 15 minutes. Following centrifugation for 5 minutes in a microcentrifuge, the supernatant of each sample was aspirated off and each protein pellet was resuspended in 10 μL 1 M dithiothreitol + 40 μL dH20 + 50 μL 2X NOVEX SDS sample buffer. The samples were then heated for 4 minutes at about 90°C, vortexed well and allowed to cool to room temperature. A final 5 minute centrifugation was then done and the supernatants were transferred to clean tubes.

Following preparation, 5 - 8 μL of each sample was loaded onto a 10 well, 1.0 mm NOVEX manufactured 12% Tris-Glycine SDS-PAGE and electrophoresed at~120 volts for 1.5 2 hours. The resulting gels were then either stained with Coomassie Blue or used for Western blot analysis.

For Western blot analysis, the SDS-PAGE gels were electroblotted onto a nitrocellulose membrane (NOVEX) in 10 mM CAPS buffer, pH 11 + 3% methanol. The membrane was then blocked using a solution of 1X NET (150 mM NaCl, 5 mM EDTA, 50 mM Tris pH 7.4, 0.05% Triton X-100) + 0.5% gelatin for approximately 30 min - 1 hours rocking at room temperature. Following the blocking step, the membrane was placed in a solution of 1X NET + 0.5% gelatin + anti-Fab antibody (peroxidase-conjugated goat IgG fraction to human IgG Fab; CAPPEL #55223) for an anti-Fab Western blot analysis. The anti-Fab antibody dilution ranged from 1:50,000 to 1:1,000,000 depending on the lot of antibody. Alternatively, the membrane was placed in a solution of 1X NET + 0.5% gelatin + anti-Fc antibody (peroxidase-conjugated goat IgG fraction to human Fc fragment; BETHYL #A80-104P-41) for an anti-Fc Western blot analysis. The anti-Fc antibody dilution ranged from 1:50,000 to 1:250,000 depending on the lot of the antibody. The membrane in each case was left in the antibody solution overnight at room temperature with rocking. The next morning, the membrane was washed a minimum of 3 x 10 minutes in 1X NET + 0.5% gelatin and then 1 x 15 minutes in TBS (20 mM Tris pH 7.5,

500 mM NaCl). The protein bands bound by the anti-Fab antibody and the anti-Fc antibody were visualized using Amersham Pharmacia Biotech ECL detection and exposing the membrane to X-Ray film.

The anti-Fab Western blot results for the anti-c-Met Fab/c antibody expression are shown in Figure 1. They reveal the expression of fully folded and assembled full-length antibody in lane 1 and the one armed Fab/c antibody in lane 2. Note that the anti-Fab antibody is not able to bind the Fc fragment, and it therefore is not detected. For the non-reduced samples, the co-expression of the Fc fragment with the full-length anti-c-Met antibody results in a substantial shift from fully folded and assembled full-length antibody to fully folded and assembled one armed Fab/c antibody. For the reduced samples, there are similar quantities of heavy and light chain detected for the full-length anti-c-Met antibody and the one armed anti-c-Met Fab/c antibody. There is a slight increase in the amount of light chain precursor with the one armed anti-c-Met Fab/c construct, possibly due to a slight back up in the secretory pathway.

Similarly, the anti-Fc Western blot results are shown in Figure 2 and they also reveal the expression of fully folded and assembled one armed Fab/c antibody in lane 2. The anti-Fc antibody is not able to bind light chain, and therefore it is not detected. For the non-reduced samples, the co-expression of the Fc region with full-length anti-c-Met antibody shows the shift from fully folded and assembled full-length antibody to fully folded and assembled one armed Fab/c antibody. In addition, the Fc polypeptide monomer and dimer are also detected on the anti-Fc Western blot. For the reduced samples, there are similar quantities of heavy chain detected for the full-length anti-c-Met antibody and the one armed anti-c-Met Fab/c antibody expression. There is also a small amount of precursor Fc fragment, possibly due to some back up in the secretory pathway with the one armed Fab/c antibody construct.

As evident from the data described above, it is possible to generate an immunoglobulin population wherein the primary antibody species is the desired one-armed Fab/c antibody. However, the intact form of antibody (i.e., the fully folded and assembled anti-c-Met full-length antibody) was still detectable by both anti-Fab and anti-Fc Western blot analysis. Since the intact form of the anti-c-met 5D5 antibody is an agonist of the c-met receptor, which is undesirable in a therapeutic scheme that requires an antagonistic effect, it is generally desirable to minimize the amount of the intact form of antibody that is generated.

*Expression and characterization of one armed Fab/c anti-c-Met antibody comprising protuberance and cavity (alse referred to below as "Knobs into Holes")*

To further minimize the formation of full-length anti-c-Met antibody in the preparation of the anti-c-Met Fab/c antibody, "knobs into holes" mutations were made in the CH3 domain of the Fc essentially as described by Merchant et al. (Nature Biotechnology, 16:677-681 (1998)). A construct was prepared for the one armed Fab/c anti-c-Met antibody (pxcM11C.H-Fc.K) by introducing the "hole" mutations (T366S, L368A, Y407V) into the full-length heavy chain, and the "knob" mutation (T366W) into the Fc fragment.

The full-length anti-c-Met antibody (pxcM11C), one armed Fab/c anti-c-Met antibody (pxcM11C-Fc), and one armed Fab/c "knobs into holes" anti-c-Met antibody (pxcM11C.H.Fc.K) constructs were then expressed in the same manner as described above. Whole cell lysates were prepared, separated by SDS-PAGE, transferred to nitrocellulose, and detected with the previously described goat anti-human Fab conjugated antibody and goat anti-human Fc conjugated antibody.

The anti-Fab Western blot results are shown in Figure 3, and they show a significant improvement in folding and assembly of the one armed Fab/c "knobs into holes" anti-c-Met antibody. In addition, the anti-Fab Western blot results show the reduction of full-length anti-c-Met antibody to undetectable levels. Again, it is important to note that the anti-Fab antibody is not able to bind the Fc fragment. For the non-reduced samples, the expression of the one armed "knobs into holes" anti-c-Met antibody results in a substantial shift from fully folded and assembled full-length antibody to fully folded and assembled one armed Fab/c antibody. There is also a significant improvement in folding and assembly of one armed Fab/c antibody moving from the wild type Fc to the "knobs into holes" Fc. For the reduced samples, there are similar quantities of heavy chain detected for the full-length, one armed Fab/c, and the one armed Fab/c "knobs into holes" anti-c-Met antibodies. There also appears to be an increase in the amount of processed light chain and a decrease in light chain precursor with the one armed Fab/c "knobs into holes" anti-c-Met construct.

Similarly, the anti-Fc Western blot results in Figure 4 show a significant improvement in folding and assembly of the one armed Fab/c "knobs into holes" over the wild type one armed Fab/c anti-c-Met antibody. Again, the anti-Fc Western blot results show the reduction of full-length anti-c-Met antibody to undetectable levels. The anti-Fc antibody is not able to bind light chain, and therefore it is not detected. For the reduced samples, there are similar quantities of heavy chain detected for the full-length, the wild type one armed Fab/c, and the one armed Fab/c "knobs into holes" anti-c-Met antibodies.

EXAMPLE 2: Pharmacokinetic characteristics and therapeutic efficacy of antibodies of the invention

*Material & Methods*

Materials - HGF and c-Met-IgG were produced at Genentech, as described previously (8;14). Maxisorb microtiter plates were purchased from NUNC (Rosklide, Denmark). Anti-hFc was purchased from Jackson Immunochemical (West Grove, PA). HRP-Streptavidin was purchased from Zymed (South San Francisco, CA). $^3$H-thymidine was purchased from Amersham, Inc. (Arlington Heights, IL). MDA-MB-435 cells were obtained from ATCC (Rockville, MD). Pyroglutamate aminopeptidase was obtained from Takara Biochemicals (Berkeley, CA). NHS-X-Biotin was purchased from Research Organics (Cleveland, OH). Immobilon-PSQ PVDF was purchased from Millipore (Marlborough, MA). Superscript II RNase H- Reverse Transcriptase was from Gibco-BRL (Gaithersburg, MD). Taq polymerase was from Perkin Elmer-Cetus (Foster City, CA), Bakerbond ABX, 40 μ particle size was from J. T. Baker (Phillipsburg, NJ) and SP-Sepharose High Performance resin was from Pharmacia Biotech, Inc. (Piscataway, NJ). Biotin-anti-P-Tyr was from Upstate Biotech (Lake Placid, NY), TMB peroxidase substrate was purchased from KPL (Gaithersburg, MD).

*Generation of antibodies and Fab fragments*

Production of Anti-c-Met Monoclonal Antibodies

Production of anti-c-met monoclonal antibodies, including the 5D5 antibody, has been described. See, e.g., U.S. Pat. Nos. 5,686,292; 5,646,036; 6,207,152; 6,214,344 & 6,468,529. BALB/c mice were immunized in each rear footpad with 2.5 ug of soluble c-Met-IgG (Mark et al., J.Biol.Chem. (1992), 267:26166-26171) suspended in MPL/TDM adjuvant on day 0, 7, 14, 21, 28, 266, 273, and 279. Four days after the last immunization the lymph node cells were harvested and fused with P3/X63-Ag8U1 myeloma cells ((13)) using 35% polyethyleneglycol as described (Laskov et al.,Cell.Immunol. (1980), 55:251). Hybridoma cell lines secreting antibody specific for c-Met were initially selected by a capture ELISA then subsequently screened by flow cytometry using BAF3 transfected cells expressing c-Met. Selected hybridomas were further tested for their ability to inhibit biotin-HGF binding to c-Met-IgG, as described below. The hybridomas were cloned twice by limiting dilution and then further characterized for their antagonistic and agonistic abilities. Ascites was produced in balb/c mice and monoclonal antibodies were purified using a protein G affinity column. The protein concentration was determined by the absorbance at 280 nm using an extinction coefficient of 1.4.

Generation and purification of Native Fab

Antibody 5D5 was dialyzed overnight in 20 mM Phosphate, 10 mM EDTA buffer and then concentrated to 7 mg/ml in a Centricon 30. One half ml of immobilized papain (Pierce, Rockford, Il) was washed with digestion buffer, then 10 mg of 5D5 was added and incubated overnight at 37°C with shaking at 200 rpm. One and one-half ml of binding buffer was added to the mixture, then the supernatant was separated from the beads and passed over a Protein A column that was previously equilibrated with binding buffer. Additional binding buffer was passed over the column and the eluate collected in 1 ml fractions. The absorbance of each fraction was read at 280 nm and the eluates containing the Fab fragment were pooled. The protein was dialyzed overnight in PBS and the protein concentration determined by its absorbance at 280 nm using an extinction coefficient of 1.53. The Fab fragment was further purified by gel filtration to remove residual F(ab')2.

N-terminal Sequencing of 5D5 Fab

An aliquot of 5D5 Fab was resolved on a 4-20% gradient SDS gel and electroblotted onto PVDF (Immobilon-PSQ) membrane for 1 hr at 250 mA constant current in a BioRad Trans-Blot transfer cell ((9)). The membrane was stained with 0.1% Coomassie Blue R-250 in 50% methanol, 0.5 minutes and destained for 2-3 minutes with 10% acetic acid in 50% methanol. The membrane was thoroughly washed with water and allowed to dry before sequencing on a model 473A automated protein sequencer, using a Blott® cartridge (Applied Biosystem). Peaks were integrated with Justice Innovation software using Nelson Analytical 760 interfaces. Sequence interpretation was performed on a DEC alpha (Henzel et al., J.Chromatog. (1996), 404:41-52).

Obtaining sequence of the 5D5 heavy chain required deblocking, which was performed as follows. The Fab fragment was reduced with 7 mM DTT at 45°C for 1h and alkylated with 180 mM isopropylacetamide at 25°C for 20 minutes (Krutzsch & Inman, Anal.Biochem. (1993), 209:109-116). The alkylated Fab fragment was then exchanged 3X in a Microcon-10 with 0.1M sodium phosphate containing 10 mM DTT (digestion buffer) and digested with 1 mU of pyroglutamate aminopeptidase at 45°C for 3h in 20 μl of digestion buffer. The deblocked Fab was then transferred to PVDF and sequenced as described above.

Cloning and Recombinant Expression of 5D5 Fab

N-terminal sequence data were used to design PCR primers specific for the 5' ends of the variable regions of the light and heavy chains, while 3' primers were designed to anneal to the consensus framework 4 of each chain (Kabat et al., Sequences of proteins of immunological interest (1991), Public Health Service, National Institutes of Health, Bethesda, MD). The primers were also designed to add restriction enzyme sites for cloning. Total RNA, extracted from $10^8$ cells of hybridoma 5D5 with a Stratagene RNA isolation kit, was used as substrate for RT-PCR. Reverse transcription was performed under standard conditions (Kawasaki, Amplification of RNA. In PCR Protocols: A Guide to Methods and Applications, pp. 21-27 (M.A. Innis, D.H. Gelfand, J.J. Sninsky, and T.J. White, editors) (Academic Press, Inc., San Diego; 1990)) using the framework 4 specific primers and Superscript II RNase H- Reverse Transcriptase. PCR amplification employed Taq polymerase, as described (Kawasaki, Amplification of RNA. In PCR Protocols: A Guide to Methods and Applications, pp. 21-27 (M.A. Innis, D.H. Gelfand, J.J. Sninsky, and T.J. White, editors) (Academic Press, Inc., San Diego; 1990)) except that 2% DMSO was included in the reaction mix. Amplified DNA fragments were digested with restriction enzymes *Sfi I* and *Rsr II* (light chain) or *Mlu I* and *Apa I* (heavy chain), gel purified, and cloned into a derivative of expression plasmid pAK19 (Carter et al., Bio/Technol. (1992), 10:163-167). This vector, pXCA730, has been modified by site-directed mutagenesis (Kunkel, Proc.Natl.Acad.Sci. USA (1985), 82:488) to contain unique restriction sites between the ST II signal sequences and the variable domains, and at the junction of the variable and constant domains of each chain. The light and heavy chain variable domain cDNAs were inserted upstream and in frame to human $C_\kappa$ and CH1 domains. The C-terminal cysteine of the heavy chain in pAK19, which could form a disulfide bridge to give $F(ab')_2$ molecules, was removed to permit expression of only the Fab form of the antibody.

Recombinant 5D5 Fab was expressed in *E. coli* K12 strain 33B6 (W3110 *ton*A *pho*A E15 *deo*C KanR *ilv*GR *deg*PD)*arg*F-*lac*)169) (Rodriques et al., Cancer Res. (1995), 55:63-70), as described by Carter et al. (Carter et al., Bio/Technol. (1992), 10:163-167). The cell pellet from a 10-L fermentation was harvested by continuous feed centrifugation, frozen and stored at -70°C. A portion of the pellet was suspended in extraction buffer, which consisted of 120 mM MES, pH 6, and 5 mM EDTA (5 ml/gram of paste). The suspension was mixed thoroughly using an ultraturrax (Janke and Kunkel) for approximately 15 minutes at 4°C. Intact cells were then disrupted using 2 passes through a cell homogenizer (Microfluidizer, by Microfluidics Corporation, Newton, MA) fitted with a cooling coil. The suspension was then adjusted to 0.1% (v/v) polyethyleneimine using a 5% (v/v) stock which had been adjusted to pH 6. Intact cells and PEI-flocculated debris were separated from the soluble fraction by centrifugation at 25,400 x g for 30 minutes. The supernatant was adjusted to a conductivity less than 4 mS by addition of purified water and loaded onto a column (1 x 10 cm) of Bakerbond ABX, 40 $\mu$ particle size. The column had been equilibrated in 50 mM MES, 5 mM EDTA, pH 6. All steps were done at a linear flow rate of 100 cm/h. After loading the conditioned supernatant, the column was washed with equilibration buffer until the absorbance of the column effluent was equivalent to baseline. Elution was performed using a 16-column volume, linear gradient from 0 to 100 mM ammonium sulfate in equilibration buffer. Column fractions were analyzed by SDS-polyacrylamide gel electrophoresis and fractions which contained the Fab were pooled. The conducticitiy of the pool from the ABX column was lowered to less than 4 mS and loaded onto a column (1 x 10 cm) of SP-Sepharose High Performance resin that had been equilibrated in 25 mM MOPS buffer, pH 6.9. All steps were performed at a linear flow rate of 100 cm/h. Following the load, the column was washed with one column volume of equilibration buffer. The 5D5 Fab was then eluted from the column using a 16-column volume, linear gradient from 0 to 200 mM sodium acetate in equilibration buffer. Column fractions were analyzed by SDS-polyacrylamide gel electrophoresis and fractions which contained the FAb were pooled.

One-Armed 5D5 Protein Production Small-scale

The expression plasmid pxcM11C.H-Fc.K (as described above) was used to transform the E.coli strain 33D3 (W3110 kanR ΔfhuA (ΔtonA) ptr3 lacIq lacL8 ompTΔ (nmpc-fepE) degP), and transformants were then grown overnight at 30 degrees C in LB media with added carbenicillin (50ug/mL). The LB culture was diluted 100 fold into C.R.A.P. media (1) containing carbenicillin (50ug/mL) and grown for approximately 24 hours with shaking at 30 degrees C. Small aliquots were removed to verify antibody expression by SDS-PAGE and Western analysis using either an anti-Fab antibody (peroxidase-conjugated goat IgG fraction to human IgG Fab; CAPPEL #55223) or an anti-Fc antibody (peroxidase-conjugated goat IgG fraction to human Fc fragment; Bethyl #A08-104P-41). The remaining culture was then centrifuged, and the cell paste frozen at -70 degrees C until the start of the antibody purification step.

Purification of one-armed 5D5. Frozen cell paste was thawed and suspended in 10 volumes (w/v) lysis buffer (25mM tris-HCl, 5mM EDTA, pH7.5), then centrifuged. The insoluble pellet was resuspended in lysis buffer using a Polytron homogenizer (Kinematica A.G, Switzerland) and the cells disrupted by passage through a Microfluidizer (Microfluidics, Newton, Mass). Polyethyleneimine (Sigma) 0.1% (v/v) was added to the lysate, followed by stirring at 4°C for one hour, then centrifugation at 15,000 x g. The resulting supernatant was mixed with a protein A affinity resin and stirred overnight at 4°C. The resin was allowed to settle, the supernatant poured off, and the resin poured into a column

attached to a liquid chromatography system (Varian Inc, Palo Alto, CA). The column was washed with 10mM tris-HCl, 1mM EDTA buffer, pH 7.5, followed by 0.5M NaCl in the same buffer, then eluted with a gradient from pH6 to pH2 in 50mM sodium citrate, 0.1M NaCl buffer. Eluted fractions were immediately adjusted to a final concentration of 2M urea and pH 5.4, and analyzed by SDS-polyacrylamide gel electrophoresis (SDS-PAGE). Fractions containing one-armed anti-cMet were pooled and subjected to cation exchange chromatography on an SP Sepharose column (Amersham Biosciences, Piscataway, NJ) equilibrated with 25mM MES, 2M urea pH 5.4. The column was eluted with a gradient of 0 - 1M NaCl in 25mM MES, pH 5.4. Following SDS-PAGE analysis, the pooled eluate was adjusted to 0.4M sodium sulfate, pH6, and loaded onto a Hi-Propyl (J.T.Baker, Phillipsburg, NJ) column equilibrated with 0.4M sodium sulfate, 25mM MES pH6. The column was eluted with a gradient of 0.4M - 0M sodium sulfate in 25mM MES, pH6. The resulting eluate, following SDS-PAGE analysis, was concentrated using CentriPrep 10 (Millipore Corp, Bedford, MA) then subjected to size exclusion chromatography on a Superdex 200 column (Amersham Biosciences) equilibrated with 10mM sodium succinate, 0.15M NaCl, pH 5.0.

Protein concentrations were determined by quantitative amino acid analysis. Endotoxin levels were determined by LAL assay. These antibody preparations were used for subsequent analysis.

*Assays*

Cell Culture

A549 lung carcinoma cells were cultured in MEM supplemented with 10% FBS. BaF3-cMet cells, which were transfected with human cMet as described previously (Schwall et al., J.Cell Biol. (1996), 133:709-718) and BaF3-neo cells, which were transfected with the vector, were cultured in RPMI 1640 supplemented with 10% FCS, 5% WEHI-231 cell culture conditional medium (contain IL-3), 2 mM glutamine. β-mercaptoethanol (4 μl/L), 0.5 mg/ml G418. U87 & U118 glioblastoma cells, 786-0 renal carcinoma cells were cultured in RPMI 1640 containing 10% FCS.

HGF-cMet Binding

HGF binding studies were conducted using biotin-HGF in a solid-phase system in which c-Met-IgG is captured through the IgG domain onto microtiter plates. HGF was biotinylated by incubating with 20-fold molar excess NHS-X-Biotin in 0.1 M NaHCO3, pH 8.5. The NHS-X-biotin was divided into 4 increments that were added at 15 minute intervals, with stirring at room temperature. Nonconjugated biotin was removed by dialysis and the labeled material was stored at 4° C. Microtiter plates were coated with 2 μg/ml AffiniPure rabbit anti-human IgG, Fc (Jackson ImmunoResearch) in coating buffer (0.05 M carbonate/bicarbonate, pH 9.6) overnight at 4 °C. The plates were blocked by PBS containing 0.5% BSA, pH 7.4 at room temperature (RT) for 1 hour, followed by 2 hours incubation with 2 μg/ml cMet-IgG. Then 53 ng/ml biotin labeled HGF with or without 0.01-1,000 nM competitors of cold HGF, anti-cMet 5D5 mAb, Fab or one-armed antibody were added at RT for 1 hour. Plates were added with horseradish peroxidase (HRP)-streptavidin (1:10,000 dilution, Amersham) at RT for 1 hour, followed by phosphatase substrate CP-nitrophenyl phosphate (Kirkegaard & Perry Laboratories), and absorbance was measured at 405 nm.

Results are shown in Figure 5. One-armed anti-c-met 5D5 antibody performed similarly to anti-c-met Fab in the competitive binding assay.

Tyrosine-phosphorylation of c-Met by KIRA

Tyrosine phosphorylation of c-Met was measured by a sandwich ELISA, based on the methods of Sadick et al. in which solubilized receptor is captured onto a plate coated with anti-receptor antibody and detected with anti-P-Tyr (Sadick et al., Anal.Biochem. (1996), 235:207-214). U87 cells were plated at $10^6$/ml in 96-well plate at 37 °C overnight. The medium was then changed to MEM, 0.1% FBS, with HGF and/or antibodies for 10 min. Cells were then extracted in 100 μl cell lysis buffer (20mM Tris, PH 7.5, 150 mM NaCl, 1 mM Na2EDTA, 1nM EGTA, 1% Triton, 1x protease inhibator cocktail (Sigma), 1x phosphatase inhibitor cocktail II (Sigma)) for 30 min at RT on a plate shaker, and stored on ice or -70 °C. The lysates were added to plates that had been coated with anti-cMet mAb 1949 (Genentech) overnight. Phospho-tyrosine cMet was detected with 1: 4000 diluted biotinylated anti-phosphotyrosine (4G10, Upstate), followed by HRP-streptavidin and color development with TMB. Total cMet was similarly measured using 1:10,000 anti-cMet antibody.

Results are shown in Figure 6.

Cell Proliferation, Migration Assays

BaF3 is a murine IL-3 dependent lymphoid cell that normally does not express cMet and does not respond to

HGF. However, in BaF3-hMet, derived by transfection with a normal, full-length cDNA for human c-Met (Schwall et al., J. Cell Biol. (1996), 133:709-718), HGF stimulates proliferation and survival in the absence of IL-3. BaF3-hMet and BaF3-neo cells are routinely passaged in RPMI 1640, 5% fetal bovine serum, 4 μl/L β-ME, 100 U/ml penicillin, 100 μg/ml streptomycin sulfate, 2 mM L-glutamine, and 5% WEHI-conditioned medium as a source of IL-3. To measure HGF-dependent proliferation the number of cells after 3 days of treatment was quantitated by adding 25 μl Alarma Blue (Trek Diagnostic Systems) and measuring fluorescence intensity 4 hours later. As a control for specificity, one-armed 5D5 was also tested in BaF3-hMet cells stimulated with IL-3.

Results are shown in Figure 7.

Migration of MDAMB-435-PGL cells and U87 cells was evaluated using a modified Boyden chamber assay. The cells were plated (2x10 4/well) onto the upper chamber with 5 μm pores polycarbonate filter coated with 10 μg/ml fibronectin. 100 ng/ml of HGF with or without 5D5 Fab or one-armed antibody at the indicated amounts were added to the medium in lower chamber. Cells were cultured overnight. Cells were scraped off topside of the filter membrane using special sponge swab, and the cells that had migrated to the undersurface of the filter were fixed and stained with YO-PRO-3 iodide (Molecular Probes), then counted by fluorescent microscopy.

Results are shown in Figure 8.

*Pharmacokinetics*

OA-5D5 or 5D5 Fab (5 mg/kg) was injected intravenously into nude mice. At the indicated time points, serum samples were collected from 4 mice and assayed by sandwich ELISA in which it was captured onto a plate coated with cMet-IgG and then detected with a light-chain specific secondary antibody.

Results are shown in Figure 9A. The half life of one-armed 5D5 antibody was significantly increased compared to its Fab counterpart.

To determine whether OA-5D5 was degrading in vivo, a volume of serum corresponding to 40 ng of OA-5D5 by ELISA from the day 7 PK samples were run on a SDS-PAGE gel under reducing or non-reducing condition. The gel was transferred on nitrocellulose and blotted by HRP conjugated anti-human Fc (1:5,000, human specific, Jackson Labs). An equal volume of serum from naïve mice was run in parallel as control.

Results are shown in Figure 9B. Serum one-armed 5D5 antibody was intact on day 7 following administration.

*In Vivo Tumor Efficacy Studies.*

Efficacy studies were performed using female athymic nude mice at age of 4-6 weeks inoculated subcutaneously with 2.5 million A549 (human lung carcinama) or U87 MG cells (human glioblastoma which secrete autocrine HGF and express cMet). Treatment with the one-armed 5D5 antibody, the 5D5 Fab antibody or a control antibody (anti-gp120) was begun either at the time of tumor cell inoculation (i.e., adjuvant treatment) or after tumors were allowed to grow to ~150 $mm^3$. All antibodies were administered intraperitoneally once per week for 4 weeks. Note that only the one-armed 5D5 antibody was tested in the adjuvant treatment regimen (with the anti-gp120 control).

Figure 10 shows results for tumors generated by inoculation with U87 MG cells. As shown in Figure 10A (adjuvant treatment) and Figure 10B (treatment following establishment of tumors), one-armed 5D5 antibody was capable of inhibiting or causing regression of tumors.
As shown in Figure 10B, one-armed 5D5 antibody had superior therapeutic efficacy compared to its Fab counterpart. (Interestingly, one-armed 5D5 antibody at 100 nM exhibited minimal effects on U87 cell number in vitro.)

The results for treatment of tumors generated from inoculation with the A549 cells were negative, which provides a specificity control which confirmed that the effects of the one-armed 5D5 anti-c-met antibody against the U87 tumors were not due to nonspecific toxicity.

The one-armed 5D5 anti-c-met antibody can also tested for ability to modulate tumor development using other art-established *in vivo* tumor models, for example the Oncotest model described in U.S. Pat. No. 6,271,342. Tumor growth of BxPC-3 (pancreatic) (ATCC No. CRL-1687) cells coinoculated with MRC-5 fibroblasts (ATCC CCL-171) showed a 50% inhibition when one-armend 5D5 antibody was administered at 30 mg/kg, 2x/week. Other illustrative data are listed below:

- ~30% inhibition of tumor growth in Oncotest RXF1220 (renal) with 10 mg/kg, q7d (i.e., every 7 days) of the one-armed 5D5 antibody;
- <20% inhibition of tumor growth in (i) Oncotest PAXF736 (pancreatic) with 10 mg/kg, q7d; (ii) Oncotest GXF97 (gastric) with 10 mg/kg, q7d; (iii) Oncotest LXFA526 (lung) with 30 mg/kg, q7d; (iv) Oncotest LSFA297 (lung) with 30 mg/kg, q7d;
- No activity was observed in A549 xenografts with 10 mg/kg, q7d of the one-armed 5D5 antibody;
- No activity was observed in Oncotest LXFA650 (lung) with 30 mg/kg, q7d.

Additional statements of invention

The following numbered clauses, describing aspects of the invention, are part of the description.

1. An antibody fragment comprising a single antigen binding arm and an Fc region that increases stability of said antibody fragment compared to a Fab molecule comprising said antigen binding arm, wherein the Fc region comprises a complex of a first and a second Fc polypeptide, wherein one but not both of the Fc polypeptides is an N- terminally truncated heavy chain.
2. The antibody fragment of clause 1 wherein the antibody fragment is aglycosylated.
3. The antibody fragment of clause 1 or 2 wherein the antibody fragment has little to no immunosuppressive properties.
4. The antibody fragment of clause 3 wherein said immunosuppressive properties comprise ability to effect T cell depletion.
5. The antibody fragment of any of clauses 1-4 which does not possess substantial effector function other than FcRn binding.
6. The antibody fragment of clause 5 wherein said effector function is complement lysis.
7. The antibody fragment of any of clauses 1-6 wherein the antibody fragment binds FcRn.
8. The antibody fragment of any of clauses 1-7 which does not specifically bind a T cell surface antigen.
9. The antibody fragment of clause 8 wherein said T cell surface antigen is CD3 or CD4.
10. The antibody fragment of clause 9 wherein said T cell surface antigen is CD3.
11. The antibody fragment of any of clauses 1-10 which specifically binds a tumor antigen.
12. The antibody fragment of any of clauses 1-11 which specifically binds a cell surface receptor that is activated upon receptor dimerization.
13. The antibody fragment of any of clauses 1-12 wherein the antibody fragment comprises a first polypeptide comprising a light chain variable domain, a second polypeptide comprising a heavy chain variable domain and said first Fc polypeptide, and a third polypeptide comprising said second Fc polypeptide.
14. The antibody fragment of clause 13 wherein the first polypeptide comprises a non- human light chain variable domain fused to a human light chain constant domain.
15. The antibody fragment of clause 13 wherein the first polypeptide comprises a CDR from a non-human species fused to a humanized or human framework sequence.
16. The antibody fragment of clause 13 wherein the second polypeptide comprises a non- human heavy chain variable domain fused to a human heavy chain constant domain.
17. The antibody fragment of clause 13 wherein the second polypeptide comprises a CDR from a non-human species fused to a humanized or human framework sequence.
18. The antibody fragment of clause 13 wherein the third polypeptide comprises an N- terminally truncated heavy chain which comprises at least a portion of the hinge sequence at the N terminus.
19. The antibody fragment of any of clauses 1-18 wherein the two Fc polypeptides are covalently linked.
20. The antibody fragment of any of clauses 1-19 wherein the two Fc polypeptides are linked through intermolecular disulfide bonds at the hinge region.
21. The antibody fragment of any of clauses 1-20 wherein the antibody fragment when bound to a target molecule inhibits target molecule multimerization.
22. The antibody fragment of any of clauses 1-21 wherein the antibody fragment when bound to a target molecule inhibits binding of a cognate binding partner to the target molecule.
23. The antibody fragment of any of clauses 1-22 wherein the first Fc polypeptide and the second Fc polypeptide meet at an interface, and the interface of the second Fc polypeptide comprises a protuberance which is positionable in a cavity in the interface of the first Fc polypeptide.
24. The antibody fragment of any of clauses 1-23 wherein the second Fc polypeptide has been altered from a template/ original polypeptide to encode the protuberance or the first Fc polypeptide has been altered from a template/ original polypeptide to encode the cavity, or both.
25. The antibody fragment of any of clauses 1-24 wherein the second Fc polypeptide has been altered from a template/ original polypeptide to encode the protuberance and the first Fc polypeptide has been altered from a template/ original polypeptide to encode the cavity, or both.
26. The antibody fragment of any of clauses 1-25 wherein the first Fc polypeptide and the second Fc polypeptide meet at an interface, wherein the interface of the second Fc polypeptide comprises a protuberance which is positionable in a cavity in the interface of the first Fc polypeptide, and wherein the cavity or protuberance, or both, have been introduced into the interface of the first and second Fc polypeptides respectively.
27. The antibody fragment of any of clauses 24-26 wherein the protuberance and cavity have been introduced into the interface of the respective Fc polypeptides.

28. The antibody of any of clauses 24-27 wherein the protuberance and cavity each comprise a naturally occurring amino acid residue.

29. The antibody fragment of any of clauses 24-28 wherein the Fc polypeptide comprising the protuberance is generated by replacing an original residue from the interface of a template/original polypeptide with an import residue having a larger side chain volume than the original residue.

30. The antibody fragment of any of clauses 24-29 wherein the Fc polypeptide comprising the protuberance is generated by a method comprising a step wherein nucleic acid encoding an original residue from the interface of said polypeptide is replaced with nucleic acid encoding an import residue having a larger side chain volume than the original.

31. The antibody fragment of clause 29 or 30 wherein the original residue is threonine.

32. The antibody fragment of any of clauses 29-31 wherein the import residue is arginine (R).

33. The antibody fragment of any of clauses 29-31 wherein the import residue is phenylalanine (F).

34. The antibody fragment of any of clauses 29-31 wherein the import residue is tyrosine (Y).

35. The antibody fragment of any of clauses 29-31 wherein the import residue is tryptophan (W).

36. The antibody fragment of any of clauses 23-28 wherein the Fc polypeptide comprising the cavity is generated by replacing an original residue in the interface of a template/original polypeptide with an import residue having a smaller side chain volume than the original residue.

37. The antibody fragment of any of clauses 23-28 and 36 wherein the Fc polypeptide comprising the cavity is generated by a method comprising a step wherein nucleic acid encoding an original residue from the interface of said polypeptide is replaced with nucleic acid encoding an import residue having a smaller side chain volume than the original.

38. The antibody fragment of clause 36 or 37 wherein the original residue is threonine.

39. The antibody fragment of clause 36 or 37 wherein the original residue is leucine.

40. The antibody fragment of clause 36 or 37 wherein the original residue is tyrosine.

41. The antibody fragment of any of clauses 36-40 wherein the import residue is not cysteine (C).

42. The antibody fragment of any of clauses 36-40 wherein the import residue is alanine (A).

43. The antibody fragment of any of clauses 36-40 wherein the import residue is serine (S).

44. The antibody fragment of any of clauses 36-40 wherein the import residue is threonine (T).

45. The antibody fragment of any of clauses 36-40 wherein the import residue is valine (V).

46. The antibody fragment of any of clauses 36-45 wherein the Fc polypeptide comprising the cavity comprises replacement of two or more original amino acids selected from the group consisting of threonine, leucine and tyrosine.

47. The antibody fragment of any of clauses 36-46 wherein the Fc polypeptide comprising the cavity comprises two or more import residues selected from the group consisting of alanine, serine, threonine and valine.

48. The antibody fragment of any of clauses 36-47 wherein the Fc polypeptide comprising the cavity comprises replacement of two or more original amino acids selected from the group consisting of threonine, leucine and tyrosine, and wherein said original amino acids are replaced with import residues selected from the group consisting of alanine, serine, threonine and valine.

49. The antibody fragment of any of clauses 23-48 wherein the Fc polypeptide comprising the cavity comprises replacement of threonine at position 366 with serine, amino acid numbering according to the EU numbering scheme of Kabat.

50. The antibody fragment of any of clauses 23-48 wherein the Fc polypeptide comprising the cavity comprises replacement of leucine at position 368 with alanine, amino acid numbering according to the EU numbering scheme of Kabat.

51. The antibody fragment of any of clauses 23-50 wherein the Fc polypeptide comprising the cavity comprises replacement of tyrosine with valine.

52. The antibody fragment of any of clauses 23-51 wherein the Fc polypeptide comprising the cavity comprises two or more amino acid replacements selected from the group consisting of T366S, L368A and Y407V.

53. The antibody fragment of any of clauses 23-52 wherein the Fc polypeptide comprising the protuberance comprises replacement of threonine at position 366 with tryptophan, amino acid numbering according to the EU numbering scheme of Kabat.

54. The antibody fragment of any of clauses 1-53 wherein the first and second Fc polypeptides each comprise an antibody constant domain.

55. The antibody fragment of clause 54 wherein the antibody constant domain is a CH2 and/or CH3 domain.

56. The antibody fragment of clause 54 or 55 wherein the antibody constant domain is from an IgG.

57. The antibody fragment of clause 56 wherein the IgG is human $IgG_1$.

58. The antibody fragment of any of clauses 1-57 which is monospecific.

59. The antibody fragment of any of clauses 1-58 which is a monospecific immunoadhesin.

60. The antibody fragment of any of clauses 1-59 which is an antibody-immunoadhesin chimera.
61. A composition comprising a population of immunoglobulins wherein at least 75% of the immunoglobulins is the antibody fragment of any of clauses 1-60.
62. A method of preparing the antibody fragment of any of clauses 1-60, the comprising the steps of:
(a) culturing a host cell comprising nucleic acid encoding the antibody fragment; and
(b) recovering the antibody fragment from the host cell culture.
63. The method of clause 62, wherein polypeptides comprising the antibody fragment are expressed at ratios that results in a population of immunoglobulins wherein at least 50% of the immunoglobulins are the antibody fragment of any of clauses 1-60.
64. The method of clause 63, wherein approximately equimolar amounts of said polypeptides are expressed.
65. The method of cluse 64, wherein nucleic acids encoding the polypeptides are operably linked to translational initiation regions (TIRs) of approximately equal strength.
66. The method of any of clauses 62-65 wherein said host cell is prokaryotic.
67. The method of clause 66, wherein the host cell is E. coli.
68. The method of clause 67, wherein the E. coli is of a strain deficient in endogenous protease activities.
69. The method of any of clauses 62-65, wherein said host cell is eukaryotic.
70. The method of clause 69, wherein the host cell is CHO.
71. The method of any of clauses 62-70, where the antibody fragment is recovered from culture medium.
72. The method of any of clauses 62-70, wherein the antibody fragment is recovered from cell lysate.
73. A method of preparing the antibody fragment of any of clauses 23-60, the comprising the steps of:
(a) culturing a host cell comprising nucleic acid encoding the antibody fragment, wherein the nucleic acid encoding the interface of the second Fc polypeptide has been altered from nucleic acid encoding the original interface of the second Fc polypeptide to encode the protuberance or the nucleic acid encoding the interface of the first Fc polypeptide has been altered from nucleic acid encoding the original interface of the first Fc polypeptide to encode the cavity or both; and
(b) recovering the antibody fragment from the host cell culture.
74. The method of clause 73, wherein the nucleic acid encoding the second Fc polypeptide has been altered from the original nucleic acid to encode the protuberance and the nucleic acid encoding the first polypeptide has been altered from the original nucleic acid to encode the cavity.
75. The method of clause 73 wherein step (a) is preceded by a step wherein nucleic acid encoding an original amino acid residue from the interace of the second Fc polypeptide is replaced with nucleic acid encoding an import amino aicd residue having a larger side chain volume than the original amino acid residue, wherein the import residue with the larger side chain volume comprises the protuberance.
76. The method of clause 73, wherein step (a) is preceded by a step wherein nucleic acid encoding an original amino acid residue in the interface of the first Fc polypeptide is replaced with nucleic acid encoding an import amino acid residue having a smaller side chain volume than the original amino acid residue so as to form the cavity.
77. The method of clauses 73 wherein step (a) is preceded by a step wherein the nucleic acid encoding the first and second Fc polypeptide is introduced in the host cell.
78. A method of preparing the antibody fragment of any of clauses 1-60 comprising the steps of: (a) preparing polypeptides that form the antibody fragment; and (b) allowing heteromultimerization to occur; whereby the antibody fragment is formed.
79. The method of any of clauses 62-78 wherein at least 50% of the immunoglobulin polypeptide complexes that are formed are the antibody fragment of any of clauses 1- 60.
80. The method of any of clauses 62-78 wherein at least 50% of the immunoglobulin polypeptide complexes that are formed are heterotrimers.
81. The method of any of clauses 62-78 wherein wherein step (b) comprises coupling the first Fc polypeptide and the second Fc polypeptide in vitro.
82. The method of any of clauses 62-78 wherein the amino acid sequence of the original interface has been altered so as to generate the protuberance and the cavity in the engineered interface.
83. Isolated nucleic acid encoding the antibody fragment of any of clauses 1-60.
84. A composition comprising two or more recombinant nucleic acids which collectively encode the antibody fragment of any of clauses 1-60.
85. A host cell comprising the nucleic acid of clause 83 or 84.
86. The host cell of clause 85 wherein the nucleic acid encoding the antigen binding arm is present in a single vector.
87. The host cell of clause 85 wherein the nucleic acid encoding the antigen binding arm is present in separate vectors.
88. The host cell of clause 85 wherein the nucleic acid encoding the antigen binding arm and N-terminally truncated heavy chain is present in a single vector.
89. A method of making the antibody fragment of any of clauses 1-60 comprising culturing a host cell comprising the

nucleic acid of clause 83 or 84 so that polypeptides are expressed, and recovering the antibody fragment from the cell culture.

90. The method of clause 89 wherein the antibody fragment is recovered from the cell lysate.
91. The method of clause 89 wherein the antibody fragment is recovered from the cell culture medium.
92. The method of clause 89 wherein the host cell is a prokaryotic cell.
93. The method of clause 90 wherein the host cell is E. coli.
94. The method of clause 89 wherein the host cell is mammalian.
95. A composition comprising the antibody fragment of any of clauses 1-60 and a carrier.
96. A method of generating an antibody fragment comprising a single antigen binding arm and an Fc region that increases stability of the antibody fragment compared to a Fab molecule comprising said antigen binding arm, said method comprising expressing in a suitable host cell nucleic encoding the antigen binding arm and a first and second Fc polypeptide under conditions the permit formation of the antigen binding arm and dimerization of the first and second Fc polypeptides to form said Fc region, wherein one but not both of the Fc polypeptides is an N-terminally truncated heavy chain.
97. The method of clause 96 wherein said method generates a heterogeneous population of immunoglobulins, and wherein at least 50% of the immunoglobulins comprise a single antigen binding arm and an Fc region that increases stability of the antibody fragment compared to a Fab molecule comprising said antigen binding arm.

## Claims

1. An antibody fragment comprising a single antigen binding arm and an Fc region that increases stability of said antibody fragment compared to a Fab molecule comprising said antigen binding arm, wherein the Fc region comprises a complex of a first and a second Fc polypeptide, wherein one but not both of the Fc polypeptides is an N-terminally truncated heavy chain, and wherein the antibody fragment is aglycosylated.

2. An antibody fragment according to claim 1, wherein the antibody does not possess substantial antibody dependent cell cytotoxicity or complement lysis effector function.

3. An antibody fragment according to claim 1 or claim 2, which specifically binds a cell surface receptor that is activated upon receptor dimerization.

4. An antibody fragment according to any of the preceding claims, wherein the antibody fragment comprises a first polypeptide comprising a light chain variable domain, a second polypeptide comprising a heavy chain variable domain and said first Fc polypeptide, and a third polypeptide comprising said second Fc polypeptide.

5. An antibody fragment according to any of the preceding claims, wherein the first Fc polypeptide and the second Fc polypeptide meet at an interface, and the interface of the second Fc polypeptide comprises a protuberance which is positionable in a cavity in the interface of the first Fc polypeptide.

6. A composition comprising a population of immunoglobulins wherein at least 75% of the immunoglobulins is an antibody fragment according to any of the preceding claims.

7. A method of preparing an antibody fragment according to any of claims 1 to 5, comprising the steps of:

    (a) culturing a host cell comprising nucleic acid encoding the antibody fragment; and
    (b) recovering the antibody fragment from the host cell culture.

8. A method according to claim 7, wherein polypeptides comprising the antibody fragment are expressed at ratios that results in a population of immunoglobulins wherein at least 50% of the immunoglobulins are the antibody fragment of any of claims 1 to 5.

9. A method according to claim 8, wherein approximately equimolar amounts of said polypeptides are expressed.

10. A method according to any of claims 7 to 9, wherein the host cell is *E. coli.*

11. A pharmaceutical formulation comprising an antibody fragment according to any of the preceding claims, and optional physiologically acceptable carriers, excipients or stabilizers.

12. An antibody fragment according to any of claims 1 to 5, or a composition according to claim 6, for use in a method of treating or delaying progression of a disease,
    the method comprising administering an effective amount of the antibody fragment to a subject having the disease.

13. An antibody fragment according to claim 2, for use in treating pathological conditions wherein an immune effector response is deleterious.

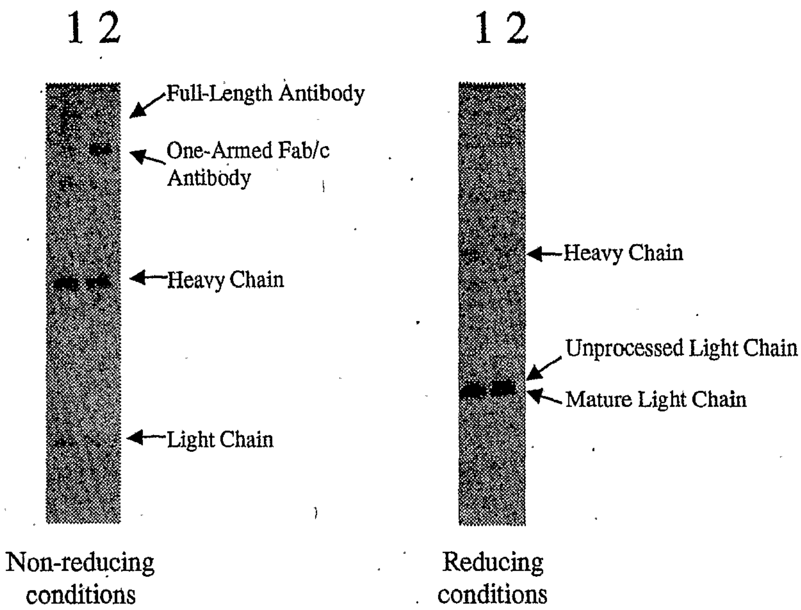


FIG._1

Anti-Fab Western Blot Analysis

1) anti-c-Met FL (pxcM11C)
2) anti-c-Met - Fc (pxcM11C-Fc)

FIG._2

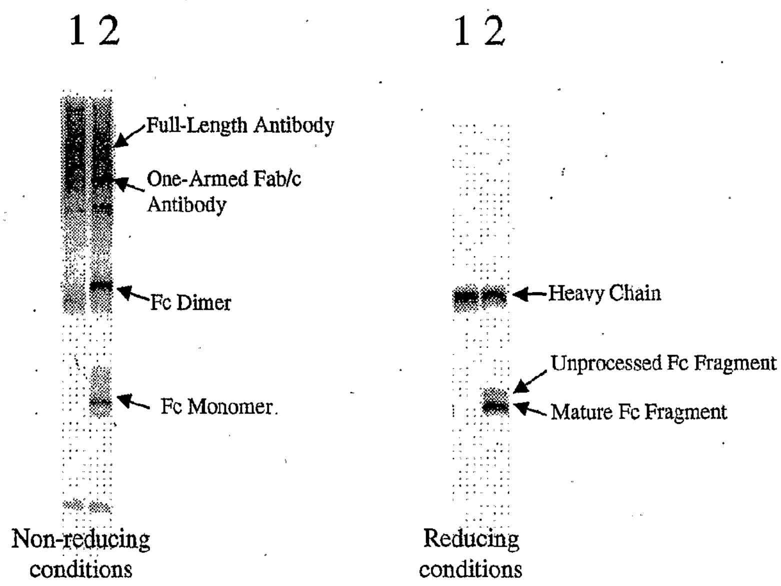


Anti-Fc Western Blot Analysis

1) anti-c-Met FL (pxcM11C)
2) anti-c-Met - Fc (pxcM11C-Fc)

# FIG._3

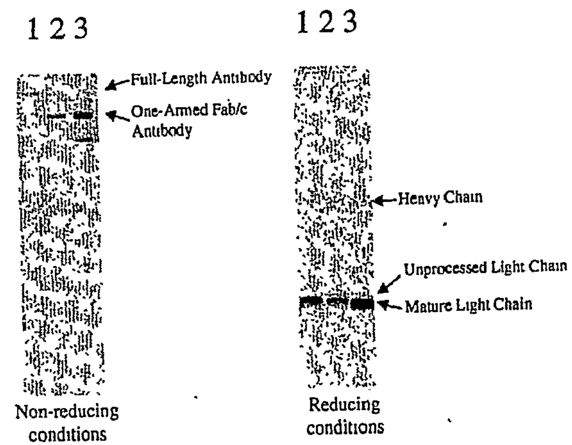


Anti-Fab Western Blot Analysis

1) anti-c-Met FL (pxcM11C)
2) anti-c-Met - Fc (pxcM11C-Fc)
3) anti-c-Met.Hole-Fc.Knob (pxcM11C.H-Fc.K)

FIG._4

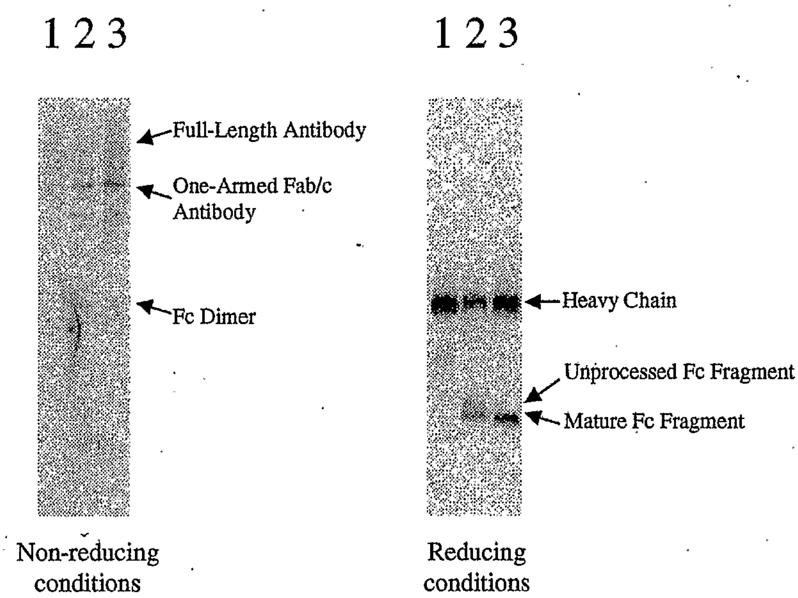


Anti-Fc Western Blot Analysis

1) anti-c-Met FL (pxcM11C)
2) anti-c-Met - Fc (pxcM11C-Fc)
3) anti-c-Met.Hole-Fc.Knob (pxcM11C.H-Fc.K)

FIG._5

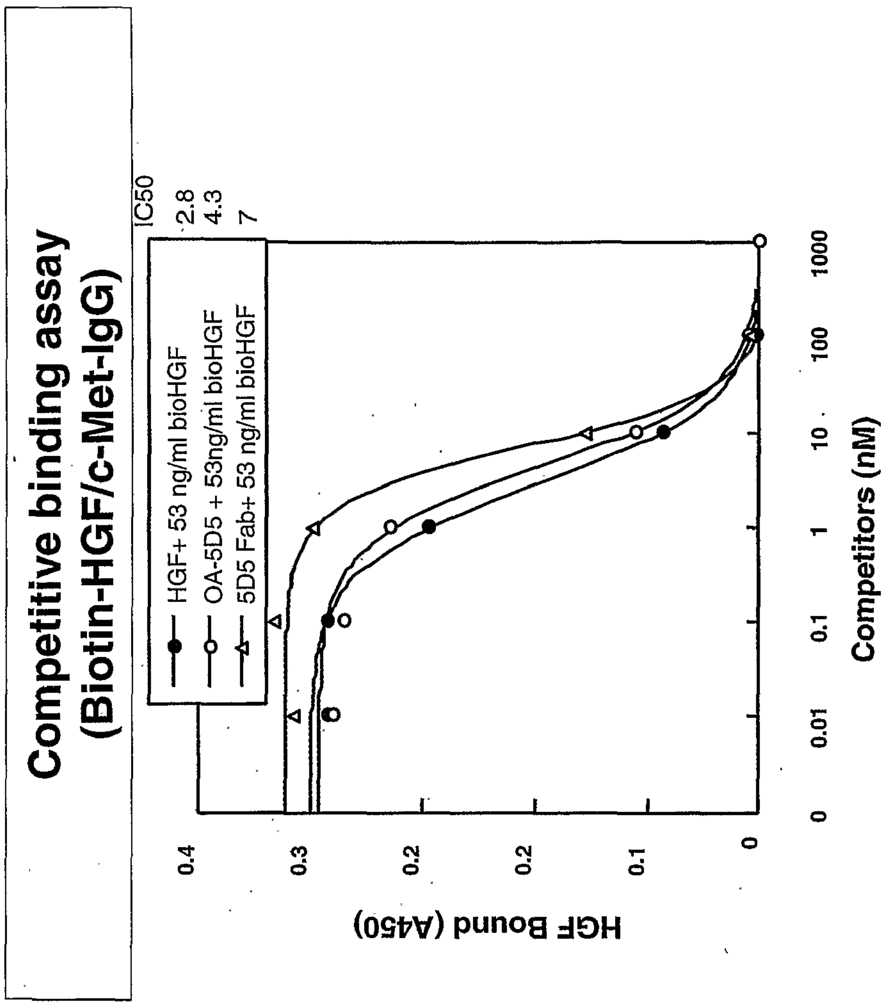
Competitive binding assay
(Biotin-HGF/c-Met-IgG)
HGF+ 53 ng/ml bioHGF
OA-5D5 + 53ng/ml bioHGF
5D5 Fab+ 53 ng/ml bioHGF
IC50
2.8
4.3
7
HGF Bound (A450)
0.4
0.3
0.2
0.2
0.1
0
0
0.01
0.1
1
10
100
1000
Competitors (nM)

FIG._6. U87 Cells, KIRA Assay

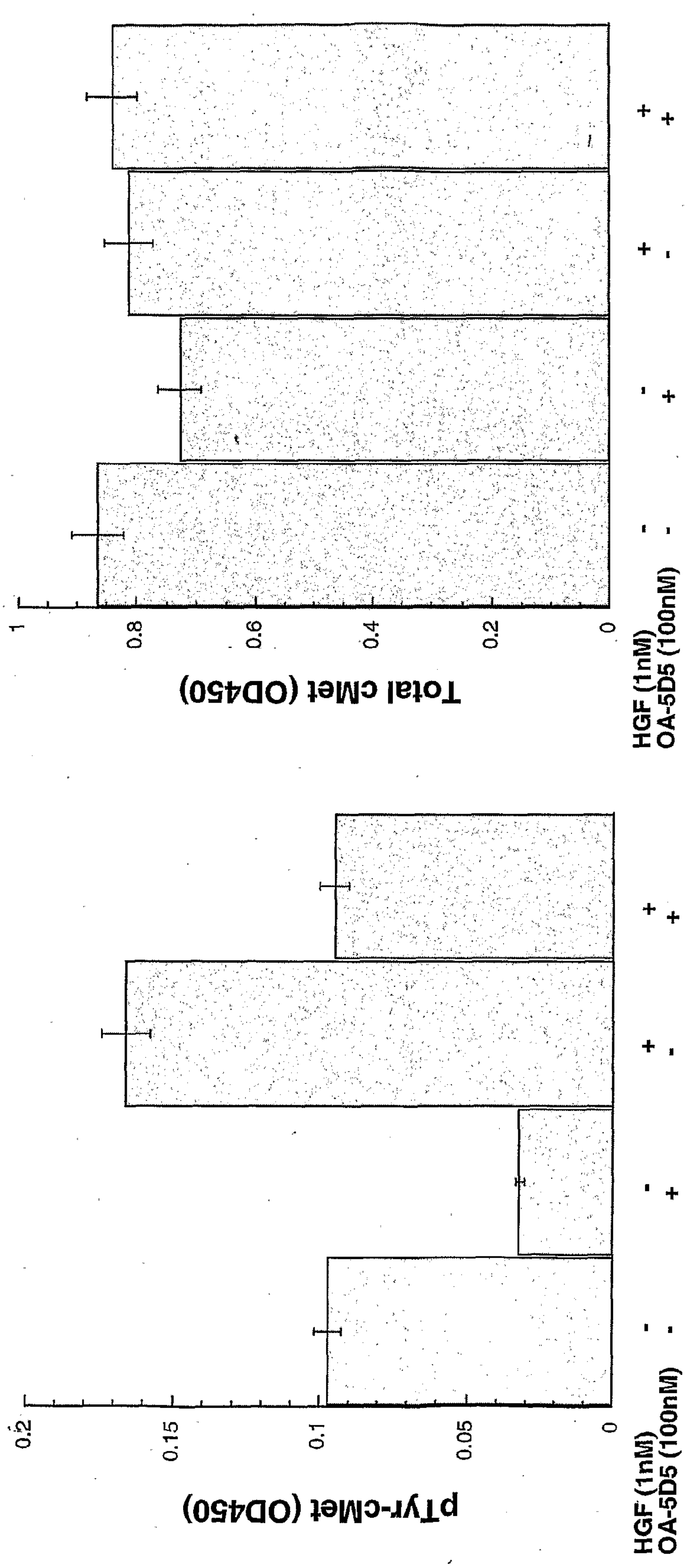

**FIG._7**. One-Armed 5D5 blocks HGF-, but not IL-3 Induced Proliferation in BaF3-hMet cells

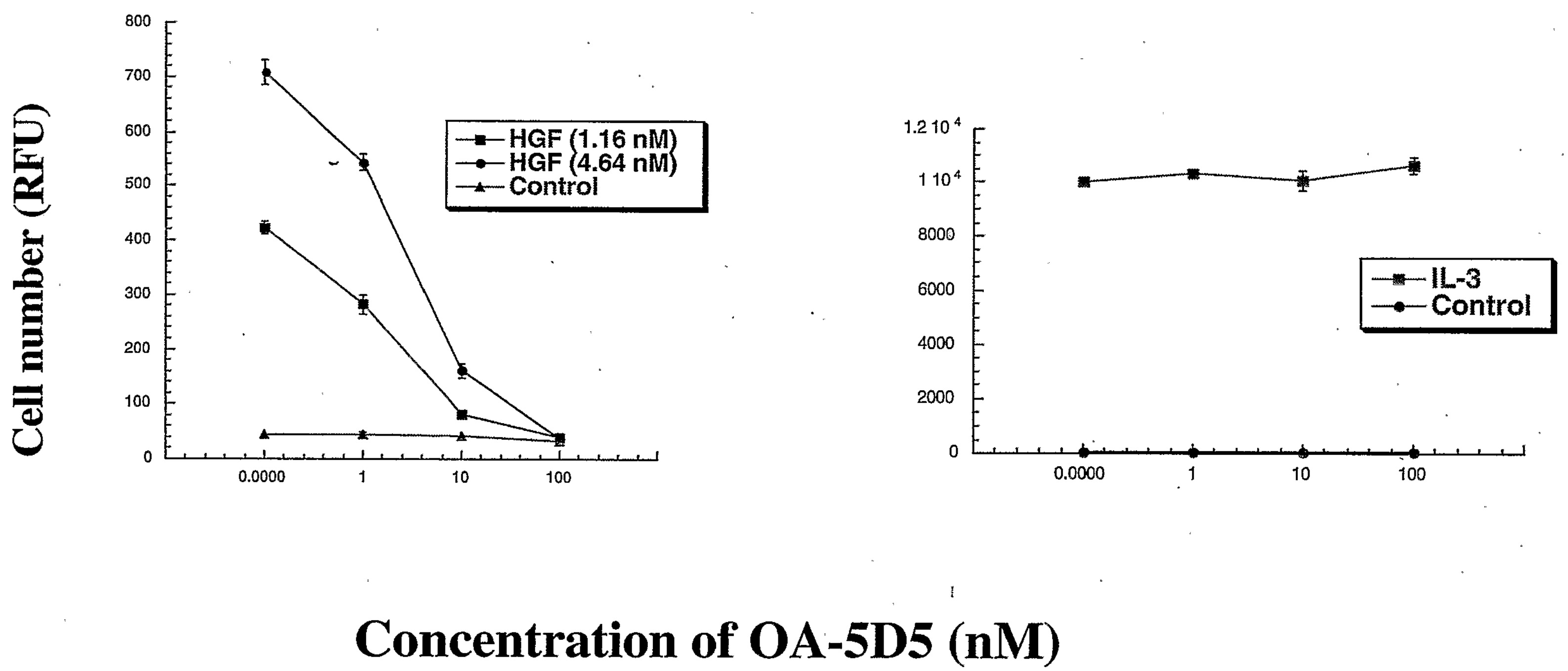

FIG._8

## Migration assay (Boyden Chamber)

MDAMB-435-PGL3 Cell Migration Assay

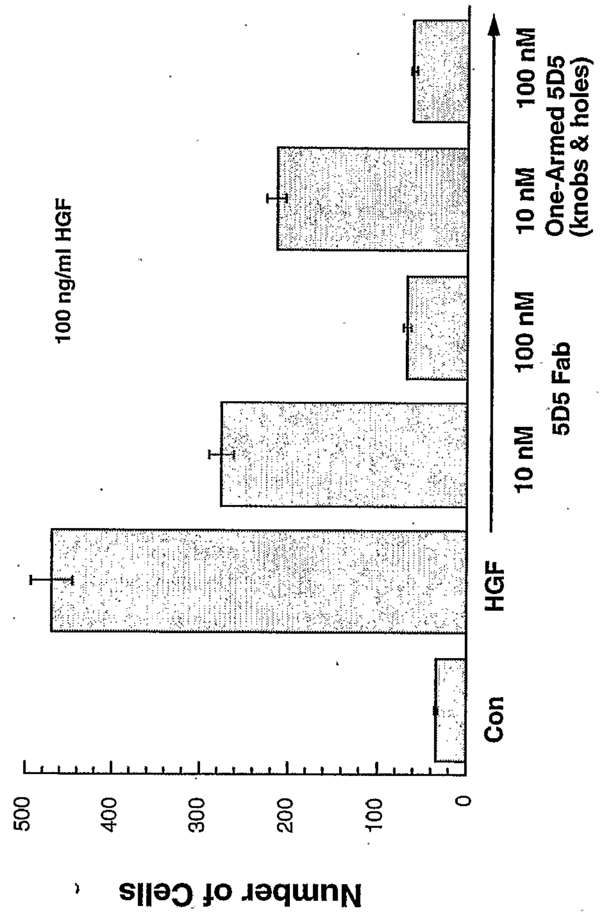

FIG._9A

| | Half-life |
|---|---|
| 5D5.Fab | 0.01 days |
| OA-5D5 | 4.3 days |

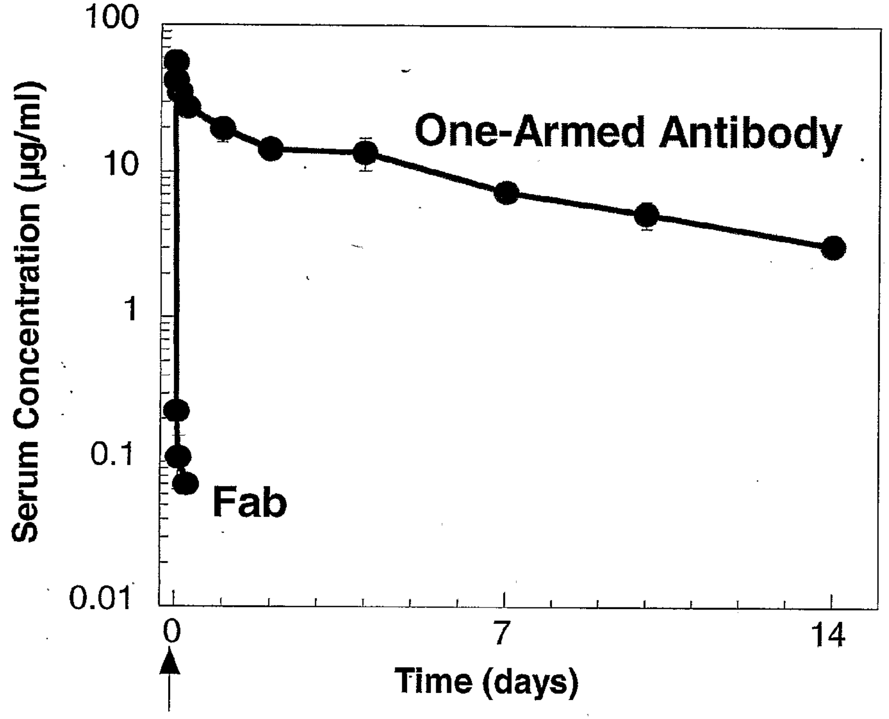


FIG._9B

**Serum OA-5D5 on Day 7 is Intact**

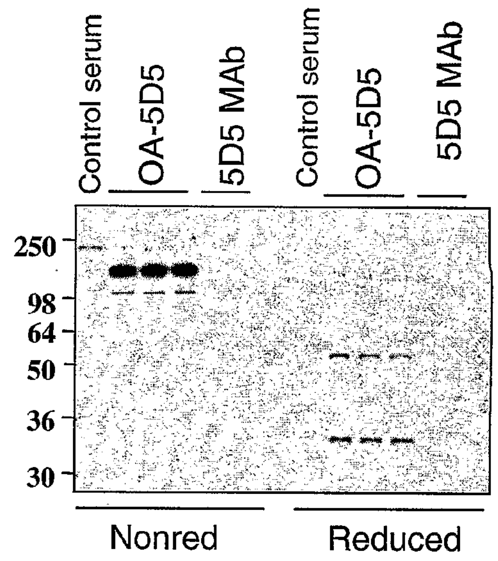


Blot:HRP-anti-Human Fc

FIG._10A

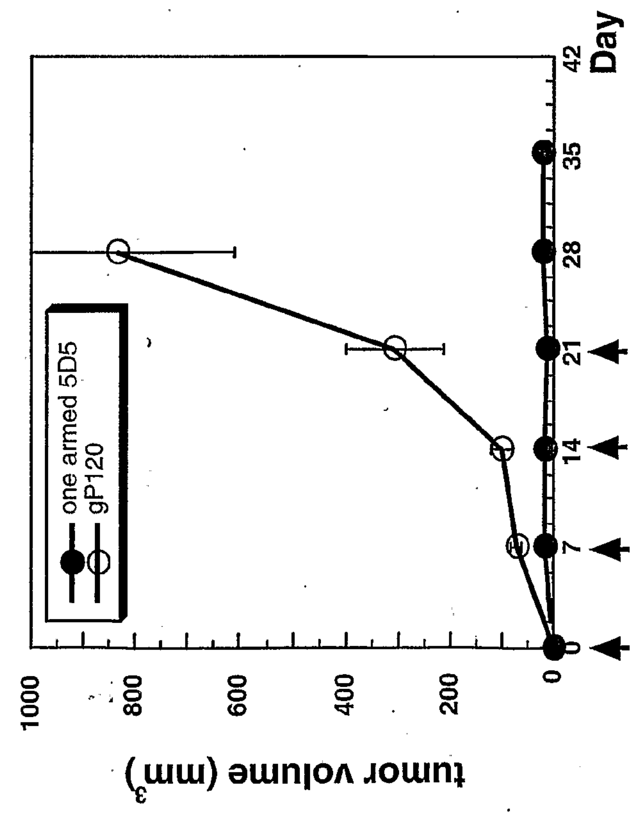


FIG._10B

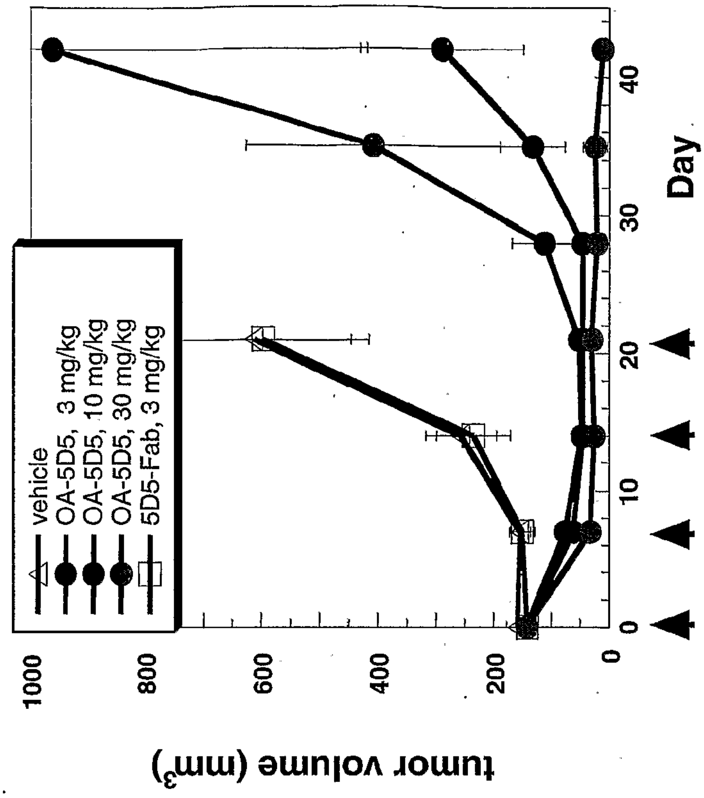

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60531409 B
- US 6468529 B
- US 5169939 A
- EP 0131424 A
- US 5968509 A
- WO 0145746 A
- US 4816567 A
- US 5648237 A, Carter
- US 5840523 A, Simmons
- US 5639635 A
- US 6083715 A, Georgiou
- US 6027888 A, Georgiou
- US 5264365 A, Georgiou
- US 5508192 A, Georgiou
- US 4965199 A
- US 4419446 A
- US 4601978 A
- WO 9411026 A
- US 4767704 A
- US 4657866 A
- US 4927762 A
- US 4560655 A
- US 5122469 A
- WO 9003430 A
- WO 8700195 A
- US RE30985 E
- US 5500362 A
- US 5821337 A
- US 5731168 A
- WO 9951642 A
- US 5648260 A
- US 5624821 A
- WO 9429351 A
- US 4975278 A
- EP 1391213 A
- US 4970198 A
- US 5079233 A
- US 5585089 A
- US 5606040 A
- US 5693762 A
- US 5739116 A
- US 5767285 A
- US 5773001 A
- WO 9321232 A
- US 3896111 A
- US 4151042 A
- US 4137230 A
- US 4248870 A
- US 4256746 A
- US 4260608 A
- US 4265814 A
- US 4294757 A
- US 4307016 A
- US 4308268 A
- US 4308269 A
- US 4309428 A
- US 4313946 A
- US 4315929 A
- US 4317821 A
- US 4322348 A
- US 4331598 A
- US 4361650 A
- US 4364866 A
- US 4424219 A
- US 4450254 A
- US 4362663 A
- US 4371533 A
- US 5208020 A
- US 5416064 A
- EP 0425235 B1
- US 5712374 A
- US 5714586 A
- US 5770701 A
- US 5770710 A
- US 5877296 A
- US 5053394 A
- US 5362852 A
- US 3773919 A
- US 5686292 A
- US 5646036 A
- US 6207152 A
- US 6214344 A
- US 6468529 A
- US 6271342 B


### Non-patent literature cited in the description


- **Cobbold ; Waldmann.** *Nature,* 1984, vol. 308, 460-462
- **Glennie ; Stevenson.** *Nature,* 1982, vol. 295, 712-714

- **Nielsen ; Routledge.** *Blood,* 2002, vol. 100, 4067-4073
- **Stevenson et al.** *Anticancer Drug Des.,* 1989, vol. 3 (4), 219-230
- **Routledge et al.** *Transplantation,* 1995, vol. 60, 847-853
- **Clark et al.** *Eur. J. Immunol.,* 1989, vol. 19, 381-388
- **Bolt et al.** *Eur. J. Immunol.,* 1993, vol. 23, 403-411
- **Routledge et al.** *Eur. J. Immunol.,* 1991, vol. 21, 2717-2725
- **Staerz et al.** *Nature,* 1985, vol. 314, 628-631
- **Dennis et al.** *J. Biol. Chem.,* 2002, vol. 277, 35035-35043
- **Kabat et al.** Sequences of proteins of immunological interest. NIH, 1991, vol. 1, 688-696
- **Simmons ; Yansura.** *Nature Biotechnol.,* 1996, vol. 14, 629-634
- **Simmons et al.** *J. Immunol. Methods,* 2002, vol. 263, 133-147
- **Sambrook et al.** Molecular Cloning: A Laboratory Manual. 1989
- Oligonucleotide Synthesis. 1984
- Animal Cell Culture. 1987
- Methods in Enzymology. Academic Press, Inc,
- Current Protocols in Molecular Biology. 1987
- PCR: The Polymerase Chain Reaction. 1994
- **Perbal Bernard V.** A Practical Guide to Molecular Cloning. 1988
- **Morrison et al.** *Proc. Natl. Acad. Sci. USA,* 1984, vol. 81, 6851-6855
- **Jones et al.** *Nature,* 1984, vol. 321, 522-525
- **Riechmann et al.** *Nature,* 1988, vol. 332, 323-329
- **Presta.** *Curr. Op. Struct. Biol.,* 1992, vol. 2, 593-596
- **Vaswani ; Hamilton.** *Ann. Allergy, Asthma & Immunol.,* 1998, vol. 1, 105-115
- **Harris.** *Biochem. Soc. Transactions,* 1995, vol. 23, 1035-1038
- **Hurle ; Gross.** *Curr. Op. Biotech.,* 1994, vol. 5, 428-433
- **Marks et al.** *Bio/Technology,* 1992, vol. 10, 779-783
- **Barbas et al.** *Proc Nat. Acad. Sci, USA,* 1994, vol. 91, 3809-3813
- **Schier et al.** *Gene,* 1995, vol. 169, 147-155
- **Yelton et al.** *J. Immunol.,* 1995, vol. 155, 1994-2004
- **Jackson et al.** *J. Immunol.,* 1995, vol. 154 (7), 3310-9
- **Hawkins et al.** *J. Mol. Biol.,* 1992, vol. 226, 889-896
- **Janeway et al.** Immuno Biology: the immune system in health and disease. Elsevier Science Ltd, 1999
- **Daëron.** *Annu. Rev. Immunol.,* 1997, vol. 15, 203-234
- **Ravetch ; Kinet.** *Annu. Rev. Immunol,* 1991, vol. 9, 457-92
- **Capel et al.** *Immunomethods,* 1994, vol. 4, 25-34
- **de Haas et al.** *J. Lab. Clin. Med.,* 1995, vol. 126, 330-41
- **Guyer et al.** *J. Immunol.,* 1976, vol. 117, 587
- **Kim et al.** *J. Immunol.,* 1994, vol. 24, 249
- **Bloom et al.** *Protein Science,* 1997, vol. 6, 407-415
- **Humphreys et al.** *J. Immunol. Methods,* 1997, vol. 209, 193-202
- **Agnew.** *Chem Intl. Ed. Engl.,* 1994, vol. 33, 183-186
- Handbook of Chemistry and Physics. Chemical Rubber Publishing Co, 1961
- **A.A. Zamyatnin.** *Prog. Biophys. Mol. Biol.,* 1972, vol. 24, 107-123
- **C. Chothia.** *J. Mol. Biol.,* 1975, vol. 105, 1-14
- **Ellman et al.** *Meth. Enzym.,* 1991, vol. 202, 301-336
- **Noren et al.** *Science,* 1989, vol. 244, 182
- Mutagenesis: a Practical Approach. IRL Press, 1991
- **Ponders ; Richards.** *J. Mol. Biol.,* 1987, vol. 193, 775-791
- **Siebenlist et al.** *Cell,* 1980, vol. 20, 269
- **Proba ; Pluckthun.** *Gene,* 1995, vol. 159, 203
- **Yansura et al.** *METHODS: A Companion to Methods in Enzymol.,* 1992, vol. 4, 151-158
- **Bachmann.** Cellular and Molecular Biology. American Society for Microbiology, 1987, vol. 2, 1190-1219
- **Bass et al.** *Proteins,* 1990, vol. 8, 309-314
- **Chen et al.** *JBio Chem,* 1999, vol. 274, 19601-19605
- **Bothmann ; Pluckthun.** *J. Biol. Chem.,* 2000, vol. 275, 17100-17105
- **Ramm ; Pluckthun.** *J. Biol. Chem.,* 2000, vol. 275, 17106-17113
- **Arie et al.** *Mol. Microbial.,* 2001, vol. 39, 199-210
- **Hara et al.** *Microbial Drug Resistance,* 1996, vol. 2, 63-72
- **Lindmark et al.** *J. Immunol. Meth.,* 1983, vol. 62, 1-13
- **Reyes et al.** *Nature,* 1982, vol. 297, 598-601
- **Yaniv.** *Nature,* 1982, vol. 297, 17-18
- **Graham et al.** *J. Gen Virol.,* 1977, vol. 36, 59
- **CHO, Urlaub et al.** *Proc. Natl. Acad. Sci. USA,* 1980, vol. 77, 4216
- **Mather.** *Biol. Reprod.,* 1980, vol. 23, 243-251
- **Mather et al.** *Annals N.Y. Acad. Sci.,* 1982, vol. 383, 44-68
- **Ham et al.** *Meth. Enz.,* 1979, vol. 58, 44

- **Barnes et al.** *Anal. Biochem,* 1980, vol. 102, 255
- **Guss et al.** *EMBO J.,* 1986, vol. 5, 15671575
- **Clynes et al.** *PNAS (USA),* 1998, vol. 95, 652-656
- **Gazzano-Santoro et al.** *J. Immunol. Methods,* 1996, vol. 202, 163
- **Jones et al.** *Nature,* 1986, vol. 321, 522-525
- **Riechmann et al.** *Nature,* 1988, vol. 332, 323-327
- **Verhoeyen et al.** *Science,* 1988, vol. 239, 1534-1536
- **Sims et al.** *J. Immunol.,* 1993, vol. 151, 2296
- **Chothia et al.** *J. Mol. Biol.,* 1987, vol. 196, 901
- **Carter et al.** *Proc. Natl. Acad. Sci. USA,* 1992, vol. 89, 4285
- **Presta et al.** *J. Immunol.,* 1993, vol. 151, 2623
- **Cunningham ; Wells.** *Science,* 1989, vol. 244, 1081-1085
- **Duncan ; Winter.** *Nature,* 1988, vol. 322, 738-40
- **Syrigos ; Epenetos.** *Anticancer Research,* 1999, vol. 19, 605-614
- **Niculescu-Duvaz ; Springer.** *Adv. Drg Del. Rev.,* 1997, vol. 26, 151-172
- **Baldwin et al.** *Lancet,* 15 March 1986, 603-05
- Antibody Carriers Of Cytotoxic Agents In Cancer Therapy: A Review. **Thorpe et al.** Monoclonal Antibodies '84: Biological And Clinical Applications. 1985, 475-506
- **Rowland et al.** *Cancer Immunol. Immunother.,* 1986, vol. 21, 183-87
- **Mandler et al.** *Jour. of the Nat. Cancer Inst.,* 2000, vol. 92 (19), 1573-1581
- **Mandler et al.** *Bioorganic & Med. Chem. Letters,* 2000, vol. 10, 1025-1028
- **Mandler et al.** *Bioconjugate Chem.,* 2002, vol. 13, 786-791
- **Liu et al.** *Proc. Natl. Acad. Sci. USA,* 1996, vol. 93, 8618-8623
- **Lode et al.** *Cancer Res.,* 1998, vol. 58, 2928
- **Hinman et al.** *Cancer Res.,* 1993, vol. 53, 3336-3342
- **Wiseman et al.** *Eur. Jour. Nucl. Med.,* 2000, vol. 27 (7), 766-77
- **Wiseman et al.** *Blood,* 2002, vol. 99 (12), 4336-42
- **Witzig et al.** *J. Clin. Oncol.,* 2002, vol. 20 (10), 2453-63
- **Witzig et al.** *J. Clin. Oncol.,* 2002, vol. 20 (15), 3262-69
- *Drugs of the Future,* 2000, vol. 25 (7), 686
- **Doronina et al.** *Nature Biotechnology,* 2003, vol. 21 (7), 778-784
- **Vitetta et al.** *Science,* 1987, vol. 238, 1098
- **Chari et al.** *Cancer Research,* 1992, vol. 52, 127-131
- **Carlsson et al.** *Biochem. J.,* 1978, vol. 173, 723-737
- **Hinman et al.** *Cancer Research,* 1993, vol. 53, 3336-3342
- **Lode et al.** *Cancer Research,* 1998, vol. 58, 2925-2928
- **Fraker et al.** *Biochem. Biophys. Res. Commun.,* 1978, vol. 80, 49-57
- **Chatal.** Monoclonal Antibodies in Immunoscintigraphy. CRC Press, 1989
- Applications Handbook and Catalog. 2003, 467-498
- **Geoghegan ; Stroh.** *Bioconjugate Chem.,* 1992, vol. 3, 138-146
- Remington's Pharmaceutical Sciences. 1980
- **Kikuchi et al.** *Nucleic Acids Res.,* 1981, vol. 9, 5671-5678
- **Yanofsky et al.** *Nucleic Acids Res.,* 1981, vol. 9, 6647-6668
- **Chang et al.** *Gene,* 1987, vol. 55, 189-196
- **Picken et al.** *Infect. Immun.,* 1983, vol. 42, 269-275
- **Lee et al.** *Infect. Immun.,* 1983, vol. 42, 264-268
- **Schlosstissek ; Grosse.** *Nucleic Acids Res.,* 1987, vol. 15, 3185
- **Sutcliffe.** *Cold Spring Harbor Symp. Quant. Biol.,* 1978, vol. 43, 77-90
- **Simmons ; Yansura.** *Nature Biotechnol,* 1996, vol. 14, 629-634
- **Merchant et al.** *Nature Biotechnology,* 1998, vol. 16, 677-681
- **Mark et al.** *J.Biol.Chem.,* 1992, vol. 267, 26166-26171
- **Laskov et al.** *Cell.Immunol.,* 1980, vol. 55, 251
- **Henzel et al.** *J.Chromatog.,* 1996, vol. 404, 41-52
- **Krutzsch ; Inman.** *Anal.Biochem.,* 1993, vol. 209, 109-116
- **Kabat et al.** Sequences of proteins of immunological interest. National Institutes of Health, 1991
- **Kawasaki.** Amplification of RNA. In PCR Protocols: A Guide to Methods and Applications. Academic Press, Inc, 1990, 21-27
- **Carter et al.** *Bio/Technol.,* 1992, vol. 10, 163-167
- **Kunkel.** *Proc.Natl.Acad.Sci. USA,* 1985, vol. 82, 488
- **Rodriques et al.** *Cancer Res.,* 1995, vol. 55, 63-70
- **Schwall et al.** *J.Cell Biol.,* 1996, vol. 133, 709-718
- **Sadick et al.** *Anal.Biochem.,* 1996, vol. 235, 207-214
- **Schwall et al.** *J. Cell Biol.,* 1996, vol. 133, 709-718